(19) Europäisches Patentamt
European Patent Office
Office européen des brevets

(11) EP 1 947 182 A1

(12) EUROPEAN PATENT APPLICATION

(43) Date of publication:
23.07.2008 Bulletin 2008/30

(21) Application number: 07017423.0

(22) Date of filing: 15.02.2001

(51) Int Cl.:
C12N 15/12 (2006.01)    C12N 15/62 (2006.01)
C12N 15/86 (2006.01)    C07K 14/50 (2006.01)
C07K 16/22 (2006.01)    C12Q 1/68 (2006.01)
A61K 9/00 (2006.01)    A61K 38/18 (2006.01)
A01K 67/027 (2006.01)    G01N 33/68 (2006.01)

(84) Designated Contracting States:
AT BE CH CY DE DK ES FI FR GB GR IE IT LI LU
MC NL PT SE TR
Designated Extension States:
AL LT LV MK RO SI

(30) Priority: 15.02.2000 US 182442 P
20.04.2000 US 198903 P
15.02.2001 US 784561

(62) Document number(s) of the earlier application(s) in
accordance with Art. 76 EPC:
01914373.4 / 1 257 645

(71) Applicant: Amgen Inc.
Thousand Oaks, CA 91320-1799 (US)

(72) Inventors:
• Luethy, Roland
Camarillo, CA 93012 (US)

• Suggs, Sidney
Newbury Park, CA 91320 (US)
• Sarosi, Iidiko
Newbury Park, CA 91320 (US)
• Yang, Robert
San Diego, CA 92130 (US)

(74) Representative: Grünecker, Kinkeldey,
Stockmair & Schwanhäusser
Anwaltssozietät
Leopoldstrasse 4
80802 München (DE)

Remarks:
This application was filed on 05.09.2007 as a
divisional application to the application mentioned
under INID code 62.

(54) Fibroblast growth factor-23 molecules and uses thereof

(57) The present invention provides Fibroblast Growth Factor-23 (FGF-23) polypeptides and nucleic acid molecules encoding the same. The invention also provides selective binding agents, vectors, host cells, and methods for producing FGF-23 polypeptides. The invention further provides pharmaceutical compositions and methods for the diagnosis, treatment, amelioration, and/or prevention of diseases, disorders, and conditions associated with FGF-23 polypeptides.

Figure 1A

```
atg ttg ggg gcc cgc ctc agg ctc tgg gtc tgt gcc ttg tgc agc gtc    48
Met Leu Gly Ala Arg Leu Arg Leu Trp Val Cys Ala Leu Cys Ser Val
 1            5              10             15

tgc agc atg agc gtc ctc aga gcc tat ccc aat gcc tcc cca ctg ctc    96
Cys Ser Met Ser Val Leu Arg Ala Tyr Pro Asn Ala Ser Pro Leu Leu
        20             25             30

ggc tcc agc tgg ggt ggc ctg atc cac ctg tac aca gcc aca gcc agg    144
Gly Ser Ser Trp Gly Gly Leu Ile His Leu Tyr Thr Ala Thr Ala Arg
        35             40             45

aac agc tac cac ctg cag atc cac aag aat ggc cat gtg gat ggc gca    192
Asn Ser Tyr His Leu Gln Ile His Lys Asn Gly His Val Asp Gly Ala
        50             55             60

ccc cat cag acc atc tac agt gcc ctg atg atc aga tca gag gat gct    240
Pro His Gln Thr Ile Tyr Ser Ala Leu Met Ile Arg Ser Glu Asp Ala
 65             70             75             80

ggc ttt gtg gtg att aca ggt gtg atg agc aga aga tac ctc tgc atg    288
Gly Phe Val Val Ile Thr Gly Val Met Ser Arg Arg Tyr Leu Cys Met
                85             90             95

gat ttc aga ggc aac att ttt gga tca cac tat ttc gac ccg gag aac    336
Asp Phe Arg Gly Asn Ile Phe Gly Ser His Tyr Phe Asp Pro Glu Asn
                100            105            110

tgc agg ttc caa cac cag acg ctg gaa aac ggg tac gac gtc tac cac    384
Cys Arg Phe Gln His Gln Thr Leu Glu Asn Gly Tyr Asp Val Tyr His
        115            120            125

tct cct cag tat cac ttc ctg gtc agt ctg ggc cgg gcg aag aga gcc    432
Ser Pro Gln Tyr His Phe Leu Val Ser Leu Gly Arg Ala Lys Arg Ala
        130            135            140

ttc ctg cca ggc atg aac cca ccc ccg tac tcc cag ttc ctg tcc cgg    480
Phe Leu Pro Gly Met Asn Pro Pro Pro Tyr Ser Gln Phe Leu Ser Arg
145            150            155            160

agg aac gag atc ccc cta att cac ttc aac acc ccc ata cca cgg cgg    528
Arg Asn Glu Ile Pro Leu Ile His Phe Asn Thr Pro Ile Pro Arg Arg
                165            170            175

cac acc cgg agc gcc gag gac gac tcg gag cgg gac ccc ctg aac gtg    576
His Thr Arg Ser Ala Glu Asp Asp Ser Glu Arg Asp Pro Leu Asn Val
                180            185            190

ctg aag ccc cgg gcc cgg atg acc ccg gcc ccg gcc tcc tgt tca cag    624
Leu Lys Pro Arg Ala Arg Met Thr Pro Ala Pro Ala Ser Cys Ser Gln
        195            200            205
```

EP 1 947 182 A1

# Figure 1B

```
gag ctc ccg agc gcc gag gac aac agc ccg atg gcc agt gac cca tta   672
Glu Leu Pro Ser Ala Glu Asp Asn Ser Pro Met Ala Ser Asp Pro Leu
    210             215             220


ggg gtg gtc agg ggc ggt cga gtg aac acg cac gct ggg gga acg ggc   720
Gly Val Val Arg Gly Gly Arg Val Asn Thr His Ala Gly Gly Thr Gly
225             230             235             240


ccg gaa ggc tgc cgc ccc ttc gcc aag ttc atc                       753
Pro Glu Gly Cys Arg Pro Phe Ala Lys Phe Ile
            245             250
```

SEQUENCE LISTING

<110> Luethy, Roland
        Yang, Robert
        Suggs, Sidney V.
        Sarosi, IIdiko

<120> Fibroblast Growth Factor-23 Molecules and Uses Thereof

<130> 01-004

<140> 60/182,442
<141> 2000-02-15

<160> 54

<170> PatentIn Ver. 2.0

<210> 1
<211> 753
<212> DNA
<213> Homo sapiens

<220>
<221> CDS
<222> (1)..(753)

<220>
<221> sig_peptide
<222> (1)..(72)

<400> 1
atg ttg ggg gcc cgc ctc agg ctc tgg gtc tgt gcc ttg tgc agc gtc    48
Met Leu Gly Ala Arg Leu Arg Leu Trp Val Cys Ala Leu Cys Ser Val
  1               5                  10                  15

tgc agc atg agc gtc ctc aga gcc tat ccc aat gcc tcc cca ctg ctc    96
Cys Ser Met Ser Val Leu Arg Ala Tyr Pro Asn Ala Ser Pro Leu Leu
              20                  25                  30

ggc tcc agc tgg ggt ggc ctg atc cac ctg tac aca gcc aca gcc agg   144
Gly Ser Ser Trp Gly Gly Leu Ile His Leu Tyr Thr Ala Thr Ala Arg
              35                  40                  45

aac agc tac cac ctg cag atc cac aag aat ggc cat gtg gat ggc gca   192
Asn Ser Tyr His Leu Gln Ile His Lys Asn Gly His Val Asp Gly Ala
          50                  55                  60

ccc cat cag acc atc tac agt gcc ctg atg atc aga tca gag gat gct   240
Pro His Gln Thr Ile Tyr Ser Ala Leu Met Ile Arg Ser Glu Asp Ala
 65                  70                  75                  80

ggc ttt gtg gtg att aca ggt gtg atg agc aga aga tac ctc tgc atg   288
Gly Phe Val Val Ile Thr Gly Val Met Ser Arg Arg Tyr Leu Cys Met
                  85                  90                  95

gat ttc aga ggc aac att ttt gga tca cac tat ttc gac ccg gag aac   336
Asp Phe Arg Gly Asn Ile Phe Gly Ser His Tyr Phe Asp Pro Glu Asn
                 100                 105                 110

tgc agg ttc caa cac cag acg ctg gaa aac ggg tac gac gtc tac cac   384

```
        Cys Arg Phe Gln His Gln Thr Leu Glu Asn Gly Tyr Asp Val Tyr His
                115             120             125

        tct cct cag tat cac ttc ctg gtc agt ctg ggc cgg gcg aag aga gcc    432
        Ser Pro Gln Tyr His Phe Leu Val Ser Leu Gly Arg Ala Lys Arg Ala
                130             135             140

        ttc ctg cca ggc atg aac cca ccc ccg tac tcc cag ttc ctg tcc cgg    480
        Phe Leu Pro Gly Met Asn Pro Pro Pro Tyr Ser Gln Phe Leu Ser Arg
        145             150             155             160

        agg aac gag atc ccc cta att cac ttc aac acc ccc ata cca cgg cgg    528
        Arg Asn Glu Ile Pro Leu Ile His Phe Asn Thr Pro Ile Pro Arg Arg
                        165             170             175

        cac acc cgg agc gcc gag gac gac tcg gag cgg gac ccc ctg aac gtg    576
        His Thr Arg Ser Ala Glu Asp Asp Ser Glu Arg Asp Pro Leu Asn Val
                180             185             190

        ctg aag ccc cgg gcc cgg atg acc ccg gcc ccg gcc tcc tgt tca cag    624
        Leu Lys Pro Arg Ala Arg Met Thr Pro Ala Pro Ala Ser Cys Ser Gln
                195             200             205

        gag ctc ccg agc gcc gag gac aac agc ccg atg gcc agt gac cca tta    672
        Glu Leu Pro Ser Ala Glu Asp Asn Ser Pro Met Ala Ser Asp Pro Leu
                210             215             220

        ggg gtg gtc agg ggc ggt cga gtg aac acg cac gct ggg gga acg ggc    720
        Gly Val Val Arg Gly Gly Arg Val Asn Thr His Ala Gly Gly Thr Gly
        225             230             235             240

        ccg gaa ggc tgc cgc ccc ttc gcc aag ttc atc                        753
        Pro Glu Gly Cys Arg Pro Phe Ala Lys Phe Ile
                        245             250


        <210> 2
        <211> 251
        <212> PRT
        <213> Homo sapiens

        <400> 2
        Met Leu Gly Ala Arg Leu Arg Leu Trp Val Cys Ala Leu Cys Ser Val
            1               5               10              15

        Cys Ser Met Ser Val Leu Arg Ala Tyr Pro Asn Ala Ser Pro Leu Leu
                    20              25              30

        Gly Ser Ser Trp Gly Gly Leu Ile His Leu Tyr Thr Ala Thr Ala Arg
                    35              40              45

        Asn Ser Tyr His Leu Gln Ile His Lys Asn Gly His Val Asp Gly Ala
                50              55              60

        Pro His Gln Thr Ile Tyr Ser Ala Leu Met Ile Arg Ser Glu Asp Ala
        65              70              75              80

        Gly Phe Val Val Ile Thr Gly Val Met Ser Arg Arg Tyr Leu Cys Met
                        85              90              95

        Asp Phe Arg Gly Asn Ile Phe Gly Ser His Tyr Phe Asp Pro Glu Asn
```

```
                    100                     105                     110
       Cys Arg Phe Gln His Gln Thr Leu Glu Asn Gly Tyr Asp Val Tyr His
               115                 120                 125

       Ser Pro Gln Tyr His Phe Leu Val Ser Leu Gly Arg Ala Lys Arg Ala
           130                 135                 140

       Phe Leu Pro Gly Met Asn Pro Pro Pro Tyr Ser Gln Phe Leu Ser Arg
       145                 150                 155                 160

       Arg Asn Glu Ile Pro Leu Ile His Phe Asn Thr Pro Ile Pro Arg Arg
                       165                 170                 175

       His Thr Arg Ser Ala Glu Asp Asp Ser Glu Arg Asp Pro Leu Asn Val
                   180                 185                 190

       Leu Lys Pro Arg Ala Arg Met Thr Pro Ala Pro Ala Ser Cys Ser Gln
               195                 200                 205

       Glu Leu Pro Ser Ala Glu Asp Asn Ser Pro Met Ala Ser Asp Pro Leu
           210                 215                 220

       Gly Val Val Arg Gly Gly Arg Val Asn Thr His Ala Gly Gly Thr Gly
       225                 230                 235                 240

       Pro Glu Gly Cys Arg Pro Phe Ala Lys Phe Ile
                       245                 250


       <210> 3
       <211> 227
       <212> PRT
       <213> Homo sapiens

       <400> 3
       Tyr Pro Asn Ala Ser Pro Leu Leu Gly Ser Ser Trp Gly Gly Leu Ile
        1               5                   10                  15

       His Leu Tyr Thr Ala Thr Ala Arg Asn Ser Tyr His Leu Gln Ile His
                   20                  25                  30

       Lys Asn Gly His Val Asp Gly Ala Pro His Gln Thr Ile Tyr Ser Ala
               35                  40                  45

       Leu Met Ile Arg Ser Glu Asp Ala Gly Phe Val Val Ile Thr Gly Val
           50                  55                  60

       Met Ser Arg Arg Tyr Leu Cys Met Asp Phe Arg Gly Asn Ile Phe Gly
       65                  70                  75                  80

       Ser His Tyr Phe Asp Pro Glu Asn Cys Arg Phe Gln His Gln Thr Leu
                       85                  90                  95

       Glu Asn Gly Tyr Asp Val Tyr His Ser Pro Gln Tyr His Phe Leu Val
                   100                 105                 110

       Ser Leu Gly Arg Ala Lys Arg Ala Phe Leu Pro Gly Met Asn Pro Pro
               115                 120                 125

       Pro Tyr Ser Gln Phe Leu Ser Arg Arg Asn Glu Ile Pro Leu Ile His
```

```
                130                    135                     140

        Phe Asn Thr Pro Ile Pro Arg Arg His Thr Arg Ser Ala Glu Asp Asp
        145                 150                 155                 160

        Ser Glu Arg Asp Pro Leu Asn Val Leu Lys Pro Arg Ala Arg Met Thr
                        165                 170                 175

        Pro Ala Pro Ala Ser Cys Ser Gln Glu Leu Pro Ser Ala Glu Asp Asn
                        180                 185                 190

        Ser Pro Met Ala Ser Asp Pro Leu Gly Val Val Arg Gly Gly Arg Val
                    195                 200                 205

        Asn Thr His Ala Gly Gly Thr Gly Pro Glu Gly Cys Arg Pro Phe Ala
                    210                 215                 220

        Lys Phe Ile
        225


        <210> 4
        <211> 155
        <212> PRT
        <213> Homo sapiens

        <400> 4
        Met Ala Glu Gly Glu Ile Thr Thr Phe Thr Ala Leu Thr Glu Lys Phe
          1               5                  10                  15

        Asn Leu Pro Pro Gly Asn Tyr Lys Lys Pro Lys Leu Leu Tyr Cys Ser
                    20                  25                  30

        Asn Gly Gly His Phe Leu Arg Ile Leu Pro Asp Gly Thr Val Asp Gly
                35                  40                  45

        Thr Arg Asp Arg Ser Asp Gln His Ile Gln Leu Gln Leu Ser Ala Glu
            50                  55                  60

        Ser Val Gly Glu Val Tyr Ile Lys Ser Thr Glu Thr Gly Gln Tyr Leu
        65                  70                  75                  80

        Ala Met Asp Thr Asp Gly Leu Leu Tyr Gly Ser Gln Thr Pro Asn Glu
                        85                  90                  95

        Glu Cys Leu Phe Leu Glu Arg Leu Glu Glu Asn His Tyr Asn Thr Tyr
                    100                 105                 110

        Ile Ser Lys Lys His Ala Glu Lys Asn Trp Phe Val Gly Leu Lys Lys
                    115                 120                 125

        Asn Gly Ser Cys Lys Arg Gly Pro Arg Thr His Tyr Gly Gln Lys Ala
                    130                 135                 140

        Ile Leu Phe Leu Pro Leu Pro Val Ser Ser Asp
        145                 150                 155


        <210> 5
        <211> 155
        <212> PRT
```

```
<213> Homo sapiens

<400> 5
Met Ala Ala Gly Ser Ile Thr Thr Leu Pro Ala Leu Pro Glu Asp Gly
  1               5                  10                  15

Gly Ser Gly Ala Phe Pro Pro Gly His Phe Lys Asp Pro Lys Arg Leu
             20                  25                  30

Tyr Cys Lys Asn Gly Gly Phe Phe Leu Arg Ile His Pro Asp Gly Arg
         35                  40                  45

Val Asp Gly Val Arg Glu Lys Ser Asp Pro His Ile Lys Leu Gln Leu
     50                  55                  60

Gln Ala Glu Glu Arg Gly Val Val Ser Ile Lys Gly Val Cys Ala Asn
 65                  70                  75                  80

Arg Tyr Leu Ala Met Lys Glu Asp Gly Arg Leu Leu Ala Ser Lys Cys
             85                  90                  95

Val Thr Asp Glu Cys Phe Phe Phe Glu Arg Leu Glu Ser Asn Asn Tyr
             100                 105                 110

Asn Thr Tyr Arg Ser Arg Lys Tyr Thr Ser Trp Tyr Val Ala Leu Lys
         115                 120                 125

Arg Thr Gly Gln Tyr Lys Leu Gly Ser Lys Thr Gly Pro Gly Gln Lys
     130                 135                 140

Ala Ile Leu Phe Leu Pro Met Ser Ala Lys Ser
145                 150                 155


<210> 6
<211> 239
<212> PRT
<213> Homo sapiens

<400> 6
Met Gly Leu Ile Trp Leu Leu Leu Leu Ser Leu Leu Glu Pro Gly Trp
  1               5                  10                  15

Pro Ala Ala Gly Pro Gly Ala Arg Leu Arg Arg Asp Ala Gly Gly Arg
             20                  25                  30

Gly Gly Val Tyr Glu His Leu Gly Gly Ala Pro Arg Arg Arg Lys Leu
         35                  40                  45

Tyr Cys Ala Thr Lys Tyr His Leu Gln Leu His Pro Ser Gly Arg Val
     50                  55                  60

Asn Gly Ser Leu Glu Asn Ser Ala Tyr Ser Ile Leu Glu Ile Thr Ala
 65                  70                  75                  80

Val Glu Val Gly Ile Val Ala Ile Arg Gly Leu Phe Ser Gly Arg Tyr
             85                  90                  95

Leu Ala Met Asn Lys Arg Gly Arg Leu Tyr Ala Ser Glu His Tyr Ser
             100                 105                 110
```

```
Ala Glu Cys Glu Phe Val Glu Arg Ile His Glu Leu Gly Tyr Asn Thr
        115                 120                 125

Tyr Ala Ser Arg Leu Tyr Arg Thr Val Ser Ser Thr Pro Gly Ala Arg
    130             135                 140

Arg Gln Pro Ser Ala Glu Arg Leu Trp Tyr Val Ser Val Asn Gly Lys
145             150                 155                 160

Gly Arg Pro Arg Arg Gly Phe Lys Thr Arg Arg Thr Gln Lys Ser Ser
            165                 170                 175

Leu Phe Leu Pro Arg Val Leu Asp His Arg Asp His Glu Met Val Arg
        180                 185                 190

Gln Leu Gln Ser Gly Leu Pro Arg Pro Pro Gly Lys Gly Val Gln Pro
        195                 200                 205

Arg Arg Arg Arg Gln Lys Gln Ser Pro Asp Asn Leu Glu Pro Ser His
    210                 215                 220

Val Gln Ala Ser Arg Leu Gly Ser Gln Leu Glu Ala Ser Ala His
225                 230                 235
```

```
<210> 7
<211> 206
<212> PRT
<213> Homo sapiens

<400> 7
Met Ser Gly Pro Gly Thr Ala Ala Val Ala Leu Leu Pro Ala Val Leu
  1             5                 10                  15

Leu Ala Leu Leu Ala Pro Trp Ala Gly Arg Gly Gly Ala Ala Ala Pro
            20                25                  30

Thr Ala Pro Asn Gly Thr Leu Glu Ala Glu Leu Glu Arg Arg Trp Glu
          35                40                 45

Ser Leu Val Ala Leu Ser Leu Ala Arg Leu Pro Val Ala Ala Gln Pro
        50              55                60

Lys Glu Ala Ala Val Gln Ser Gly Ala Gly Asp Tyr Leu Leu Gly Ile
 65                 70                 75                    80

Lys Arg Leu Arg Arg Leu Tyr Cys Asn Val Gly Ile Gly Phe His Leu
                85                  90                    95

Gln Ala Leu Pro Asp Gly Arg Ile Gly Gly Ala His Ala Asp Thr Arg
          100                105                 110

Asp Ser Leu Leu Glu Leu Ser Pro Val Glu Arg Gly Val Val Ser Ile
        115                120                 125

Phe Gly Val Ala Ser Arg Phe Phe Val Ala Met Ser Ser Lys Gly Lys
    130             135                 140

Leu Tyr Gly Ser Pro Phe Phe Thr Asp Glu Cys Thr Phe Lys Glu Ile
145             150                 155                 160
```

50

```
Leu Leu Pro Asn Asn Tyr Asn Ala Tyr Glu Ser Tyr Lys Tyr Pro Gly
            165             170             175

Met Phe Ile Ala Leu Ser Lys Asn Gly Lys Thr Lys Lys Gly Asn Arg
            180             185             190

Val Ser Pro Thr Met Lys Val Thr His Phe Leu Pro Arg Leu
            195             200             205
```

<210> 8
<211> 268
<212> PRT
<213> Homo sapiens

<400> 8

```
Met Ser Leu Ser Phe Leu Leu Leu Leu Phe Phe Ser His Leu Ile Leu
  1           5               10                  15

Ser Ala Trp Ala His Gly Glu Lys Arg Leu Ala Pro Lys Gly Gln Pro
            20              25              30

Gly Pro Ala Ala Thr Asp Arg Asn Pro Ile Gly Ser Ser Ser Arg Gln
            35              40              45

Ser Ser Ser Ser Ala Met Ser Ser Ser Ser Ala Ser Ser Ser Pro Ala
        50              55              60

Ala Ser Leu Gly Ser Gln Gly Ser Gly Leu Glu Gln Ser Ser Phe Gln
65              70              75                  80

Trp Ser Pro Ser Gly Arg Arg Thr Gly Ser Leu Tyr Cys Arg Val Gly
            85              90                  95

Ile Gly Phe His Leu Gln Ile Tyr Pro Asp Gly Lys Val Asn Gly Ser
            100             105             110

His Glu Ala Asn Met Leu Ser Val Leu Glu Ile Phe Ala Val Ser Gln
            115             120             125

Gly Ile Val Gly Ile Arg Gly Val Phe Ser Asn Lys Phe Leu Ala Met
    130             135             140

Ser Lys Lys Gly Lys Leu His Ala Ser Ala Lys Phe Thr Asp Asp Cys
145             150             155             160

Lys Phe Arg Glu Arg Phe Gln Glu Asn Ser Tyr Asn Thr Tyr Ala Ser
            165             170             175

Ala Ile His Arg Thr Glu Lys Thr Gly Arg Glu Trp Tyr Val Ala Leu
            180             185             190

Asn Lys Arg Gly Lys Ala Lys Arg Gly Cys Ser Pro Arg Val Lys Pro
            195             200             205

Gln His Ile Ser Thr His Phe Leu Pro Arg Phe Lys Gln Ser Glu Gln
    210             215             220

Pro Glu Leu Ser Phe Thr Val Thr Val Pro Glu Lys Lys Asn Pro Pro
225             230             235             240
```

```
        Ser Pro Ile Lys Ser Lys Ile Pro Leu Ser Ala Pro Arg Lys Asn Thr
                        245             250             255

        Asn Ser Val Lys Tyr Arg Leu Lys Phe Arg Phe Gly
                        260             265
```

```
<210> 9
<211> 208
<212> PRT
<213> Homo sapiens
```

```
<400> 9
Met Ala Leu Gly Gln Lys Leu Phe Ile Thr Met Ser Arg Gly Ala Gly
 1               5                  10                  15

Arg Leu Gln Gly Thr Leu Trp Ala Leu Val Phe Leu Gly Ile Leu Val
                20              25                  30

Gly Met Val Val Pro Ser Pro Ala Gly Thr Arg Ala Asn Asn Thr Leu
            35                  40                  45

Leu Asp Ser Arg Gly Trp Gly Thr Leu Leu Ser Arg Ser Arg Ala Gly
        50                  55                  60

Leu Ala Gly Glu Ile Ala Gly Val Asn Trp Glu Ser Gly Tyr Leu Val
65                  70                  75                      80

Gly Ile Lys Arg Gln Arg Arg Leu Tyr Cys Asn Val Gly Ile Gly Phe
                85                  90                      95

His Leu Gln Val Leu Pro Asp Gly Arg Ile Ser Gly Thr His Glu Glu
                100                 105                 110

Asn Pro Tyr Ser Leu Leu Glu Ile Ser Thr Val Glu Arg Gly Val Val
            115                 120                 125

Ser Leu Phe Gly Val Arg Ser Ala Leu Phe Val Ala Met Asn Ser Lys
        130                 135                 140

Gly Arg Leu Tyr Ala Thr Pro Ser Phe Gln Glu Glu Cys Lys Phe Arg
145                 150                 155                 160

Glu Thr Leu Leu Pro Asn Asn Tyr Asn Ala Tyr Glu Ser Asp Leu Tyr
                165                 170                 175

Gln Gly Thr Tyr Ile Ala Leu Ser Lys Tyr Gly Arg Val Lys Arg Gly
                180                 185                 190

Ser Lys Val Ser Pro Ile Met Thr Val Thr His Phe Leu Pro Arg Ile
            195                 200                 205
```

```
<210> 10
<211> 194
<212> PRT
<213> Homo sapiens
```

```
<400> 10
Met His Lys Trp Ile Leu Thr Trp Ile Leu Pro Thr Leu Leu Tyr Arg
  1               5                  10                  15

Ser Cys Phe His Ile Ile Cys Leu Val Gly Thr Ile Ser Leu Ala Cys
             20                  25                  30

Asn Asp Met Thr Pro Glu Gln Met Ala Thr Asn Val Asn Cys Ser Ser
         35                  40                  45

Pro Glu Arg His Thr Arg Ser Tyr Asp Tyr Met Glu Gly Gly Asp Ile
     50                  55                  60

Arg Val Arg Arg Leu Phe Cys Arg Thr Gln Trp Tyr Leu Arg Ile Asp
 65                  70                  75                  80

Lys Arg Gly Lys Val Lys Gly Thr Gln Glu Met Lys Asn Asn Tyr Asn
             85                  90                  95

Ile Met Glu Ile Arg Thr Val Ala Val Gly Ile Val Ala Ile Lys Gly
            100                 105                 110

Val Glu Ser Glu Phe Tyr Leu Ala Met Asn Lys Glu Gly Lys Leu Tyr
            115                 120                 125

Ala Lys Lys Glu Cys Asn Glu Asp Cys Asn Phe Lys Glu Leu Ile Leu
    130                 135                 140

Glu Asn His Tyr Asn Thr Tyr Ala Ser Ala Lys Trp Thr His Asn Gly
145                 150                 155                 160

Gly Glu Met Phe Val Ala Leu Asn Gln Lys Gly Ile Pro Val Arg Gly
                165                 170                 175

Lys Lys Thr Lys Lys Glu Gln Lys Thr Ala His Phe Leu Pro Met Ala
            180                 185                 190

Ile Thr


<210> 11
<211> 233
<212> PRT
<213> Homo sapiens

<400> 11
Met Gly Ser Pro Arg Ser Ala Leu Ser Cys Leu Leu Leu His Leu Leu
  1               5                  10                  15

Val Leu Cys Leu Gln Ala Gln Glu Gly Pro Gly Arg Gly Pro Ala Leu
             20                  25                  30

Gly Arg Glu Leu Ala Ser Leu Phe Arg Ala Gly Arg Glu Pro Gln Gly
         35                  40                  45

Val Ser Gln Gln His Val Arg Glu Gln Ser Leu Val Thr Asp Gln Leu
     50                  55                  60

Ser Arg Arg Leu Ile Arg Thr Tyr Gln Leu Tyr Ser Arg Thr Ser Gly
 65                  70                  75                  80
```

```
Lys His Val Gln Val Leu Ala Asn Lys Arg Ile Asn Ala Met Ala Glu
                85                  90                  95

Asp Gly Asp Pro Phe Ala Lys Leu Ile Val Glu Thr Asp Thr Phe Gly
            100                 105                 110

Ser Arg Val Arg Val Arg Gly Ala Glu Thr Gly Leu Tyr Ile Cys Met
        115                 120                 125

Asn Lys Lys Gly Lys Leu Ile Ala Lys Ser Asn Gly Lys Gly Lys Asp
    130                 135                 140

Cys Val Phe Thr Glu Ile Val Leu Glu Asn Asn Tyr Thr Ala Leu Gln
145             150                 155                 160

Asn Ala Lys Tyr Glu Gly Trp Tyr Met Ala Phe Thr Arg Lys Gly Arg
            165                 170                 175

Pro Arg Lys Gly Ser Lys Thr Arg Gln His Gln Arg Glu Val His Phe
            180                 185                 190

Met Lys Arg Leu Pro Arg Gly His His Thr Thr Glu Gln Ser Leu Arg
        195                 200                 205

Phe Glu Phe Leu Asn Tyr Pro Pro Phe Thr Arg Ser Leu Arg Gly Ser
    210                 215                 220

Gln Arg Thr Trp Ala Pro Glu Pro Arg
225                 230


<210> 12
<211> 208
<212> PRT
<213> Homo sapiens

<400> 12
Met Ala Pro Leu Gly Glu Val Gly Asn Tyr Phe Gly Val Gln Asp Ala
  1             5                   10                  15

Val Pro Phe Gly Asn Val Pro Val Leu Pro Val Asp Ser Pro Val Leu
            20                  25                  30

Leu Ser Asp His Leu Gly Gln Ser Glu Ala Gly Gly Leu Pro Arg Gly
        35                  40                  45

Pro Ala Val Thr Asp Leu Asp His Leu Lys Gly Ile Leu Arg Arg Arg
    50                  55                  60

Gln Leu Tyr Cys Arg Thr Gly Phe His Leu Glu Ile Phe Pro Asn Gly
65              70                  75                  80

Thr Ile Gln Gly Thr Arg Lys Asp His Ser Arg Phe Gly Ile Leu Glu
            85                  90                  95

Phe Ile Ser Ile Ala Val Gly Leu Val Ser Ile Arg Gly Val Asp Ser
            100                 105                 110

Gly Leu Tyr Leu Gly Met Asn Glu Lys Gly Glu Leu Tyr Gly Ser Glu
        115                 120                 125
```

```
Lys Leu Thr Gln Glu Cys Val Phe Arg Glu Gln Phe Glu Glu Asn Trp
    130                 135             140
Tyr Asn Thr Tyr Ser Ser Asn Leu Tyr Lys His Val Asp Thr Gly Arg
145                 150             155                 160
Arg Tyr Tyr Val Ala Leu Asn Lys Asp Gly Thr Pro Arg Glu Gly Thr
                165             170                 175
Arg Thr Lys Arg His Gln Lys Phe Thr His Phe Leu Pro Arg Pro Val
            180             185             190
Asp Pro Asp Lys Val Pro Glu Leu Tyr Lys Asp Ile Leu Ser Gln Ser
        195             200             205
```

```
<210> 13
<211> 208
<212> PRT
<213> Homo sapiens

<400> 13
Met Trp Lys Trp Ile Leu Thr His Cys Ala Ser Ala Phe Pro His Leu
  1               5                 10                  15
Pro Gly Cys Cys Cys Cys Cys Phe Leu Leu Leu Phe Leu Val Ser Ser
            20              25                  30
Val Pro Val Thr Cys Gln Ala Leu Gly Gln Asp Met Val Ser Pro Glu
            35              40                  45
Ala Thr Asn Ser Ser Ser Ser Ser Phe Ser Ser Pro Ser Ser Ala Gly
        50              55              60
Arg His Val Arg Ser Tyr Asn His Leu Gln Gly Asp Val Arg Trp Arg
 65             70              75                  80
Lys Leu Phe Ser Phe Thr Lys Tyr Phe Leu Lys Ile Glu Lys Asn Gly
                85              90                  95
Lys Val Ser Gly Thr Lys Lys Glu Asn Cys Pro Tyr Ser Ile Leu Glu
            100             105                 110
Ile Thr Ser Val Glu Ile Gly Val Val Ala Val Lys Ala Ile Asn Ser
            115             120                 125
Asn Tyr Tyr Leu Ala Met Asn Lys Lys Gly Lys Leu Tyr Gly Ser Lys
    130             135                 140
Glu Phe Asn Asn Asp Cys Lys Leu Lys Glu Arg Ile Glu Glu Asn Gly
145                 150             155                 160
Tyr Asn Thr Tyr Ala Ser Phe Asn Trp Gln His Asn Gly Arg Gln Met
                165             170                 175
Tyr Val Ala Leu Asn Gly Lys Gly Ala Pro Arg Arg Gly Gln Lys Thr
            180             185                 190
```

Arg Arg Lys Asn Thr Ser Ala His Phe Leu Pro Met Val Val His Ser
     195           200          205

<210> 14
<211> 225
<212> PRT
<213> Homo sapiens

<400> 14
Met Ala Ala Leu Ala Ser Ser Leu Ile Arg Gln Lys Arg Glu Val Arg
  1         5         10          15

Glu Pro Gly Gly Ser Arg Pro Val Ser Ala Gln Arg Arg Val Cys Pro
       20          25          30

Arg Gly Thr Lys Ser Leu Cys Gln Lys Gln Leu Leu Ile Leu Leu Ser
     35           40          45

Lys Val Arg Leu Cys Gly Gly Arg Pro Ala Arg Pro Asp Arg Gly Pro
    50           55          60

Glu Pro Gln Leu Lys Gly Ile Val Thr Lys Leu Phe Cys Arg Gln Gly
 65          70         75          80

Phe Tyr Leu Gln Ala Asn Pro Asp Gly Ser Ile Gln Gly Thr Pro Glu
       85          90          95

Asp Thr Ser Ser Phe Thr His Phe Asn Leu Ile Pro Val Gly Leu Arg
      100         105        110

Val Val Thr Ile Gln Ser Ala Lys Leu Gly His Tyr Met Ala Met Asn
     115          120        125

Ala Glu Gly Leu Leu Tyr Ser Ser Pro His Phe Thr Ala Glu Cys Arg
   130         135        140

Phe Lys Glu Cys Val Phe Glu Asn Tyr Tyr Val Leu Tyr Ala Ser Ala
145          150        155        160

Leu Tyr Arg Gln Arg Arg Ser Gly Arg Ala Trp Tyr Leu Gly Leu Asp
      165        170        175

Lys Glu Gly Gln Val Met Lys Gly Asn Arg Val Lys Lys Thr Lys Ala
      180        185        190

Ala Ala His Phe Leu Pro Lys Leu Leu Glu Val Ala Met Tyr Gln Glu
     195          200        205

Pro Ser Leu His Ser Val Pro Glu Ala Ser Pro Ser Ser Pro Pro Ala
     210          215        220

Pro
225

<210> 15

```
<211> 243
<212> PRT
<213> Homo sapiens

<400> 15
Met Ala Ala Ala Ile Ala Ser Ser Leu Ile Arg Gln Lys Arg Gln Ala
  1               5                  10                  15

Arg Glu Ser Asn Ser Asp Arg Val Ser Ala Ser Lys Arg Arg Ser Ser
             20                  25                  30

Pro Ser Lys Asp Gly Arg Ser Leu Cys Glu Arg His Val Leu Gly Val
         35                  40                  45

Phe Ser Lys Val Arg Phe Cys Ser Gly Arg Lys Arg Pro Val Arg Arg
     50                  55                  60

Arg Pro Glu Pro Gln Leu Lys Gly Ile Val Thr Arg Leu Phe Ser Gln
 65                  70                  75                  80

Gln Gly Tyr Phe Leu Gln Met His Pro Asp Gly Thr Ile Asp Gly Thr
             85                  90                  95

Lys Asp Glu Asn Ser Asp Tyr Thr Leu Phe Asn Leu Ile Pro Val Gly
            100                 105                 110

Leu Arg Val Val Ala Ile Gln Gly Val Lys Ala Ser Leu Tyr Val Ala
            115                 120                 125

Met Asn Gly Glu Gly Tyr Leu Tyr Ser Ser Asp Val Phe Thr Pro Glu
        130                 135                 140

Cys Lys Phe Lys Glu Ser Val Phe Glu Asn Tyr Tyr Val Ile Tyr Ser
145                 150                 155                 160

Ser Thr Leu Tyr Arg Gln Gln Glu Ser Gly Arg Ala Trp Phe Leu Gly
                165                 170                 175

Leu Asn Lys Glu Gly Gln Ile Met Lys Gly Asn Arg Val Lys Lys Thr
                180                 185                 190

Lys Pro Ser Ser His Phe Val Pro Lys Pro Ile Glu Val Cys Met Tyr
            195                 200                 205

Arg Glu Pro Ser Leu His Glu Ile Gly Glu Lys Gln Gly Arg Ser Arg
        210                 215                 220

Lys Ser Ser Gly Thr Pro Thr Met Asn Gly Gly Lys Val Val Asn Gln
225                 230                 235                 240

Asp Ser Thr


<210> 16
<211> 245
<212> PRT
<213> Homo sapiens

<400> 16
Met Ala Ala Ala Ile Ala Ser Ser Leu Ile Arg Gln Lys Arg Gln Ala
```

```
                1               5                       10                      15

        Arg Glu Arg Glu Lys Ser Asn Ala Cys Lys Cys Val Ser Ser Pro Ser
                    20              25              30

        Lys Gly Lys Thr Ser Cys Asp Lys Asn Lys Leu Asn Val Phe Ser Arg
                    35              40              45

        Val Lys Leu Phe Gly Ser Lys Lys Arg Arg Arg Arg Pro Glu Pro
                    50              55              60

        Gln Leu Lys Gly Ile Val Thr Lys Leu Tyr Ser Arg Gln Gly Tyr His
        65                  70              75                      80

        Leu Gln Leu Gln Ala Asp Gly Thr Ile Asp Gly Thr Lys Asp Glu Asp
                        85              90                      95

        Ser Thr Tyr Thr Leu Phe Asn Leu Ile Pro Val Gly Leu Arg Val Val
                    100             105             110

        Ala Ile Gln Gly Val Gln Thr Lys Leu Tyr Leu Ala Met Asn Ser Glu
                115             120             125

        Gly Tyr Leu Tyr Thr Ser Glu Leu Phe Thr Pro Glu Cys Lys Phe Lys
            130             135             140

        Glu Ser Val Phe Glu Asn Tyr Tyr Val Thr Tyr Ser Ser Met Ile Tyr
        145             150             155             160

        Arg Gln Gln Gln Ser Gly Arg Gly Trp Tyr Leu Gly Leu Asn Lys Glu
                    165             170             175

        Gly Glu Ile Met Lys Gly Asn His Val Lys Lys Asn Lys Pro Ala Ala
                    180             185             190

        His Phe Leu Pro Lys Pro Leu Lys Val Ala Met Tyr Lys Glu Pro Ser
                195             200             205

        Leu His Asp Leu Thr Glu Phe Ser Arg Ser Gly Ser Gly Thr Pro Thr
            210             215             220

        Lys Ser Arg Ser Val Ser Gly Val Leu Asn Gly Gly Lys Ser Met Ser
        225                 230             235             240

        His Asn Glu Ser Thr
                        245


        <210> 17
        <211> 247
        <212> PRT
        <213> Homo sapiens

        <400> 17
        Met Ala Ala Ala Ile Ala Ser Gly Leu Ile Arg Gln Lys Arg Gln Ala
        1               5                       10                      15

        Arg Glu Gln His Trp Asp Arg Pro Ser Ala Ser Arg Arg Arg Ser Ser
                    20              25              30

        Pro Ser Lys Asn Arg Gly Leu Cys Asn Gly Asn Leu Val Asp Ile Phe
```

```
                    35                      40                      45
        Ser Lys Val Arg Ile Phe Gly Leu Lys Lys Arg Arg Leu Arg Arg Gln
            50                  55                  60

        Asp Pro Gln Leu Lys Gly Ile Val Thr Arg Leu Tyr Cys Arg Gln Gly
        65                  70                  75                  80

        Tyr Tyr Leu Gln Met His Pro Asp Gly Ala Leu Asp Gly Thr Lys Asp
                        85                  90                  95

        Asp Ser Thr Asn Ser Thr Leu Phe Asn Leu Ile Pro Val Gly Leu Arg
                    100                 105                 110

        Val Val Ala Ile Gln Gly Val Lys Thr Gly Leu Tyr Ile Ala Met Asn
                115                 120                 125

        Gly Glu Gly Tyr Leu Tyr Pro Ser Glu Leu Phe Thr Pro Glu Cys Lys
            130                 135                 140

        Phe Lys Glu Ser Val Phe Glu Asn Tyr Tyr Val Ile Tyr Ser Ser Met
        145                 150                 155                 160

        Leu Tyr Arg Gln Gln Glu Ser Gly Arg Ala Trp Phe Leu Gly Leu Asn
                    165                 170                 175    .

        Lys Glu Gly Gln Ala Met Lys Gly Asn Arg Val Lys Lys Thr Lys Pro
                    180                 185                 190

        Ala Ala His Phe Leu Pro Lys Pro Leu Glu Val Ala Met Tyr Arg Glu
                    195                 200                 205

        Pro Ser Leu His Asp Val Gly Glu Thr Val Pro Lys Pro Gly Val Thr
            210                 215                 220

        Pro Ser Lys Ser Thr Ser Ala Ser Ala Ile Met Asn Gly Gly Lys Pro
        225                 230                 235                 240

        Val Asn Lys Ser Lys Thr Thr
                    245


        <210> 18
        <211> 207
        <212> PRT
        <213> Homo sapiens

        <400> 18
        Met Ala Glu Val Gly Gly Val Phe Ala Ser Leu Asp Trp Asp Leu His
            1                   5                   10                  15

        Gly Phe Ser Ser Ser Leu Gly Asn Val Pro Leu Ala Asp Ser Pro Gly
                    20                  25                  30

        Phe Leu Asn Glu Arg Leu Gly Gln Ile Glu Gly Lys Leu Gln Arg Gly
                    35                  40                  45

        Ser Pro Thr Asp Phe Ala His Leu Lys Gly Ile Leu Arg Arg Arg Gln
            50                  55                  60

        Leu Tyr Cys Arg Thr Gly Phe His Leu Glu Ile Phe Pro Asn Gly Thr
```

```
                65                    70                    75                    80

        Val His Gly Thr Arg His Asp His Ser Arg Phe Gly Ile Leu Glu Phe
                        85                    90                    95

        Ile Ser Leu Ala Val Gly Leu Ile Ser Ile Arg Gly Val Asp Ser Gly
                        100                   105                   110

        Leu Tyr Leu Gly Met Asn Glu Arg Gly Glu Leu Tyr Gly Ser Lys Lys
                    115                   120                   125

        Leu Thr Arg Glu Cys Val Phe Arg Glu Gln Phe Glu Glu Asn Trp Tyr
                130                   135                   140

        Asn Thr Tyr Ala Ser Thr Leu Tyr Lys His Ser Asp Ser Glu Arg Gln
        145                   150                   155                   160

        Tyr Tyr Val Ala Leu Asn Lys Asp Gly Ser Pro Arg Glu Gly Tyr Arg
                        165                   170                   175

        Thr Lys Arg His Gln Lys Phe Thr His Phe Leu Pro Arg Pro Val Asp
                    180                   185                   190

        Pro Ser Lys Leu Pro Ser Met Ser Arg Asp Leu Phe His Tyr Arg
                    195                   200                   205


        <210> 19
        <211> 207
        <212> PRT
        <213> Homo sapiens


        <400> 19
        Met Tyr Ser Ala Pro Ser Ala Cys Thr Cys Leu Cys Leu His Phe Leu
            1               5                   10                    15

        Leu Leu Cys Phe Gln Val Gln Val Leu Val Ala Glu Glu Asn Val Asp
                    20                    25                    30

        Phe Arg Ile His Val Glu Asn Gln Thr Arg Ala Arg Asp Asp Val Ser
                    35                    40                    45

        Arg Lys Gln Leu Arg Leu Tyr Gln Leu Tyr Ser Arg Thr Ser Gly Lys
                50                    55                    60

        His Ile Gln Val Leu Gly Arg Arg Ile Ser Ala Arg Gly Glu Asp Gly
                65                    70                    75                    80

        Asp Lys Tyr Ala Gln Leu Leu Val Glu Thr Asp Thr Phe Gly Ser Gln
                        85                    90                    95

        Val Arg Ile Lys Gly Lys Glu Thr Glu Phe Tyr Leu Cys Met Asn Arg
                    100                   105                   110

        Lys Gly Lys Leu Val Gly Lys Pro Asp Gly Thr Ser Lys Glu Cys Val
                    115                   120                   125

        Phe Ile Glu Lys Val Leu Glu Asn Asn Tyr Thr Ala Leu Met Ser Ala
                130                   135                   140

        Lys Tyr Ser Gly Trp Tyr Val Gly Phe Thr Lys Lys Gly Arg Pro Arg
```

```
                145                    150                    155                    160

Lys Gly Pro Lys Thr Arg Glu Asn Gln Gln Asp Val His Phe Met Lys
                165                    170                    175

Arg Tyr Pro Lys Gly Gln Pro Glu Leu Gln Lys Pro Phe Lys Tyr Thr
                180                    185                    190

Thr Val Thr Lys Arg Ser Arg Arg Ile Arg Pro Thr His Pro Ala
                195                    200                    205


<210> 20
<211> 216
<212> PRT
<213> Homo sapiens

<400> 20
Met Arg Ser Gly Cys Val Val Val His Val Trp Ile Leu Ala Gly Leu
  1               5                   10                     15

Trp Leu Ala Val Ala Gly Arg Pro Leu Ala Phe Ser Asp Ala Gly Pro
                20                    25                     30

His Val His Tyr Gly Trp Gly Asp Pro Ile Arg Leu Arg His Leu Tyr
                35                    40                     45

Thr Ser Gly Pro His Gly Leu Ser Ser Cys Phe Leu Arg Ile Arg Ala
        50                    55                    60

Asp Gly Val Val Asp Cys Ala Arg Gly Gln Ser Ala His Ser Leu Leu
 65                    70                    75                     80

Glu Ile Lys Ala Val Ala Leu Arg Thr Val Ala Ile Lys Gly Val His
                85                    90                     95

Ser Val Arg Tyr Leu Cys Met Gly Ala Asp Gly Lys Met Gln Gly Leu
                100                   105                    110

Leu Gln Tyr Ser Glu Glu Asp Cys Ala Phe Glu Glu Glu Ile Arg Pro
        115                   120                    125

Asp Gly Tyr Asn Val Tyr Arg Ser Glu Lys His Arg Leu Pro Val Ser
        130                   135                    140

Leu Ser Ser Ala Lys Gln Arg Gln Leu Tyr Lys Asn Arg Gly Phe Leu
145                   150                    155                    160

Pro Leu Ser His Phe Leu Pro Met Leu Pro Met Val Pro Glu Glu Pro
                165                   170                    175

Glu Asp Leu Arg Gly His Leu Glu Ser Asp Met Phe Ser Ser Pro Leu
                180                   185                    190

Glu Thr Asp Ser Met Asp Pro Phe Gly Leu Val Thr Gly Leu Glu Ala
        195                   200                    205

Val Arg Ser Pro Ser Phe Glu Lys
        210                   215
```

61

```
<210> 21
<211> 233
<212> PRT
<213> Homo sapiens

<400> 21
Met Ser Val Leu Arg Ala Tyr Pro Asn Ala Ser Pro Leu Leu Gly Ser
  1               5                  10                  15

Ser Trp Gly Gly Leu Ile His Leu Tyr Thr Ala Thr Ala Arg Asn Ser
             20                  25                  30

Tyr His Leu Gln Ile His Lys Asn Gly His Val Asp Gly Ala Pro His
         35                  40                  45

Gln Thr Ile Tyr Ser Ala Leu Met Ile Arg Ser Glu Asp Ala Gly Phe
         50                  55                  60

Val Val Ile Thr Gly Val Met Ser Arg Arg Tyr Leu Cys Met Asp Phe
 65                  70                  75                  80

Arg Gly Asn Ile Phe Gly Ser His Tyr Phe Asp Pro Glu Asn Cys Arg
                 85                  90                  95

Phe Gln His Gln Thr Leu Glu Asn Gly Tyr Asp Val Tyr His Ser Pro
            100                 105                 110

Gln Tyr His Phe Leu Val Ser Leu Gly Arg Ala Lys Arg Ala Phe Leu
            115                 120                 125

Pro Gly Met Asn Pro Pro Tyr Ser Gln Phe Leu Ser Arg Arg Asn
            130                 135                 140

Glu Ile Pro Leu Ile His Phe Asn Thr Pro Ile Pro Arg Arg His Thr
145                 150                 155                 160

Arg Ser Ala Glu Asp Asp Ser Glu Arg Asp Pro Leu Asn Val Leu Lys
                165                 170                 175

Pro Arg Ala Arg Met Thr Pro Ala Pro Ala Ser Cys Ser Gln Glu Leu
                180                 185                 190

Pro Ser Ala Glu Asp Asn Ser Pro Met Ala Ser Asp Pro Leu Gly Val
            195                 200                 205

Val Arg Gly Gly Arg Val Asn Thr His Ala Gly Gly Thr Gly Pro Glu
            210                 215                 220

Gly Cys Arg Pro Phe Ala Lys Phe Ile
225                 230

<210> 22
<211> 155
<212> PRT
<213> Mus musculus

<400> 22
Met Ala Glu Gly Glu Ile Thr Thr Phe Ala Ala Leu Thr Glu Arg Phe
  1               5                  10                  15
```

```
Asn Leu Pro Leu Gly Asn Tyr Lys Lys Pro Lys Leu Leu Tyr Cys Ser
            20                  25              30

Asn Gly Gly His Phe Leu Arg Ile Leu Pro Asp Gly Thr Val Asp Gly
        35                  40              45

Thr Arg Asp Arg Ser Asp Gln His Ile Gln Leu Gln Leu Ser Ala Glu
    50                  55                  60

Ser Ala Gly Glu Val Tyr Ile Lys Gly Thr Glu Thr Gly Gln Tyr Leu
65                  .70                  75                      80

Ala Met Asp Thr Glu Gly Leu Leu Tyr Gly Ser Gln Thr Pro Asn Glu
                85                  90                  95      .

Glu Cys Leu Phe Leu Glu Arg Leu Glu Glu Asn His Tyr Asn Thr Tyr
            100                 105             110

Thr Ser Lys Lys His Ala Glu Lys Asn Trp Phe Val Gly Leu Lys Lys
        115                 120             125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155
```

```
<210> 23
<211> 154
<212> PRT
<213> Mus musculus
```

```
<400> 23
Met Ala Ala Ser Gly Ile Thr Ser Leu Pro Ala Leu Pro Glu Asp Gly
    1               5                   10                  15

Gly Ala Ala Phe Pro Pro Gly His Phe Lys Asp Pro Lys Arg Leu Tyr
            20                  25              30

Cys Lys Asn Gly Gly Phe Phe Leu Arg Ile His Pro Asp Gly Arg Val
        35                  40              45

Asp Gly Val Arg Glu Lys Ser Asp Pro His Val Lys Leu Gln Leu Gln
    50                  55                  60

Ala Glu Glu Arg Gly Val Val Ser Ile Lys Gly Val Cys Ala Asn Arg
65                  70                  75                      80

Tyr Leu Ala Met Lys Glu Asp Gly Arg Leu Leu Ala Ser Lys Cys Val
                85                  90                  95

Thr Glu Glu Cys Phe Phe Phe Glu Arg Leu Glu Ser Asn Asn Tyr Asn
            100                 105             110

Thr Tyr Arg Ser Arg Lys Tyr Ser Ser Trp Tyr Val Ala Leu Lys Arg
        115                 120             125

Thr Gly Gln Tyr Lys Leu Gly Ser Lys Thr Gly Pro Gly Gln Lys Ala
    130                 135                 140
```

```
Ile Leu Phe Leu Pro Met Ser Ala Lys Ser
145                 150
```

<210> 24
<211> 245
<212> PRT
<213> Mus musculus

<400> 24

```
Met Gly Leu Ile Trp Leu Leu Leu Leu Ser Leu Leu Glu Pro Ser Trp
  1              5                 10                 15

Pro Thr Thr Gly Pro Gly Thr Arg Leu Arg Arg Asp Ala Gly Gly Arg
            20                 25                 30

Gly Gly Val Tyr Glu His Leu Gly Gly Ala Pro Arg Arg Arg Lys Leu
        35                 40                 45

Tyr Cys Ala Thr Lys Tyr His Leu Gln Leu His Pro Ser Gly Arg Val
    50                 55                 60

Asn Gly Ser Leu Glu Asn Ser Ala Tyr Ser Ile Leu Glu Ile Thr Ala
65                 70                 75                 80

Val Glu Val Gly Val Val Ala Ile Lys Gly Leu Phe Ser Gly Arg Tyr
                85                 90                 95

Leu Ala Met Asn Lys Arg Gly Arg Leu Tyr Ala Ser Asp His Tyr Asn
            100                105                110

Ala Glu Cys Glu Phe Val Glu Arg Ile His Glu Leu Gly Tyr Asn Thr
            115                120                125

Tyr Ala Ser Arg Leu Tyr Arg Thr Gly Ser Ser Gly Pro Gly Ala Gln
    130                135                140

Arg Gln Pro Gly Ala Gln Arg Pro Trp Tyr Val Ser Val Asn Gly Lys
145                150                155                160

Gly Arg Pro Arg Arg Gly Phe Lys Thr Arg Arg Thr Gln Lys Ser Ser
            165                170                175

Leu Phe Leu Pro Arg Val Leu Gly His Lys Asp His Glu Met Val Arg
            180                185                190

Leu Leu Gln Ser Ser Gln Pro Arg Ala Pro Gly Glu Gly Ser Gln Pro
            195                200                205

Arg Gln Arg Arg Gln Lys Lys Gln Ser Pro Gly Asp His Gly Lys Met
    210                215                220

Glu Thr Leu Ser Thr Arg Ala Thr Pro Ser Thr Gln Leu His Thr Gly
225                230                235                240

Gly Leu Ala Val Ala
                245
```

<210> 25
<211> 202

64

```
<212> PRT
<213> Mus musculus

<400> 25
Met Ala Lys Arg Gly Pro Thr Thr Gly Thr Leu Leu Pro Arg Val Leu
  1               5                  10                  15

Leu Ala Leu Val Val Ala Leu Ala Asp Arg Gly Thr Ala Ala Pro Asn
             20                  25                  30

Gly Thr Arg His Ala Glu Leu Gly His Gly Trp Asp Gly Leu Val Ala
         35                  40                  45

Arg Ser Leu Ala Arg Leu Pro Val Ala Ala Gln Pro Pro Gln Ala Ala
     50                  55                  60

Val Arg Ser Gly Ala Gly Asp Tyr Leu Leu Gly Leu Lys Arg Leu Arg
 65                  70                  75                      80

Arg Leu Tyr Cys Asn Val Gly Ile Gly Phe His Leu Gln Val Leu Pro
             85                  90                      95

Asp Gly Arg Ile Gly Gly Val His Ala Asp Thr Arg Asp Ser Leu Leu
             100                 105                 110

Glu Leu Ser Pro Val Gln Arg Gly Val Val Ser Ile Phe Gly Val Ala
         115                 120                 125

Ser Arg Phe Phe Val Ala Met Ser Ser Arg Gly Lys Leu Phe Gly Val
     130                 135                 140

Pro Phe Phe Thr Asp Glu Cys Lys Phe Lys Glu Ile Leu Leu Pro Asn
145                 150                 155                 160

Asn Tyr Asn Ala Tyr Glu Ala Tyr Ala Tyr Pro Gly Met Phe Met Ala
             165                 170                 175

Leu Ser Lys Asn Gly Arg Thr Lys Lys Gly Asn Arg Val Ser Pro Thr
             180                 185                 190

Met Lys Val Thr His Phe Leu Pro Arg Leu
         195                 200


<210> 26
<211> 264
<212> PRT
<213> Mus musculus

<400> 26
Met Ser Leu Ser Leu Leu Phe Leu Ile Phe Cys Ser His Leu Ile His
  1               5                  10                  15

Ser Ala Trp Ala His Gly Glu Lys Arg Leu Thr Pro Glu Gly Gln Pro
             20                  25                  30

Ala Pro Pro Arg Asn Pro Gly Asp Ser Ser Gly Ser Arg Gly Arg Ser
         35                  40                  45

Ser Ala Thr Phe Ser Ser Ser Ser Ala Ser Ser Pro Val Ala Ala Ser
     50                  55                  60
```

Pro Gly Ser Gln Gly Ser Gly Ser Glu His Ser Ser Phe Gln Trp Ser
65                70                75                80

Pro Ser Gly Arg Arg Thr Gly Ser Leu Tyr Cys Arg Val Gly Ile Gly
                85                90                95

Phe His Leu Gln Ile Tyr Pro Asp Gly Lys Val Asn Gly Ser His Glu
            100               105               110

Ala Ser Val Leu Ser Ile Leu Glu Ile Phe Ala Val Ser Gln Gly Ile
        115               120               125

Val Gly Ile Arg Gly Val Phe Ser Asn Lys Phe Leu Ala Met Ser Lys
    130               135               140

Lys Gly Lys Leu His Ala Ser Ala Lys Phe Thr Asp Asp Cys Lys Phe
145               150               155               160

Arg Glu Arg Phe Gln Glu Asn Ser Tyr Asn Thr Tyr Ala Ser Ala Ile
            165               170               175

His Arg Thr Glu Lys Thr Gly Arg Glu Trp Tyr Val Ala Leu Asn Lys
            180               185               190

Arg Gly Lys Ala Lys Arg Gly Cys Ser Pro Arg Val Lys Pro Gln His
        195               200               205

Val Ser Thr His Phe Leu Pro Arg Phe Lys Gln Ser Glu Gln Pro Glu
    210               215               220

Leu Ser Phe Thr Val Thr Val Pro Glu Lys Lys Lys Pro Pro Val Lys
225               230               235               240

Pro Lys Val Pro Leu Ser Gln Pro Arg Arg Ser Pro Ser Pro Val Lys
                245               250               255

Tyr Arg Leu Lys Phe Arg Phe Gly
            260

<210> 27
<211> 208
<212> PRT
<213> Mus musculus

<400> 27
Met Ala Leu Gly Gln Arg Leu Phe Ile Thr Met Ser Arg Gly Ala Gly
    1                5                10               15

Arg Val Gln Gly Thr Leu Gln Ala Leu Val Phe Leu Gly Val Leu Val
            20                25                30

Gly Met Val Val Pro Ser Pro Ala Gly Ala Arg Ala Asn Gly Thr Leu
            35                40                45

Leu Asp Ser Arg Gly Trp Gly Thr Leu Leu Ser Arg Ser Arg Ala Gly
        50                55                60

Leu Ala Gly Glu Ile Ser Gly Val Asn Trp Glu Ser Gly Tyr Leu Val
    65                70                75                80

```
Gly Ile Lys Arg Gln Arg Arg Leu Tyr Cys Asn Val Gly Ile Gly Phe
                85                  90                  95

His Leu Gln Val Pro Pro Asp Gly Arg Ile Ser Gly Thr His Glu Glu
            100                 105                 110

Asn Pro Tyr Ser Leu Leu Glu Ile Ser Thr Val Glu Arg Gly Val Val
            115                 120                 125

Ser Leu Phe Gly Val Lys Ser Ala Leu Phe Ile Ala Met Asn Ser Lys
        130                 135                 140

Gly Arg Leu Tyr Thr Thr Pro Ser Phe His Asp Glu Cys Lys Phe Arg
145                 150                 155                 160

Glu Thr Leu Leu Pro Asn Asn Tyr Asn Ala Tyr Glu Ser Asp Leu Tyr
                165                 170                 175

Arg Gly Thr Tyr Ile Ala Leu Ser Lys Tyr Gly Arg Val Lys Arg Gly
            180                 185                 190

Ser Lys Val Ser Pro Ile Met Thr Val Thr His Phe Leu Pro Arg Ile
        195                 200                 205
```

```
<210> 28
<211> 194
<212> PRT
<213> Mus musculus

<400> 28
Met Arg Lys Trp Ile Leu Thr Arg Ile Leu Pro Thr Leu Leu Tyr Arg
  1           5                  10                  15

Ser Cys Phe His Leu Val Cys Leu Val Gly Thr Ile Ser Leu Ala Cys
            20                  25                  30

Asn Asp Met Ser Pro Glu Gln Thr Ala Thr Ser Val Asn Cys Ser Ser
            35                  40                  45

Pro Glu Arg His Thr Arg Ser Tyr Asp Tyr Met Glu Gly Gly Asp Ile
        50                  55                  60

Arg Val Arg Arg Leu Phe Cys Arg Thr Gln Trp Tyr Leu Arg Ile Asp
65                  70                  75                  80

Lys Arg Gly Lys Val Lys Gly Thr Gln Glu Met Lys Asn Ser Tyr Asn
            85                  90                  95

Ile Met Glu Ile Arg Thr Val Ala Val Gly Ile Val Ala Ile Lys Gly
            100                 105                 110

Val Glu Ser Glu Tyr Tyr Leu Ala Met Asn Lys Glu Gly Lys Leu Tyr
            115                 120                 125

Ala Lys Lys Glu Cys Asn Glu Asp Cys Asn Phe Lys Glu Leu Ile Leu
        130                 135                 140
```

Glu Asn His Tyr Asn Thr Tyr Ala Ser Ala Lys Trp Thr His Ser Gly
145                 150             155                 160

Gly Glu Met Phe Val Ala Leu Asn Gln Lys Gly Ile Pro Val Lys Gly
                165             170             175

Lys Lys Thr Lys Lys Glu Gln Lys Thr Ala His Phe Leu Pro Met Ala
            180             185             190

Ile Thr


<210> 29
<211> 268
<212> PRT
<213> Mus musculus

<400> 29
Met Gly Ser Pro Arg Ser Ala Leu Ser Cys Leu Leu Leu His Leu Leu
  1             5                 10                  15

Val Leu Cys Leu Gln Ala Gln Val Arg Ser Ala Ala Gln Lys Arg Gly
            20              25              30

Pro Gly Ala Gly Asn Pro Ala Asp Thr Leu Gly Gln Gly His Glu Asp
            35              40              45

Arg Pro Phe Gly Gln Arg Ser Arg Ala Gly Lys Asn Phe Thr Asn Pro
        50              55              60

Ala Pro Asn Tyr Pro Glu Glu Gly Ser Lys Glu Gln Arg Asp Ser Val
65              70              75              80

Leu Pro Lys Val Thr Gln Arg His Val Arg Glu Gln Ser Leu Val Thr
                85              90              95

Asp Gln Leu Ser Arg Arg Leu Ile Arg Thr Tyr Gln Leu Tyr Ser Arg
            100             105             110

Thr Ser Gly Lys His Val Gln Val Leu Ala Asn Lys Arg Ile Asn Ala
        115             120             125

Met Ala Glu Asp Gly Asp Pro Phe Ala Lys Leu Ile Val Glu Thr Asp
    130             135             140

Thr Phe Gly Ser Arg Val Arg Val Arg Gly Ala Glu Thr Gly Leu Tyr
145             150             155             160

Ile Cys Met Asn Lys Lys Gly Lys Leu Ile Ala Lys Ser Asn Gly Lys
            165             170             175

Gly Lys Asp Cys Val Phe Thr Glu Ile Val Leu Glu Asn Asn Tyr Thr
            180             185             190

Ala Leu Gln Asn Ala Lys Tyr Glu Gly Trp Tyr Met Ala Phe Thr Arg
        195             200             205

Lys Gly Arg Pro Arg Lys Gly Ser Lys Thr Arg Gln His Gln Arg Glu
    210             215             220

```
Val His Phe Met Lys Arg Leu Pro Arg Gly His His Thr Thr Glu Gln
225             230             235             240

Ser Leu Arg Phe Glu Phe Leu Asn Tyr Pro Pro Phe Thr Arg Ser Leu
            245             250             255

Arg Gly Ser Gln Arg Thr Trp Ala Pro Glu Pro Arg
            260             265


<210> 30
<211> 208
<212> PRT
<213> Mus musculus

<400> 30
Met Ala Pro Leu Gly Glu Val Gly Ser Tyr Phe Gly Val Gln Asp Ala
  1           5               10              15

Val Pro Phe Gly Asn Val Pro Val Leu Pro Val Asp Ser Pro Val Leu
            20              25              30

Leu Asn Asp His Leu Gly Gln Ser Glu Ala Gly Gly Leu Pro Arg Gly
            35              40              45

Pro Ala Val Thr Asp Leu Asp His Leu Lys Gly Ile Leu Arg Arg Arg
            50              55              60

Gln Leu Tyr Cys Arg Thr Gly Phe His Leu Glu Ile Phe Pro Asn Gly
 65              70              75              80

Thr Ile Gln Gly Thr Arg Lys Asp His Ser Arg Phe Gly Ile Leu Glu
                85              90              95

Phe Ile Ser Ile Ala Val Gly Leu Val Ser Ile Arg Gly Val Asp Ser
            100             105             110

Gly Leu Tyr Leu Gly Met Asn Glu Lys Gly Glu Leu Tyr Gly Ser Glu
            115             120             125

Lys Leu Thr Gln Glu Cys Val Phe Arg Glu Gln Phe Glu Glu Asn Trp
            130             135             140

Tyr Asn Thr Tyr Ser Ser Asn Leu Tyr Lys His Val Asp Thr Gly Arg
145             150             155             160

Arg Tyr Tyr Val Ala Leu Asn Lys Asp Gly Thr Pro Arg Glu Gly Thr
            165             170             175

Arg Thr Lys Arg His Gln Lys Phe Thr His Phe Leu Pro Arg Pro Val
            180             185             190

Asp Pro Asp Lys Val Pro Glu Leu Tyr Lys Asp Ile Leu Ser Gln Ser
            195             200             205


<210> 31
```

```
<211> 209
<212> PRT
<213> Mus musculus

<400> 31
Met Trp Lys Trp Ile Leu Thr His Cys Ala Ser Ala Phe Pro His Leu
1               5               10              15

Pro Gly Cys Cys Cys Cys Phe Leu Leu Leu Phe Leu Val Ser Ser Phe
            20              25              30

Pro Val Thr Cys Gln Ala Leu Gly Gln Asp Met Val Ser Gln Glu Ala
        35              40              45

Thr Asn Cys Ser Ser Ser Ser Ser Ser Phe Ser Ser Pro Ser Ser Ala
        50              55              60

Gly Arg His Val Arg Ser Tyr Asn His Leu Gln Gly Asp Val Arg Trp
65              70              75              80

Arg Arg Leu Phe Ser Phe Thr Lys Tyr Phe Leu Thr Ile Glu Lys Asn
            85              90              95

Gly Lys Val Ser Gly Thr Lys Asn Glu Asp Cys Pro Tyr Ser Val Leu
            100             105             110

Glu Ile Thr Ser Val Glu Ile Gly Val Val Ala Val Lys Ala Ile Asn
            115             120             125

Ser Asn Tyr Tyr Leu Ala Met Asn Lys Lys Gly Lys Leu Tyr Gly Ser
        130             135             140

Lys Glu Phe Asn Asn Asp Cys Lys Leu Lys Glu Arg Ile Glu Glu Asn
145             150             155             160

Gly Tyr Asn Thr Tyr Ala Ser Phe Asn Trp Gln His Asn Gly Arg Gln
            165             170             175

Met Tyr Val Ala Leu Asn Gly Lys Gly Ala Pro Arg Arg Gly Gln Lys
            180             185             190

Thr Arg Arg Lys Asn Thr Ser Ala His Phe Leu Pro Met Thr Ile Gln
            195             200             205

Thr


<210> 32
<211> 225
<212> PRT
<213> Mus musculus

<400> 32
Met Ala Ala Leu Ala Ser Ser Leu Ile Arg Gln Lys Arg Glu Val Arg
1               5               10              15

Glu Pro Gly Gly Ser Arg Pro Val Ser Ala Gln Arg Arg Val Cys Pro
            20              25              30

Arg Gly Thr Lys Ser Leu Cys Gln Lys Gln Leu Leu Ile Leu Leu Ser
```

```
                  35                    40                    45

        Lys Val Arg Leu Cys Gly Gly Arg Pro Thr Arg Gln Asp Arg Gly Pro
             50                  55                  60

        Glu Pro Gln Leu Lys Gly Ile Val Thr Lys Leu Phe Cys Arg Gln Gly
        65                  70                  75                  80

        Phe Tyr Leu Gln Ala Asn Pro Asp Gly Ser Ile Gln Gly Thr Pro Glu
                         85                  90                  95

        Asp Thr Ser Ser Phe Thr His Phe Asn Leu Ile Pro Val Gly Leu Arg
                     100                 105                 110

        Val Val Thr Ile Gln Ser Ala Lys Leu Gly His Tyr Met Ala Met Asn
                 115                 120                 125

        Ala Glu Gly Leu Leu Tyr Ser Ser Pro His Phe Thr Ala Glu Cys Arg
             130                 135                 140

        Phe Lys Glu Cys Val Phe Glu Asn Tyr Tyr Val Leu Tyr Ala Ser Ala
        145                 150                 155                 160

        Leu Tyr Arg Gln Arg Arg Ser Gly Arg Ala Trp Tyr Leu Gly Leu Asp
                         165                 170                 175

        Lys Glu Gly Arg Val Met Lys Gly Asn Arg Val Lys Lys Thr Lys Ala
                     180                 185                 190

        Ala Ala His Phe Val Pro Lys Leu Leu Glu Val Ala Met Tyr Arg Glu
                     195                 200                 205

        Pro Ser Leu His Ser Val Pro Glu Thr Ser Pro Ser Ser Pro Pro Ala
             210                 215                 220

        His
        225



        <210> 33
        <211> 243
        <212> PRT
        <213> Mus musculus

        <400> 33
        Met Ala Ala Ala Ile Ala Ser Ser Leu Ile Arg Gln Lys Arg Gln Ala
        1                   5                   10                  15

        Arg Glu Ser Asn Ser Asp Arg Val Ser Ala Ser Lys Arg Arg Ser Ser
                     20                  25                  30

        Pro Ser Lys Asp Gly Arg Ser Leu Cys Glu Arg His Val Leu Gly Val
                 35                  40                  45

        Phe Ser Lys Val Arg Phe Cys Ser Gly Arg Lys Arg Pro Val Arg Arg
             50                  55                  60

        Arg Pro Glu Pro Gln Leu Lys Gly Ile Val Thr Arg Leu Phe Ser Gln
        65                  70                  75                  80

        Gln Gly Tyr Phe Leu Gln Met His Pro Asp Gly Thr Ile Asp Gly Thr
```

```
                    85                  90                  95

Lys Asp Glu Asn Ser Asp Tyr Thr Leu Phe Asn Leu Ile Pro Val Gly
            100                 105                 110

Leu Arg Val Val Ala Ile Gln Gly Val Lys Ala Ser Leu Tyr Val Ala
            115                 120                 125

Met Asn Gly Glu Gly Tyr Leu Tyr Ser Ser Asp Val Phe Thr Pro Glu
            130                 135                 140

Cys Lys Phe Lys Glu Ser Val Phe Glu Asn Tyr Tyr Val Ile Tyr Ser
145                 150                 155                 160

Ser Thr Leu Tyr Arg Gln Gln Glu Ser Gly Arg Ala Trp Phe Leu Gly
                165                 170                 175

Leu Asn Lys Glu Gly Gln Ile Met Lys Gly Asn Arg Val Lys Lys Thr
            180                 185                 190

Lys Pro Ser Ser His Phe Val Pro Lys Pro Ile Glu Val Cys Met Tyr
            195                 200                 205

Arg Glu Pro Ser Leu His Glu Ile Gly Glu Lys Gln Gly Arg Ser Arg
            210                 215                 220

Lys Ser Ser Gly Thr Pro Thr Met Asn Gly Gly Lys Val Val Asn Gln
225                 230                 235                 240

Asp Ser Thr


<210> 34
<211> 245
<212> PRT
<213> Mus musculus

<400> 34
Met Thr Ala Ala Ile Ala Ser Ser Leu Ile Arg Gln Lys Arg Gln Ala
    1               5                   10                  15

Arg Glu Arg Glu Lys Ser Asn Ala Cys Lys Cys Val Ser Ser Pro Ser
            20                  25                  30

Lys Gly Lys Thr Ser Cys Asp Lys Asn Lys Leu Asn Val Phe Ser Arg
            35                  40                  45

Val Lys Leu Phe Gly Ser Lys Lys Arg Arg Arg Arg Pro Glu Pro
        50                  55                  60

Gln Leu Lys Gly Ile Val Thr Lys Leu Tyr Ser Arg Gln Gly Tyr His
65                  70                  75                  80

Leu Gln Leu Gln Ala Asp Gly Thr Ile Asp Gly Thr Lys Asp Glu Asp
                85                  90                  95

Ser Thr Tyr Thr Leu Phe Asn Leu Ile Pro Val Gly Leu Arg Val Val
            100                 105                 110

Ala Ile Gln Gly Val Gln Thr Lys Leu Tyr Leu Ala Met Asn Ser Glu
```

72

```
                  115                 120                 125

        Gly Tyr Leu Tyr Thr Ser Glu His Phe Thr Pro Glu Cys Lys Phe Lys
            130                 135                 140

        Glu Ser Val Phe Glu Asn Tyr Tyr Val Thr Tyr Ser Ser Met Ile Tyr
        145                 150                 155                 160

        Arg Gln Gln Gln Ser Gly Arg Gly Trp Tyr Leu Gly Leu Asn Lys Glu
                        165         .           170                 175

        Gly Glu Ile Met Lys Gly Asn His Val Lys Lys Asn Lys Pro Ala Ala
                        180                 185                 190

        His Phe Leu Pro Lys Pro Leu Lys Val Ala Met Tyr Lys Glu Pro Ser
                    195                 200                 205

        Leu His Asp Leu Thr Glu Phe Ser Arg Ser Gly Ser Gly Thr Pro Thr
            210                 215                 220

        Lys Ser Arg Ser Val Ser Gly Val Leu Asn Gly Gly Lys Ser Met Ser
        225                 230                 235                 240

        His Asn Glu Ser Thr
                        245


        <210> 35
        <211> 247
        <212> PRT
        <213> Mus musculus

        <400> 35
        Met Ala Ala Ala Ile Ala Ser Gly Leu Ile Arg Gln Lys Arg Gln Ala
            1               5               10                  15

        Arg Glu Gln His Trp Asp Arg Pro Ser Ala Ser Arg Arg Arg Ser Ser
                        20                  25                  30

        Pro Ser Lys Asn Arg Gly Leu Phe Asn Gly Asn Leu Val Asp Ile Phe
                    35                  40                  45

        Ser Lys Val Arg Ile Phe Gly Leu Lys Lys Arg Arg Leu Arg Arg Gln
            50                  55                  60

        Asp Pro Gln Leu Lys Gly Ile Val Thr Arg Leu Tyr Cys Arg Gln Gly
        65                  70                  75                  80

        Tyr Tyr Leu Gln Met His.Pro Asp Gly Ala Leu Asp Gly Thr Lys Asp
                        85                  90                  95

        Asp Ser Thr Asn Ser Thr Leu Phe Asn Leu Ile Pro Val Gly Leu Arg
                    100                 105                 110

        Val Val Ala Ile Gln Gly Val Lys Thr Gly Leu Tyr Ile Ala Met Asn
                    115                 120                 125

        Gly Glu Gly Tyr Leu Tyr Pro Ser Glu Leu Phe Thr Pro Glu Cys Lys
            130                 135                 140

        Phe Lys Glu Ser Val Phe Glu Asn Tyr Tyr Val Ile Tyr Ser Ser Met
```

```
                145                  150                    155                      160

        Leu Tyr Arg Gln Gln Glu Ser Gly Arg Ala Trp Phe Leu Gly Leu Asn
                        165                 170                 175

        Lys Glu Gly Gln Val Met Lys Gly Asn Arg Val Lys Lys Thr Lys Pro
                    180                 185                 190

        Ala Ala His Phe Leu Pro Lys Pro Leu Glu Val Ala Met Tyr Arg Glu
                    195                 200                 205

        Pro Ser Leu His Asp Val Gly Glu Thr Val Pro Lys Ala Gly Val Thr
                210                 215                 220

        Pro Ser Lys Ser Thr Ser Ala Ser Ala Ile Met Asn Gly Gly Lys Pro
        225                 230                 235                 240

        Val Asn Lys Cys Lys Thr Thr
                        245


        <210> 36
        <211> 218
        <212> PRT
        <213> Mus musculus

        <400> 36
        Met Ala Arg Lys Trp Asn Gly Arg Ala Val Ala Arg Ala Leu Val Leu
            1               5                   10                  15

        Ala Thr Leu Trp Leu Ala Val Ser Gly Arg Pro Leu Ala Gln Gln Ser
                    20                  25                  30

        Gln Ser Val Ser Asp Glu Asp Pro Leu Phe Leu Tyr Gly Trp Gly Lys
                    35                  40                  45

        Ile Thr Arg Leu Gln Tyr Leu Tyr Ser Ala Gly Pro Tyr Val Ser Asn
                50                  55                  60

        Cys Phe Leu Arg Ile Arg Ser Asp Gly Ser Val Asp Cys Glu Glu Asp
        65                  70                  75                  80

        Gln Asn Glu Arg Asn Leu Leu Glu Phe Arg Ala Val Ala Leu Lys Thr
                        85                  90                  95

        Ile Ala Ile Lys Asp Val Ser Ser Val Arg Tyr Leu Cys Met Ser Ala
                    100                 105                 110

        Asp Gly Lys Ile Tyr Gly Leu Ile Arg Tyr Ser Glu Glu Asp Cys Thr
                    115                 120                 125

        Phe Arg Glu Glu Met Asp Cys Leu Gly Tyr Asn Gln Tyr Arg Ser Met
                130                 135                 140

        Lys His His Leu His Ile Ile Phe Ile Gln Ala Lys Pro Arg Glu Gln
        145                 150                 155                 160

        Leu Gln Asp Gln Lys Pro Ser Asn Phe Ile Pro Val Phe His Arg Ser
                        165                 170                 175

        Phe Phe Glu Thr Gly Asp Gln Leu Arg Ser Lys Met Phe Ser Leu Pro
```

```
                    180                     185                     190

       Leu Glu Ser Asp Ser Met Asp Pro Phe Arg Met Val Glu Asp Val Asp
                195                     200                     205

       His Leu Val Lys Ser Pro Ser Phe Gln Lys
           210                     215


       <210> 37
       <211> 207
       <212> PRT
       <213> Rattus norvegicus

       <400> 37
       Met Ala Glu Val Gly Gly Val Phe Ala Ser Leu Asp Trp Asp Leu Gln
         1               5                  10                      15

       Gly Phe Ser Ser Ser Leu Gly Asn Val Pro Leu Ala Asp Ser Pro Gly
                  20                      25                      30

       Phe Leu Asn Glu Arg Leu Gly Gln Ile Glu Gly Lys Leu Gln Arg Gly
                  35                      40                      45

       Ser Pro Thr Asp Phe Ala His Leu Lys Gly Ile Leu Arg Arg Arg Gln
           50                      55                      60

       Leu Tyr Cys Arg Thr Gly Phe His Leu Glu Ile Phe Pro Asn Gly Thr
         65                  70                      75                  80

       Val His Gly Thr Arg His Asp His Ser Arg Phe Gly Ile Leu Glu Phe
                      85                      90                      95

       Ile Ser Leu Ala Val Gly Leu Ile Ser Ile Arg Gly Val Asp Ser Gly
                  100                     105                     110

       Leu Tyr Leu Gly Met Asn Glu Arg Gly Glu Leu Phe Gly Ser Lys Lys
                  115                     120                     125

       Leu Thr Arg Glu Cys Val Phe Arg Glu Gln Phe Glu Glu Asn Trp Tyr
                  130                     135                     140

       Asn Thr Tyr Ala Ser Thr Leu Tyr Lys His Ser Asp Ser Glu Arg Gln
       145                     150                     155                 160

       Tyr Tyr Val Ala Leu Asn Lys Asp Gly Ser Pro Arg Glu Gly Tyr Arg
                      165                     170                     175

       Thr Lys Arg His Gln Lys Phe Thr His Phe Leu Pro Arg Pro Val Asp
                      180                     185                     190

       Pro Ser Lys Leu Pro Ser Met Ser Arg Asp Leu Phe Arg Tyr Arg
                  195                     200                     205


       <210> 38
       <211> 207
       <212> PRT
       <213> Mus musculus

       <400> 38
```

```
Met Tyr Ser Ala Pro Ser Ala Cys Thr Cys Leu Cys Leu His Phe Leu
 1               5                10               15

Leu Leu Cys Phe Gln Val Gln Val Leu Ala Ala Glu Glu Asn Val Asp
            20               25               30

Phe Arg Ile His Val Glu Asn Gln Thr Arg Ala Arg Asp Asp Val Ser
        35               40               45

Arg Lys Gln Leu Arg Leu Tyr Gln Leu Tyr Ser Arg Thr Ser Gly Lys
    50               55               60

His Ile Gln Val Leu Gly Arg Arg Ile Ser Ala Arg Gly Glu Asp Gly
65               70               75               80

Asp Lys Tyr Ala Gln Leu Leu Val Glu Thr Asp Thr Phe Gly Ser Gln
            85               90               95

Val Arg Ile Lys Gly Lys Glu Thr Glu Phe Tyr Leu Cys Met Asn Arg
            100              105              110

Lys Gly Lys Leu Val Gly Lys Pro Asp Gly Thr Ser Lys Glu Cys Val
            115              120              125

Phe Ile Glu Lys Val Leu Glu Asn Asn Tyr Thr Ala Leu Met Ser Ala
    130              135              140

Lys Tyr Ser Gly Trp Tyr Val Gly Phe Thr Lys Lys Gly Arg Pro Arg
 145             150              155              160

Lys Gly Pro Lys Thr Arg Glu Asn Gln Gln Asp Val His Phe Met Lys
            165              170              175

Arg Tyr Pro Lys Gly Gln Ala Glu Leu Gln Lys Pro Phe Lys Tyr Thr
            180              185              190

Thr Val Thr Lys Arg Ser Arg Arg Ile Arg Pro Thr His Pro Gly
        195              200              205
```

```
<210> 39
<211> 11
<212> PRT
<213> Human immunodeficiency virus type 1

<400> 39
Tyr Gly Arg Lys Lys Arg Arg Gln Arg Arg Arg
 1               5                10


<210> 40
<211> 15
<212> PRT
<213> Artificial Sequence

<220>
<223> Description of Artificial Sequence: internalizing
      domain derived from HIV tat protein

<400> 40
Gly Gly Gly Gly Tyr Gly Arg Lys Lys Arg Arg Gln Arg Arg Arg
```

```
                1              5              10              15

<210> 41
<211> 22
<212> DNA
<213> Artificial Sequence

<220>
<223> Description of Artificial Sequence:
      oligonucleotide; PCR primer

<400> 41
ctatcccaat gcctccccac tg                                           22


<210> 42
<211> 21
<212> DNA
<213> Artificial Sequence

<220>
<223> Description of Artificial Sequence:
      oligonucleotide; PCR primer

<400> 42
cgcccctgac cacccctaat g                                            21


<210> 43
<211> 23
<212> DNA
<213> Artificial Sequence

<220>
<223> Description of Artificial Sequence:
      oligonucleotide; 5'RACE primer

<400> 43
gtgtggaatt gtgagcggat aac                                          23


<210> 44
<211> 22
<212> DNA
<213> Artificial Sequence

<220>
<223> Description of Artificial Sequence:
      oligonucleotide; 5'RACE primer

<400> 44
ctgatggggt gcgccatcca ca                                           22


<210> 45
<211> 23
<212> DNA
<213> Artificial Sequence

<220>
```

<223> Description of Artificial Sequence:
     oligonucleotide; nested PCR primer

<400> 45
ctatgaccat gattacgcca agc                                    23


<210> 46
<211> 24
<212> DNA
<213> Artificial Sequence

<220>
<223> Description of Artificial Sequence:
     oligonucleotide; nested PCR primer

<400> 46
cattcttgtg gatctgcagg tggt                                   24


<210> 47
<211> 23
<212> DNA
<213> Artificial Sequence

<220>
<223> Description of Artificial Sequence:
     oligonucleotide; 3'RACE primer

<400> 47
cggcctcctg ttcacaggag ctc                                    23


<210> 48
<211> 21
<212> DNA
<213> Artificial Sequence

<220>
<223> Description of Artificial Sequence:
     oligonucleotide; 3'RACE primer

<400> 48
cgggcctctt cgctattacg c                                      21


<210> 49
<211> 21
<212> DNA
<213> Artificial Sequence

<220>
<223> Description of Artificial Sequence:
     oligonucleotide; nested PCR primer

<400> 49
gcgccgagga caacagcccg a                                      21


<210> 50
<211> 21

```
<212> DNA
<213> Artificial Sequence

<220>
<223> Description of Artificial Sequence:
      oligonucleotide; nested PCR primer

<400> 50
tggcgaaagg gggatgtgct g                                          21


<210> 51
<211> 21
<212> DNA
<213> Artificial Sequence

<220>
<223> Description of Artificial Sequence:
      oligonucleotide; PCR primer

<400> 51
tccaccaccc tgttgctgta g                                          21


<210> 52
<211> 22
<212> DNA
<213> Artificial Sequence

<220>
<223> Description of Artificial Sequence:
      oligonucleotide; PCR primer

<400> 52
gaccacagtc catgccatca ct                                         22


<210> 53
<211> 22
<212> DNA
<213> Artificial Sequence

<220>
<223> Description of Artificial Sequence:
      oligonucleotide; PCR primer

<400> 53
ctatcccaat gcctccccac tg                                         22


<210> 54
<211> 21
<212> DNA
<213> Artificial Sequence

<220>
<223> Description of Artificial Sequence:
      oligonucleotide; PCR primer

<400> 54
cgcccctgac cacccctaat g                                          21
```

**Description**

<u>Field of the Invention</u>

The present invention relates to Fibroblast Growth Factor-23 (FGF-23) polypeptides and nucleic acid molecules encoding the same. The invention also relates to selective binding agents, vectors, host cells, and methods for producing FGF-23 polypeptides. The invention further relates to pharmaceutical compositions and methods for the diagnosis, treatment, amelioration, and/or prevention of diseases, disorders, and conditions associated with FGF-23 polypeptides.

<u>Background of the Invention</u>

Technical advances in the identification, cloning, expression, and manipulation of nucleic acid molecules and the deciphering of the human genome have greatly accelerated the discovery of novel therapeutics. Rapid nucleic acid sequencing techniques can now generate sequence information at unprecedented rates and, coupled with computational analyses, allow the assembly of overlapping sequences into partial and entire genomes and the identification of polypeptide-encoding regions. A comparison of a predicted amino acid sequence against a database compilation of known amino acid sequences allows one to determine the extent of homology to previously identified sequences and/or structural landmarks. The cloning and expression of a polypeptide-encoding region of a nucleic acid molecule provides a polypeptide product for structural and functional analyses. The manipulation of nucleic acid molecules and encoded polypeptides may confer advantageous properties on a product for use as a therapeutic.

In spite of the significant technical advances in genome research over the past decade, the potential for the development of novel therapeutics based on the human genome is still largely unrealized. Many genes encoding potentially beneficial polypeptide therapeutics or those encoding polypeptides, which may act as "targets" for therapeutic molecules, have still not been identified.

Accordingly, it is an object of the invention to identify novel polypeptides, and nucleic acid molecules encoding the same, which have diagnostic or therapeutic benefit.

<u>Summary of the Invention</u>

The present invention relates to novel FGF-23 nucleic acid molecules and encoded polypeptides.

The invention provides for an isolated nucleic acid molecule comprising a nucleotide sequence selected from the group consisting of:

(a) the nucleotide sequence as set forth in SEQ ID NO: 1;
(b) the nucleotide sequence of the DNA insert in ATCC Deposit No. PTA-1617;
(c) a nucleotide sequence encoding the polypeptide as set forth in SEQ ID NO: 2;
(d) a nucleotide sequence which hybridizes under moderately or highly stringent conditions to the complement of any of (a) - (c); and
(e) a nucleotide sequence complementary to any of (a) - (c).

The invention also provides for an isolated nucleic acid molecule comprising a nucleotide sequence selected from the group consisting of:

(a) a nucleotide sequence encoding a polypeptide which is at least about 70 percent identical to the polypeptide as set forth in SEQ ID NO: 2, wherein the encoded polypeptide has an activity of the polypeptide set forth in SEQ ID NO: 2;
(b) a nucleotide sequence encoding an allelic variant or splice variant of the nucleotide sequence as set forth in SEQ ID NO: 1, the nucleotide sequence of the DNA insert in ATCC Deposit No. PTA-1617, or (a);
(c) a region of the nucleotide sequence of SEQ ID NO: 1, the DNA insert in ATCC Deposit No. PTA-1617, (a), or (b) encoding a polypeptide fragment of at least about 25 amino acid residues, wherein the polypeptide fragment has an activity of the encoded polypeptide as set forth in SEQ ID NO: 2, or is antigenic;
(d) a region of the nucleotide sequence of SEQ ID NO: 1, the DNA insert in ATCC Deposit No. PTA-1617, or any of (a) - (c) comprising a fragment of at least about 16 nucleotides;
(e) a nucleotide sequence which hybridizes under moderately or highly stringent conditions to the complement of any of (a) - (d); and
(f) a nucleotide sequence complementary to any of (a) - (d).

The invention further provides for an isolated nucleic acid molecule comprising a nucleotide sequence selected from the group consisting of:

(a) a nucleotide sequence encoding a polypeptide as set forth in SEQ ID NO: 2 with at least one conservative amino acid substitution, wherein the encoded polypeptide has an activity of the polypeptide set forth in SEQ ID NO: 2;

(b) a nucleotide sequence encoding a polypeptide as set forth in SEQ ID NO: 2 with at least one amino acid insertion, wherein the encoded polypeptide has an activity of the polypeptide set forth in SEQ ID NO: 2;

(c) a nucleotide sequence encoding a polypeptide as set forth in SEQ ID NO: 2 with at least one amino acid deletion, wherein the encoded polypeptide has an activity of the polypeptide set forth in SEQ ID NO: 2;

(d) a nucleotide sequence encoding a polypeptide as set forth in SEQ ID NO: 2 which has a C- and/or N- terminal truncation, wherein the encoded polypeptide has an activity of the polypeptide set forth in SEQ ID NO: 2;

(e) a nucleotide sequence encoding a polypeptide as set forth in SEQ ID NO: 2 with at least one modification selected from the group consisting of amino acid substitutions, amino acid insertions, amino acid deletions, C-terminal truncation, and N-terminal truncation, wherein the encoded polypeptide has an activity of the polypeptide set forth in SEQ ID NO: 2;

(f) a nucleotide sequence of any of (a) - (e) comprising a fragment of at least about 16 nucleotides;

(g) a nucleotide sequence which hybridizes under moderately or highly stringent conditions to the complement of any of (a) - (f); and

(h) a nucleotide sequence complementary to any of (a) - (e).

The present invention provides for an isolated polypeptide comprising an amino acid sequence selected from the group consisting of:

(a) the amino acid sequence as set forth in SEQ ID NO: 2; and

(b) the amino acid sequence encoded by the DNA insert in ATCC Deposit No. PTA-1617.

The invention also provides for an isolated polypeptide comprising the amino acid sequence selected from the group consisting of:

(a) the amino acid sequence as set forth in SEQ ID NO: 3, optionally further comprising an amino-terminal methionine;

(b) an amino acid sequence for an ortholog of SEQ ID NO: 2;

(c) an amino acid sequence which is at least about 70 percent identical to the amino acid sequence of SEQ ID NO: 2, wherein the polypeptide has an activity of the polypeptide set forth in SEQ ID NO: 2;

(d) a fragment of the amino acid sequence set forth in SEQ ID NO: 2 comprising at least about 25 amino acid residues, wherein the fragment has an activity of the polypeptide set forth in SEQ ID NO: 2, or is antigenic; and

(e) an amino acid sequence for an allelic variant or splice variant of the amino acid sequence as set forth in SEQ ID NO: 2, the amino acid sequence encoded by the DNA insert in ATCC Deposit No. PTA-1617, or (a) - (c).

The invention further provides for an isolated polypeptide comprising the amino acid sequence selected from the group consisting of:

(a) the amino acid sequence as set forth in SEQ ID NO: 2 with at least one conservative amino acid substitution, wherein the polypeptide has an activity of the polypeptide set forth in SEQ ID NO: 2;

(b) the amino acid sequence as set forth in SEQ ID NO: 2 with at least one amino acid insertion, wherein the polypeptide has an activity of the polypeptide set forth in SEQ ID NO: 2;

(c) the amino acid sequence as set forth in SEQ ID NO: 2 with at least one amino acid deletion, wherein the polypeptide has an activity of the polypeptide set forth in SEQ ID NO: 2;

(d) the amino acid sequence as set forth in SEQ ID NO: 2 which has a C- and/or N- terminal truncation, wherein the polypeptide has an activity of the polypeptide set forth in SEQ ID NO: 2; and

(e) the amino acid sequence as set forth in SEQ ID NO: 2 with at least one modification selected from the group consisting of amino acid substitutions, amino acid insertions, amino acid deletions, C-terminal truncation, and N-terminal truncation, wherein the polypeptide has an activity of the polypeptide set forth in SEQ ID NO: 2.

Also provided are fusion polypeptides comprising FGF-23 amino acid sequences.

The present invention also provides for an expression vector comprising the isolated nucleic acid molecules as set forth herein, recombinant host cells comprising the recombinant nucleic acid molecules as set forth herein, and a method of producing an FGF-23 polypeptide comprising culturing the host cells and optionally isolating the polypeptide so produced.

A transgenic non-human animal comprising a nucleic acid molecule encoding an FGF-23 polypeptide is also encompassed by the invention. The FGF-23 nucleic acid molecules are introduced into the animal in a manner that allows expression and increased levels of an FGF-23 polypeptide, which may include increased circulating levels.

Alternatively, the FGF-23 nucleic acid molecules are introduced into the animal in a manner that prevents expression of endogenous FGF-23 polypeptide (*i.e.,* generates a transgenic animal possessing an FGF-23 polypeptide gene knockout). The transgenic non-human animal is preferably a mammal, and more preferably a rodent, such as a rat or a mouse.

Also provided are derivatives of the FGF-23 polypeptides of the present invention.

Additionally provided are selective binding agents such as antibodies and peptides capable of specifically binding the FGF-23 polypeptides of the invention. Such antibodies and peptides may be agonistic or antagonistic.

Pharmaceutical compositions comprising the nucleotides, polypeptides, or selective binding agents of the invention and one or more pharmaceutically acceptable formulation agents are also encompassed by the invention. The pharmaceutical compositions are used to provide therapeutically effective amounts of the nucleotides or polypeptides of the present invention. The invention is also directed to methods of using the polypeptides, nucleic acid molecules, and selective binding agents.

The FGF-23 polypeptides and nucleic acid molecules of the present invention may be used to treat, prevent, ameliorate, and/or detect diseases and disorders, including those recited herein.

The present invention also provides a method of assaying test molecules to identify a test molecule that binds to an FGF-23 polypeptide. The method comprises contacting an FGF-23 polypeptide with a test molecule to determine the extent of binding of the test molecule to the polypeptide. The method further comprises determining whether such test molecules are agonists or antagonists of an FGF-23 polypeptide. The present invention further provides a method of testing the impact of molecules on the expression of FGF-23 polypeptide or on the activity of FGF-23 polypeptide.

Methods of regulating expression and modulating (*i.e.*, increasing or decreasing) levels of an FGF-23 polypeptide are also encompassed by the invention. One method comprises administering to an animal a nucleic acid molecule encoding an FGF-23 polypeptide. In another method, a nucleic acid molecule comprising elements that regulate or modulate the expression of an FGF-23 polypeptide may be administered. Examples of these methods include gene therapy, cell therapy, and anti-sense therapy as further described herein.

In another aspect of the present invention, the FGF-23 polypeptides may be used for identifying receptors thereof ("FGF-23 polypeptide receptors"). Various forms of "expression cloning" have been extensively used to clone receptors for protein ligands. *See, e.g.,* Simonsen and Lodish, 1994, Trends Pharmacol. Sci. 15:437-41 and Tartaglia et al., 1995, Cell 83:1263-71. The isolation of an FGF-23 polypeptide receptor is useful for identifying or developing novel agonists and antagonists of the FGF-23 polypeptide signaling pathway. Such agonists and antagonists include soluble FGF-23 polypeptide receptors, anti-FGF-23 polypeptide receptor-selective binding agents (such as antibodies and derivatives thereof), small molecules, and antisense oligonucleotides, any of which can be used for treating one or more disease or disorder, including those disclosed herein.

Brief Description of the Figures

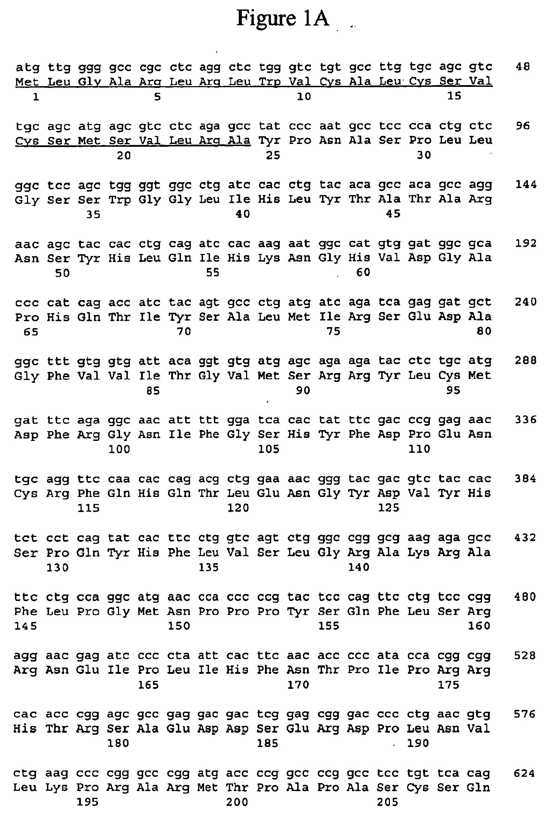

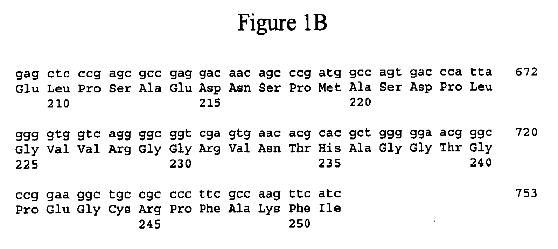

Figures 1A-1B illustrate the nucleotide sequence of the human FGF-23 gene (SEQ ID NO: 1) and the deduced amino acid sequence of human FGFR polypeptide (SEQ ID NO: 2). The predicted signal peptide is indicated (underlined);

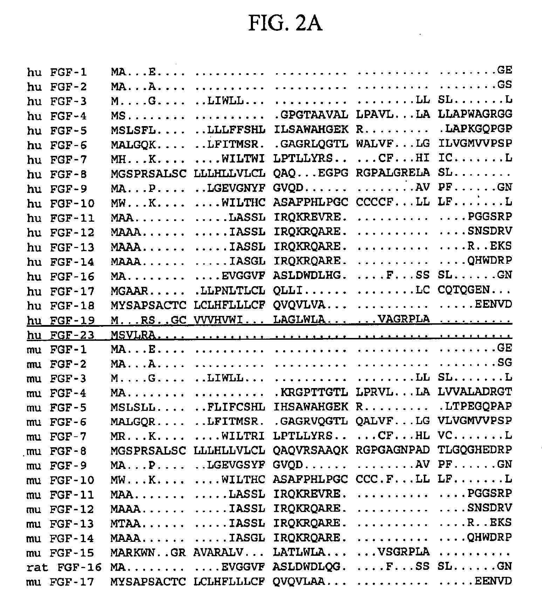

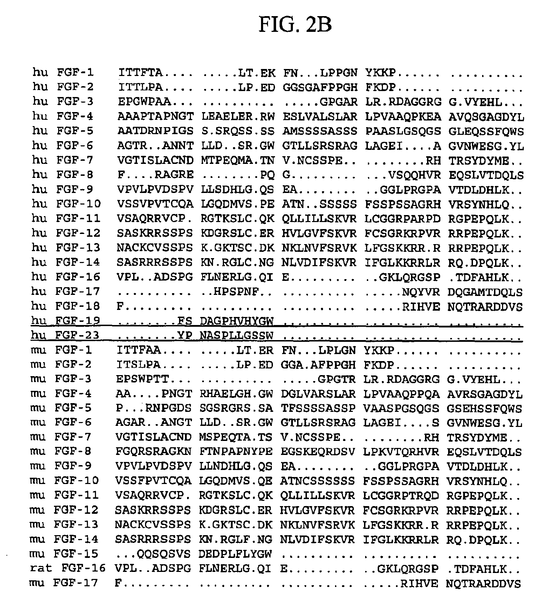

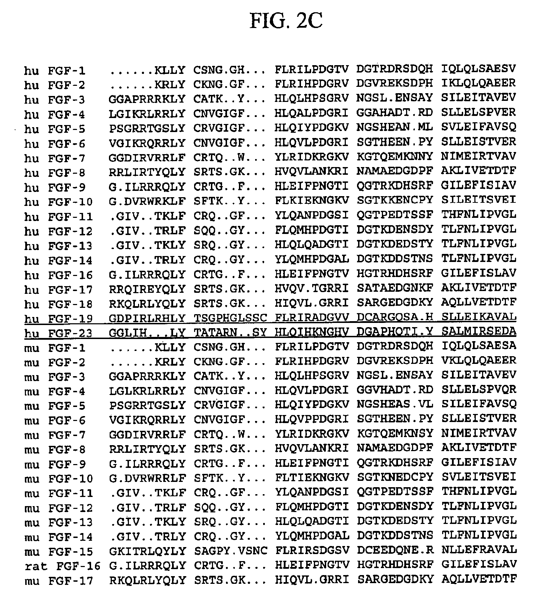

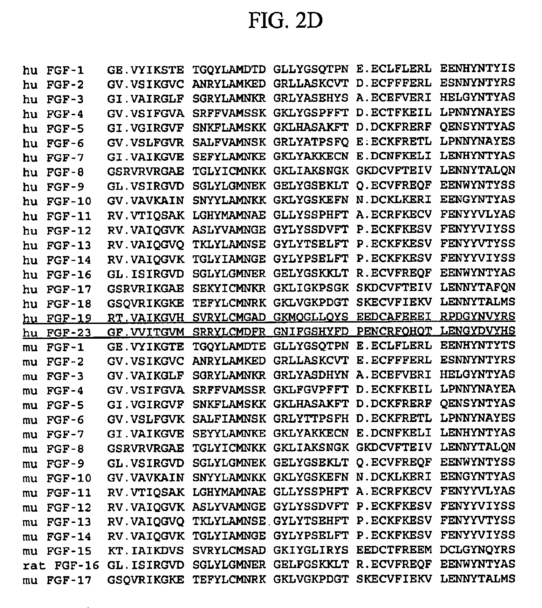

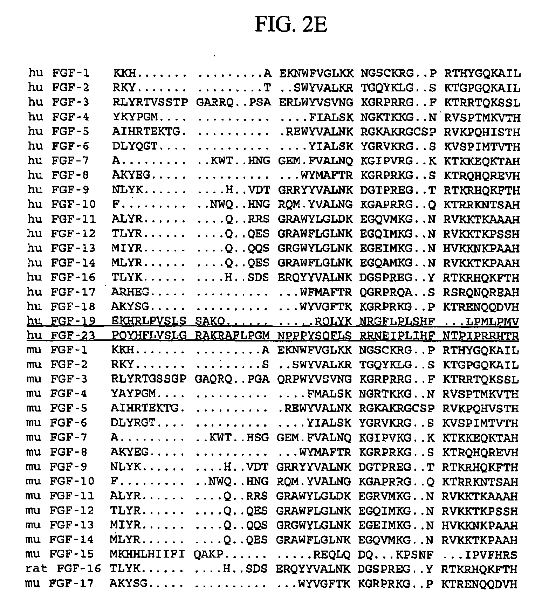

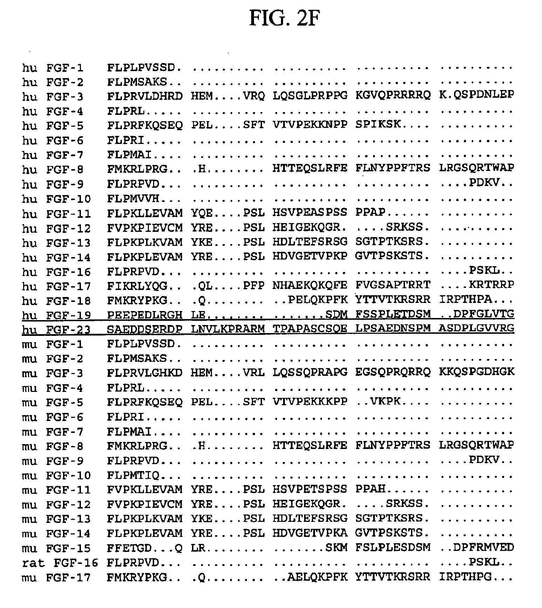

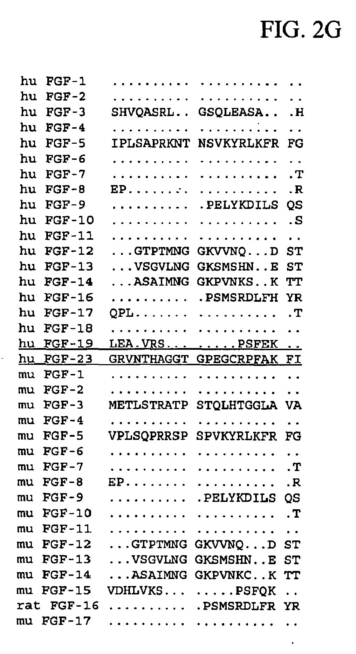

Figures 2A-2G illustrate the amino acid sequence alignment of human FGF-1 (hu FGF-1; SEQ ID NO: 4), human FGF-2 (hu FGF-2; SEQ ID NO: 5), human FGF-3 (hu FGF-3; SEQ ID NO: 6), human FGF-4 (hu FGF-4; SEQ ID NO: 7), human FGF-5 (hu FGF-5; SEQ ID NO: 8), human FGF-6 (hu FGF-6; SEQ ID NO: 9), human FGF-7 (hu FGF-7; SEQ ID NO: 10), human FGF-8 (hu FGF-8; SEQ ID NO: 11), human FGF-9 (hu FGF-9; SEQ ID NO: 12), human FGF-10 (hu FGF-10; SEQ ID NO: 13), human FGF-11 (hu FGF-11; SEQ ID NO: 14), human FGF-12 (hu FGF-12; SEQ ID NO: 15), human FGF-13 (hu FGF-13; SEQ ID NO: 16), human FGF-14 (hu FGF-14; SEQ ID NO: 17), human FGF-16 (hu FGF-16; SEQ ID NO: 18), human FGF-17 (hu FGF-17; SEQ ID NO: 19), human FGF-18 (hu FGF-18; SEQ ID NO: 20), human FGF-19 (hu FGF-19; SEQ ID NO: 21), human FGF-23 (hu FGF-23; SEQ ID NO: 22), murine FGF-1 (mu FGF-1; SEQ ID NO: 23), murine FGF-2 (mu FGF-2; SEQ ID NO: 24), murine FGF-3 (mu FGF-3; SEQ ID NO: 25), murine FGF-4 (mu FGF-4; SEQ ID NO: 26), murine FGF-5 (mu FGF-5; SEQ ID NO: 27), murine FGF-6 (mu FGF-6; SEQ ID NO: 28), murine FGF-7 (mu FGF-7; SEQ ID NO: 29), murine FGF-8 (mu FGF-8; SEQ ID NO: 30), murine FGF-9 (mu FGF-9; SEQ ID NO: 31), murine FGF-10 (mu FGF-10; SEQ ID NO: 32), murine FGF-11 (mu FGF-11; SEQ ID NO: 33), murine FGF-12 (mu FGF-12; SEQ ID NO: 34), murine FGF-13 (mu FGF-13; SEQ ID NO: 35), murine FGF-14 (mu FGF-14; SEQ ID NO: 36), murine FGF-15 (mu FGF-15; SEQ ID NO: 37), rat FGF-16 (rat FGF-16; SEQ ID NO: 38), murine FGF-17 (mu FGF-17; SEQ ID NO: 39);

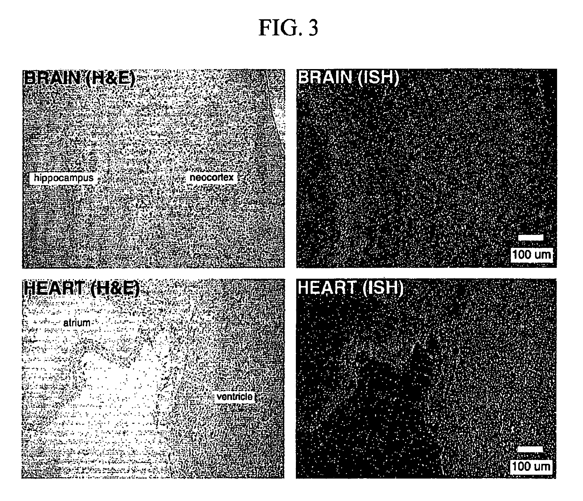

Figure 3 illustrates the expression of FGF-23 mRNA as detected by *in situ* hybridization in the brain and cardiac muscle (heart) of a normal adult mouse (H&E = hematoxylin and eosin counterstaining; ISH = *in situ* hybridization);

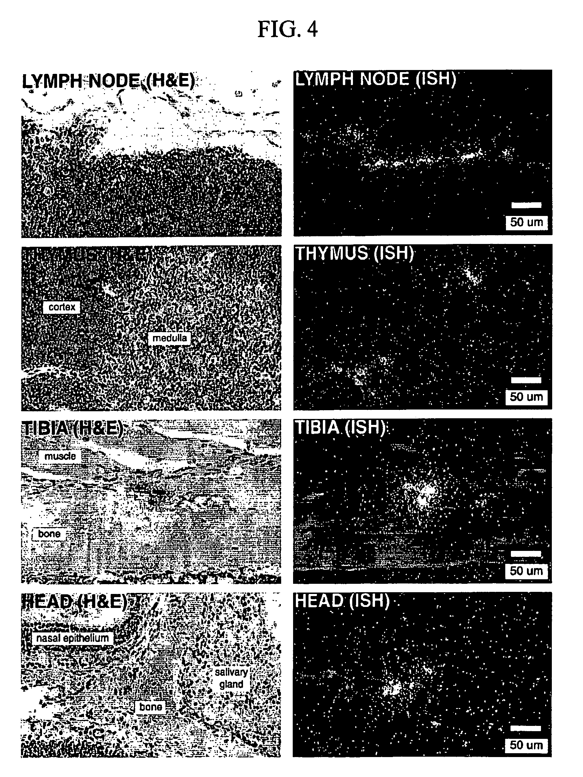

Figure 4 illustrates the expression of FGF-23 mRNA as detected by *in situ* hybridization in the subcapsular region of the lymph node (lymph node), thymic medulla (thymus), lacunae of cortical bone from the tibia (tibia), and trabecular bone in the head (head) of a non-expressing transgenic mouse (H&E = hematoxylin and eosin counterstaining; ISH = *in situ* hybridization);

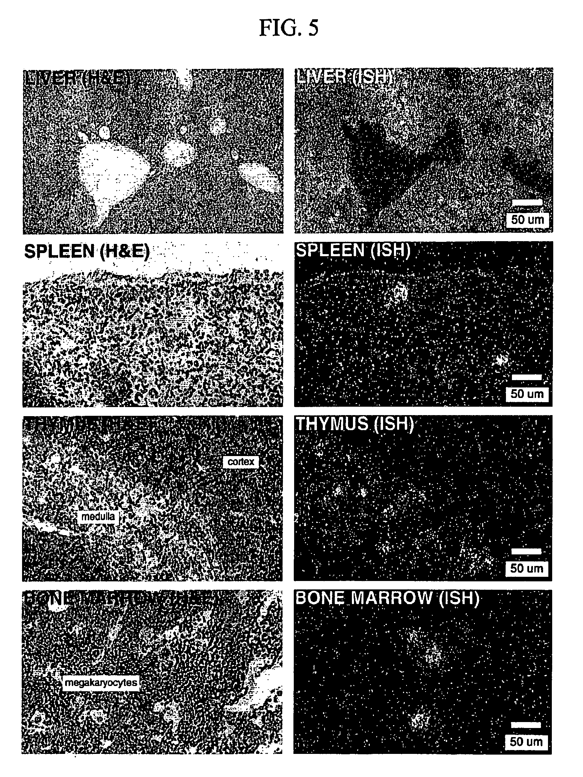

Figure 5 illustrates the expression of FGF-23 mRNA as detected by *in situ* hybridization in the liver, spleen, thymic medulla (thymus), and megakaryocytes in the bone marrow (bone marrow) of a high expressing transgenic mouse (H&E = hematoxylin and eosin counterstaining; ISH = *in situ* hybridization);

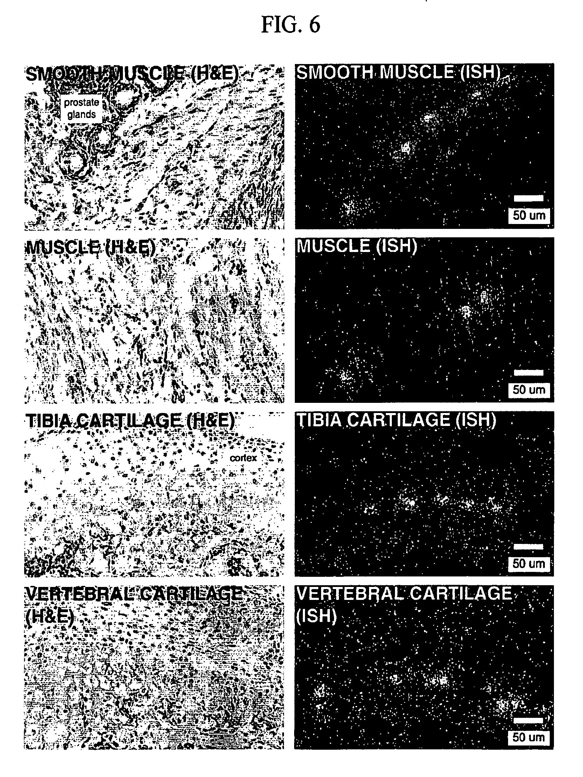

Figure 6 illustrates the expression of FGF-23 mRNA as detected by *in situ* hybridization in the smooth muscle tissue near the prostrate (smooth muscle), muscle tissue of the jaw (muscle), chondrocytes in the tibia (tibia), and chondrocytes in the vertebrae (vertebrae) of a high expressing transgenic mouse (H&E = hematoxylin and eosin counterstaining; ISH = *in situ* hybridization).

Detailed Description of the Invention

The section headings used herein are for organizational purposes only and are not to be construed as limiting the subject matter described. All references cited in this application are expressly incorporated by reference herein.

Definitions

The terms "FGF-23 gene" or "FGF-23 nucleic acid molecule" or "FGF-23 polynucleotide" refer to a nucleic acid molecule comprising or consisting of a nucleotide sequence as set forth in SEQ ID NO: 1, a nucleotide sequence encoding the polypeptide as set forth in SEQ ID NO: 2, a nucleotide sequence of the DNA insert in ATCC Deposit No. PTA-1617, and nucleic acid molecules as defined herein.

The term "FGF-23 polypeptide allelic variant" refers to one of several possible naturally occurring alternate forms of a gene occupying a given locus on a chromosome of an organism or a population of organisms.

The term "FGF-23 polypeptide splice variant" refers to a nucleic acid molecule, usually RNA, which is generated by alternative processing of intron sequences in an RNA transcript of FGF-23 polypeptide amino acid sequence as set forth in SEQ ID NO: 2.

The term "isolated nucleic acid molecule" refers to a nucleic acid molecule of the invention that (1) has been separated from at least about 50 percent of proteins, lipids, carbohydrates, or other materials with which it is naturally found when total nucleic acid is isolated from the source cells, (2) is not linked to all or a portion of a polynucleotide to which the "isolated nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule (s) or other contaminants that are found in its natural environment that would interfere with its use in polypeptide production or its therapeutic, diagnostic, prophylactic or research use.

The term "nucleic acid sequence" or "nucleic acid molecule" refers to a DNA or RNA sequence. The term encompasses molecules formed from any of the known base analogs of DNA and RNA such as, but not limited to 4-acetylcytosine, 8-hydroxy-N6-methyladenosine, aziridinyl-cytosine, pseudoisocytosine, 5-(carboxyhydroxylmethyl) uracil, 5-fluorouracil, 5-bromouracil, 5-carboxymethylaminomethyl-2-thiouracil, 5-carboxy-methylaminomethyluracil, dihydrouracil, inosine, N6-iso-pentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5'-methoxycarbonyl-methyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "vector" is used to refer to any molecule (e.g., nucleic acid, plasmid, or virus) used to transfer coding information to a host cell.

The term "expression vector" refers to a vector that is suitable for transformation of a host cell and contains nucleic acid sequences that direct and/or control the expression of inserted heterologous nucleic acid sequences. Expression includes, but is not limited to, processes such as transcription, translation, and RNA splicing, if introns are present.

The term "operably linked" is used herein to refer to an arrangement of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

The term "host cell" is used to refer to a cell which has been transformed, or is capable of being transformed with a nucleic acid sequence and then of expressing a selected gene of interest. The term includes the progeny of the parent cell, whether or not the progeny is identical in morphology or in genetic make-up to the original parent, so long as the selected gene is present.

The term "FGF-23 polypeptide" refers to a polypeptide comprising the amino acid sequence of SEQ ID NO: 2 and related polypeptides. Related polypeptides include FGF-23 polypeptide fragments, FGF-23 polypeptide orthologs, FGF-23 polypeptide variants, and FGF-23 polypeptide derivatives, which possess at least one activity of the polypeptide as set forth in SEQ ID NO: 2. FGF-23 polypeptides may be mature polypeptides, as defined herein, and may or may not have an amino-terminal methionine residue, depending on the method by which they are prepared.

The term "FGF-23 polypeptide fragment" refers to a polypeptide that comprises a truncation at the amino-terminus (with or without a leader sequence) and/or a truncation at the carboxyl-terminus of the polypeptide as set forth in SEQ ID NO: 2. The term "FGF-23 polypeptide fragment" also refers to amino-terminal and/or carboxyl-terminal truncations of FGF-23 polypeptide orthologs, FGF-23 polypeptide derivatives, or FGF-23 polypeptide variants, or to amino-terminal and/or carboxyl-terminal truncations of the polypeptides encoded by FGF-23 polypeptide allelic variants or FGF-23 polypeptide splice variants. FGF-23 polypeptide fragments may result from alternative RNA splicing or from *in vivo* protease activity. Membrane-bound forms of an FGF-23 polypeptide are also contemplated by the present invention. In preferred embodiments, truncations and/or deletions comprise about 10 amino acids, or about 20 amino acids, or about 50 amino acids, or about 75 amino acids, or about 100 amino acids, or more than about 100 amino acids. The polypeptide fragments so produced will comprise about 25 contiguous amino acids, or about 50 amino acids, or about 75 amino acids, or about 100 amino acids, or about 150 amino acids, or about 200 amino acids, or more than about 200 amino acids. Such FGF-23 polypeptide fragments may optionally comprise an amino-terminal methionine residue. It will be appreciated that such fragments can be used, for example, to generate antibodies to FGF-23 polypeptides.

The term "FGF-23 polypeptide ortholog" refers to a polypeptide from another species that corresponds to FGF-23 polypeptide amino acid sequence as set forth in SEQ ID NO: 2. For example, mouse and human FGF-23 polypeptides are considered orthologs of each other.

The term "FGF-23 polypeptide variants" refers to FGF-23 polypeptides comprising amino acid sequences having one or more amino acid sequence substitutions, deletions (such as internal deletions and/or FGF-23 polypeptide fragments), and/or additions (such as internal additions and/or FGF-23 fusion polypeptides) as compared to the FGF-23 polypeptide amino acid sequence set forth in SEQ ID NO: 2 (with or without a leader sequence). Variants may be naturally occurring (e.g., FGF-23 polypeptide allelic variants, FGF-23 polypeptide orthologs, and FGF-23 polypeptide splice variants) or artificially constructed. Such FGF-23 polypeptide variants may be prepared from the corresponding nucleic acid molecules having a DNA sequence that varies accordingly from the DNA sequence as set forth in SEQ ID NO: 1. In preferred embodiments, the variants have from 1 to 3, or from 1 to 5, or from 1 to 10, or from 1 to 15, or from 1 to 20, or from 1 to 25, or from 1 to 50, or from 1 to 75, or from 1 to 100, or more than 100 amino acid substitutions, insertions, additions and/or deletions, wherein the substitutions may be conservative, or non-conservative, or any combination thereof.

The term "FGF-23 polypeptide derivatives" refers to the polypeptide as set forth in SEQ ID NO: 2, FGF-23 polypeptide fragments, FGF-23 polypeptide orthologs, or FGF-23 polypeptide variants, as defined herein, that have been chemically modified. The term "FGF-23 polypeptide derivatives" also refers to the polypeptides encoded by FGF-23 polypeptide allelic variants or FGF-23 polypeptide splice variants, as defined herein, that have been chemically modified.

The term "mature FGF-23 polypeptide" refers to an FGF-23 polypeptide lacking a leader sequence. A mature FGF-23 polypeptide may also include other modifications such as proteolytic processing of the amino-terminus (with or without a leader sequence) and/or the carboxyl-terminus, cleavage of a smaller polypeptide from a larger precursor, N-linked and/or O-linked glycosylation, and the like. An exemplary mature CHL polypeptide is depicted by the amino acid sequence of SEQ ID NO: 3.

The term "FGF-23 fusion polypeptide" refers to a fusion of one or more amino acids (such as a heterologous protein or peptide) at the amino- or carboxyl-terminus of the polypeptide as set forth in SEQ ID NO: 2, FGF-23 polypeptide fragments, FGF-23 polypeptide orthologs, FGF-23 polypeptide variants, or FGF-23 derivatives, as defined herein. The term "FGF-23 fusion polypeptide" also refers to a fusion of one or more amino acids at the amino- or carboxyl-terminus of the polypeptide encoded by FGF-23 polypeptide allelic variants or FGF-23 polypeptide splice variants, as defined herein.

The term "biologically active FGF-23 polypeptides" refers to FGF-23 polypeptides having at least one activity characteristic of the polypeptide comprising the amino acid sequence of SEQ ID NO: 2. In addition, an FGF-23 polypeptide may be active as an immunogen; that is, the FGF-23 polypeptide contains at least one epitope to which antibodies may be raised.

The term "isolated polypeptide" refers to a polypeptide of the present invention that (1) has been separated from at least about 50 percent of polynucleotides, lipids, carbohydrates, or other materials with which it is naturally found when isolated from the source cell, (2) is not linked (by covalent or noncovalent interaction) to all or a portion of a polypeptide to which the "isolated polypeptide" is linked in nature, (3) is operably linked (by covalent or noncovalent interaction) to a polypeptide with which it is not linked in nature, or (4) does not occur in nature. Preferably, the isolated polypeptide is substantially free from any other contaminating polypeptides or other contaminants that are found in its natural environment that would interfere with its therapeutic, diagnostic, prophylactic or research use.

The term "identity," as known in the art, refers to a relationship between the sequences of two or more polypeptide molecules or two or more nucleic acid molecules, as determined by comparing the sequences. In the art, "identity" also means the degree of sequence relatedness between nucleic acid molecules or polypeptides, as the case may be, as determined by the match between strings of two or more nucleotide or two or more amino acid sequences. "Identity" measures the percent of identical matches between the smaller of two or more sequences with gap alignments (if any) addressed by a particular mathematical model or computer program (*i.e.,* "algorithms").

The term "similarity" is a related concept, but in contrast to "identity," "similarity" refers to a measure of relatedness which includes both identical matches and conservative substitution matches. If two polypeptide sequences have, for example, 10/20 identical amino acids, and the remainder are all non-conservative substitutions, then the percent identity and similarity would both be 50%. If in the same example, there are five more positions where there are conservative substitutions, then the percent identity remains 50%, but the percent similarity would be 75% (15/20). Therefore, in cases where there are conservative substitutions, the percent similarity between two polypeptides will be higher than the percent identity between those two polypeptides.

The term "naturally occurring" or "native" when used in connection with biological materials such as nucleic acid molecules, polypeptides, host cells, and the like, refers to materials which are found in nature and are not manipulated by man. Similarly, "non-naturally occurring" or "non-native" as used herein refers to a material that is not found in nature or that has been structurally modified or synthesized by man.

The terms "effective amount" and "therapeutically effective amount" each refer to the amount of an FGF-23 polypeptide or FGF-23 nucleic acid molecule used to support an observable level of one or more biological activities of the FGF-23 polypeptides as set forth herein.

The term "pharmaceutically acceptable carrier" or "physiologically acceptable carrier" as used herein refers to one or more formulation materials suitable for accomplishing or enhancing the delivery of the FGF-23 polypeptide, FGF-23 nucleic acid molecule, or FGF-23 selective binding agent as a pharmaceutical composition.

The term "antigen" refers to a molecule or a portion of a molecule capable of being bound by a selective binding agent, such as an antibody, and additionally capable of being used in an animal to produce antibodies capable of binding to an epitope of that antigen. An antigen may have one or more epitopes.

The term "selective binding agent" refers to a molecule or molecules having specificity for an FGF-23 polypeptide. As used herein, the terms, "specific" and "specificity" refer to the ability of the selective binding agents to bind to human FGF-23 polypeptides and not to bind to human non-FGF-23 polypeptides. It will be appreciated, however, that the selective binding agents may also bind orthologs of the polypeptide as set forth in SEQ ID NO: 2, that is, interspecies versions thereof, such as mouse and rat FGF-23 polypeptides.

The term "transduction" is used to refer to the transfer of genes from one bacterium to another, usually by a phage. "Transduction" also refers to the acquisition and transfer of eukaryotic cellular sequences by retroviruses.

The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. *See, e.g.,* Graham et al., 1973, Virology 52:456; Sambrook et al., Molecular Cloning, A Laboratory Manual (Cold Spring Harbor Laboratories, 1989); Davis et al., Basic Methods in Molecular Biology (Elsevier, 1986); and Chu et al., 1981, Gene 13:197. Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, may be maintained transiently as an episomal element without being replicated, or may replicate independently as a plasmid. A cell is considered to have been stably transformed when the DNA is replicated with the division of the cell.

Relatedness of Nucleic Acid Molecules and/or Polypeptides

It is understood that related nucleic acid molecules include allelic or splice variants of the nucleic acid molecule of SEQ ID NO: 1, and include sequences which are complementary to any of the above nucleotide sequences. Related nucleic acid molecules also include a nucleotide sequence encoding a polypeptide comprising or consisting essentially of a substitution, modification, addition and/or deletion of one or more amino acid residues compared to the polypeptide in SEQ ID NO: 2. Such related FGF-23 polypeptides may comprise, for example, an addition and/or a deletion of one or more N-linked or O-linked glycosylation sites or an addition and/or a deletion of one or more cysteine residues.

Related nucleic acid molecules also include fragments of FGF-23 nucleic acid molecules which encode a polypeptide of at least about 25 contiguous amino acids, or about 50 amino acids, or about 75 amino acids, or about 100 amino acids, or more than 100 amino acid residues of the FGF-23 polypeptide of SEQ ID NO: 2.

In addition, related FGF-23 nucleic acid molecules also include those molecules which comprise nucleotide sequences which hybridize under moderately or highly stringent conditions as defined herein with the fully complementary sequence of the FGF-23 nucleic acid molecule of SEQ ID NO: 1, or of a molecule encoding a polypeptide, which polypeptide comprises the amino acid sequence as shown in SEQ ID NO: 2, or of a nucleic acid fragment as defined herein, or of a nucleic acid fragment encoding a polypeptide as defined herein. Hybridization probes may be prepared using the FGF-23 sequences provided herein to screen cDNA, genomic or synthetic DNA libraries for related sequences. Regions of the DNA and/or amino acid sequence of FGF-23 polypeptide that exhibit significant identity to known sequences are readily determined using sequence alignment algorithms as described herein and those regions may be used to design probes for screening.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength, and the concentration of denaturing agents such as formamide. Examples of "highly stringent conditions" for hybridization and washing are 0.015 M sodium chloride, 0.0015 M sodium citrate at 65-68°C or 0.015 M sodium chloride, 0.0015 M sodium citrate, and 50% formamide at 42°C. *See* Sambrook, Fritsch & Maniatis, Molecular Cloning: A Laboratory Manual (2nd ed., Cold Spring Harbor Laboratory, 1989); Anderson et al., Nucleic Acid Hybridisation: A Practical Approach Ch. 4 (IRL Press Limited).

More stringent conditions (such as higher temperature, lower ionic strength, higher formamide, or other denaturing agent) may also be used - however, the rate of hybridization will be affected. Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate, $NaDodSO_4$, (SDS), ficoll, Denhardt's solution, sonicated salmon sperm DNA (or another non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4; however, at typical ionic strength conditions, the rate of hybridization is nearly independent of pH. *See* Anderson et al., Nucleic Acid Hybridisation: A Practical Approach Ch. 4 (IRL Press Limited).

Factors affecting the stability of DNA duplex include base composition, length, and degree of base pair mismatch. Hybridization conditions can be adjusted by one skilled in the art in order to accommodate these variables and allow DNAs of different sequence relatedness to form hybrids. The melting temperature of a perfectly matched DNA duplex can be estimated by the following equation:

$$T_m(^oC) = 81.5 + 16.6(\log[Na+]) + 0.41(\%G+C) - 600/N - 0.72(\%formamide)$$

where N is the length of the duplex formed, [Na+] is the molar concentration of the sodium ion in the hybridization or washing solution, %G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015 M sodium chloride, 0.0015 M sodium citrate at 50-65°C or 0.015 M sodium chloride, 0.0015 M sodium citrate, and 20% formamide at 37-50°C. By way of example, "moderately stringent conditions" of 50°C in 0.015 M sodium ion will allow about a 21% mismatch.

It will be appreciated by those skilled in the art that there is no absolute distinction between "highly stringent conditions" and "moderately stringent conditions." For example, at 0.015 M sodium ion (no formamide), the melting temperature of perfectly matched long DNA is about 71°C. With a wash at 65°C (at the same ionic strength), this would allow for approximately a 6% mismatch. To capture more distantly related sequences, one skilled in the art can simply lower the temperature or raise the ionic strength.

A good estimate of the melting temperature in 1M NaCl* for oligonucleotide probes up to about 20nt is given by:

$$Tm = 2^{o}C \text{ per A-T base pair} + 4^{o}C \text{ per G-C base pair}$$

* The sodium ion concentration in 6X salt sodium citrate (SSC) is 1M. *See* Suggs et al., Developmental Biology Using Purified Genes 683 (Brown and Fox, eds., 1981).

High stringency washing conditions for oligonucleotides are usually at a temperature of 0-5°C below the Tm of the oligonucleotide in 6X SSC, 0.1 % SDS.

In another embodiment, related nucleic acid molecules comprise or consist of a nucleotide sequence that is at least about 70 percent identical to the nucleotide sequence as shown in SEQ ID NO: 1, or comprise or consist essentially of a nucleotide sequence encoding a polypeptide that is at least about 70 percent identical to the polypeptide as set forth in SEQ ID NO: 2. In preferred embodiments, the nucleotide sequences are about 75 percent, or about 80 percent, or about 85 percent, or about 90 percent, or about 95, 96, 97, 98, or 99 percent identical to the nucleotide sequence as shown in SEQ ID NO: 1, or the nucleotide sequences encode a polypeptide that is about 75 percent, or about 80 percent, or about 85 percent, or about 90 percent, or about 95, 96, 97, 98, or 99 percent identical to the polypeptide sequence as set forth in SEQ ID NO: 2. Related nucleic acid molecules encode polypeptides possessing at least one activity of the polypeptide set forth in SEQ ID NO: 2.

Differences in the nucleic acid sequence may result in conservative and/or non-conservative modifications of the amino acid sequence relative to the amino acid sequence of SEQ ID NO: 2.

Conservative modifications to the amino acid sequence of SEQ ID NO: 2 (and the corresponding modifications to the encoding nucleotides) will produce a polypeptide having functional and chemical characteristics similar to those of FGF-23 polypeptides. In contrast, substantial modifications in the functional and/or chemical characteristics of FGF-23 polypeptides may be accomplished by selecting substitutions in the amino acid sequence of SEQ ID NO: 2 that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis."

Conservative amino acid substitutions also encompass non-naturally occurring amino acid residues that are typically incorporated by chemical peptide synthesis rather than by synthesis in biological systems. These include peptidomimetics, and other reversed or inverted forms of amino acid moieties.

Naturally occurring residues may be divided into classes based on common side chain properties:

1) hydrophobic: norleucine, Met, Ala, Val, Leu, Ile;
2) neutral hydrophilic: Cys, Ser, Thr;
3) acidic: Asp, Glu;
4) basic: Asn, Gln, His, Lys, Arg;
5) residues that influence chain orientation: Gly, Pro; and
6) aromatic: Trp, Tyr, Phe.

For example, non-conservative substitutions may involve the exchange of a member of one of these classes for a member from another class. Such substituted residues may be introduced into regions of the human FGF-23 polypeptide that are homologous with non-human FGF-23 polypeptides, or into the non-homologous regions of the molecule.

In making such changes, the hydropathic index of amino acids may be considered. Each amino acid has been assigned a hydropathic index on the basis of its hydrophobicity and charge characteristics. The hydropathic indices are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte et al., 1982, J. Mol. Biol. 157:105-31). It is known that certain amino acids may be substituted for other amino acids having a similar hydropathic index or score and still retain a similar biological activity. In making changes based upon the hydropathic index, the substitution of amino acids whose hydropathic indices are within $\pm2$ is preferred, those which are within $\pm1$ are particularly preferred, and those within $\pm0.5$ are even more particularly preferred.

It is also understood in the art that the substitution of like amino acids can be made effectively on the basis of hydrophilicity, particularly where the biologically functionally equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, *i.e.,* with a biological property of the protein.

The following hydrophilicity values have been assigned to these amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). In making changes based upon similar hydrophilicity values, the substitution of amino acids whose hydrophilicity values are within ±2 is preferred, those which are within ±1 are particularly preferred, and those within ±0.5 are even more particularly preferred. One may also identify epitopes from primary amino acid sequences on the basis of hydrophilicity. These regions are also referred to as "epitopic core regions."

Desired amino acid substitutions (whether conservative or non-conservative) can be determined by those skilled in the art at the time such substitutions are desired. For example, amino acid substitutions can be used to identify important residues of the FGF-23 polypeptide, or to increase or decrease the affinity of the FGF-23 polypeptides described herein. Exemplary amino acid substitutions are set forth in Table I.

<u>Table I</u>
Amino Acid Substitutions

| Original Residues | Exemplary Substitutions | Preferred Substitutions |
|---|---|---|
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of the polypeptide as set forth in SEQ ID NO: 2 using well-known techniques. For identifying suitable areas of the molecule that may be changed without destroying biological activity, one skilled in the art may target areas not believed to be important for activity. For example, when similar polypeptides with similar activities from the same species or from other species are known, one skilled in the art may compare the amino acid sequence of an FGF-23 polypeptide to such similar polypeptides. With such a comparison, one can identify residues and portions of the molecules that are conserved among similar polypeptides. It will be appreciated that changes in areas of the FGF-23 molecule that are not conserved relative to such similar polypeptides would be less likely to adversely affect the biological activity and/or structure of an FGF-23 polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in an FGF-23 polypeptide that correspond to amino acid residues that are important for activity or structure in similar polypeptides. One skilled in the art may opt for chemically similar amino acid substitutions for such predicted important amino acid residues of FGF-23 polypeptides.

One skilled in the art can also analyze the three-dimensional structure and amino acid sequence in relation to that structure in similar polypeptides. In view of such information, one skilled in the art may predict the alignment of amino acid residues of FGF-23 polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each amino acid residue. The variants could be screened using activity assays known to those with skill in the art. Such variants could be used to gather information about suitable variants. For example, if one discovered that a change to a particular amino acid residue resulted in destroyed, undesirably reduced, or unsuitable activity, variants with such a change would be avoided. In other words, based on information gathered from such routine experiments, one skilled in the art can readily determine the amino acids where further substitutions should be avoided either alone or in combination with other mutations.

A number of scientific publications have been devoted to the prediction of secondary structure. *See* Moult, 1996, Curr. Opin. Biotechnol. 7:422-27; Chou et al., 1974, Biochemistry 13:222-45; Chou et al., 1974, Biochemistry 113: 211-22; Chou et al., 1978, Adv. Enzymol. Relat. Areas Mol. Biol. 47:45-48; Chou et al., 1978, Ann. Rev. Biochem. 47: 251-276; and Chou et al., 1979, Biophys. J. 26:367-84. Moreover, computer programs are currently available to assist with predicting secondary structure. One method of predicting secondary structure is based upon homology modeling. For example, two polypeptides or proteins which have a sequence identity of greater than 30%, or similarity greater than 40%, often have similar structural topologies. The recent growth of the protein structural database (PDB) has provided enhanced predictability of secondary structure, including the potential number of folds within the structure of a polypeptide or protein. *See* Holm et al., 1999, Nucleic Acids Res. 27:244-47. It has been suggested that there are a limited number of folds in a given polypeptide or protein and that once a critical number of structures have been resolved, structural prediction will become dramatically more accurate (Brenner et al., 1997, Curr. Opin. Struct. Biol. 7:369-76).

Additional methods of predicting secondary structure include "threading" (Jones, 1997, Curr. Opin. Struct. Biol. 7:377-87; Sippl et al., 1996, Structure 4:15-19), "profile analysis" (Bowie et al., 1991, Science, 253:164-70; Gribskov et al., 1990, Methods Enzymol. 183:146-59; Gribskov et al., 1987, Proc. Nat. Acad. Sci. U.S.A. 84:4355-58), and "evolutionary linkage" (*See* Holm *et al., supra,* and *Brenner et al., supra*).

Preferred FGF-23 polypeptide variants include glycosylation variants wherein the number and/or type of glycosylation sites have been altered compared to the amino acid sequence set forth in SEQ ID NO: 2. In one embodiment, FGF-23 polypeptide variants comprise a greater or a lesser number of N-linked glycosylation sites than the amino acid sequence set forth in SEQ ID NO: 2. An N-linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions that eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred FGF-23 variants include cysteine variants, wherein one or more cysteine residues are deleted or substituted with another amino acid (*e.g.,* serine) as compared to the amino acid sequence set forth in SEQ ID NO: 2. Cysteine variants are useful when FGF-23 polypeptides must be refolded into a biologically active conformation such as after the isolation of insoluble inclusion bodies. Cysteine variants generally have fewer cysteine residues than the native protein, and typically have an even number to minimize interactions resulting from unpaired cysteines.

In other embodiments, related nucleic acid molecules comprise or consist of a nucleotide sequence encoding a polypeptide as set forth in SEQ ID NO: 2 with at least one amino acid insertion and wherein the polypeptide has an activity of the polypeptide set forth in SEQ ID NO: 2, or a nucleotide sequence encoding a polypeptide as set forth in SEQ ID NO: 2 with at least one amino acid deletion and wherein the polypeptide has an activity of the polypeptide set forth in SEQ ID NO: 2. Related nucleic acid molecules also comprise or consist of a nucleotide sequence encoding a polypeptide as set forth in SEQ ID NO: 2 wherein the polypeptide has a carboxyl- and/or amino-terminal truncation and further wherein the polypeptide has an activity of the polypeptide set forth in SEQ ID NO: 2. Related nucleic acid molecules also comprise or consist of a nucleotide sequence encoding a polypeptide as set forth in SEQ ID NO: 2 with at least one modification selected from the group consisting of amino acid substitutions, amino acid insertions, amino acid deletions, carboxyl-terminal truncations, and amino-terminal truncations and wherein the polypeptide has an activity of the polypeptide set forth in SEQ ID NO: 2.

In addition, the polypeptide comprising the amino acid sequence of SEQ ID NO: 2, or other FGF-23 polypeptide, may be fused to a homologous polypeptide to form a homodimer or to a heterologous polypeptide to form a heterodimer. Heterologous peptides and polypeptides include, but are not limited to: an epitope to allow for the detection and/or isolation of an FGF-23 fusion polypeptide; a transmembrane receptor protein or a portion thereof, such as an extracellular domain or a transmembrane and intracellular domain; a ligand or a portion thereof which binds to a transmembrane receptor protein; an enzyme or portion thereof which is catalytically active; a polypeptide or peptide which promotes oligomerization, such as a leucine zipper domain; a polypeptide or peptide which increases stability, such as an immunoglobulin constant region; and a polypeptide which has a therapeutic activity different from the polypeptide comprising the amino acid sequence as set forth in SEQ ID NO: 2, or other FGF-23 polypeptide.

Fusions can be made either at the amino-terminus or at the carboxyl-terminus of the polypeptide comprising the amino acid sequence set forth in SEQ ID NO: 2, or other FGF-23 polypeptide. Fusions may be direct with no linker or adapter molecule or may be through a linker or adapter molecule. A linker or adapter molecule may be one or more amino acid residues, typically from about 20 to about 50 amino acid residues. A linker or adapter molecule may also be designed with a cleavage site for a DNA restriction endonuclease or for a protease to allow for the separation of the fused moieties. It will be appreciated that once constructed, the fusion polypeptides can be derivatized according to the methods described herein.

In a further embodiment of the invention, the polypeptide comprising the amino acid sequence of SEQ ID NO: 2, or other FGF-23 polypeptide, is fused to one or more domains of an Fc region of human IgG. Antibodies comprise two functionally independent parts, a variable domain known as "Fab," that binds an antigen, and a constant domain known as "Fc," that is involved in effector functions such as complement activation and attack by phagocytic cells. An Fc has a long serum half-life, whereas an Fab is short-lived. Capon et al., 1989, Nature 337:525-31. When constructed together with a therapeutic protein, an Fc domain can provide longer half-life or incorporate such functions as Fc receptor binding, protein A binding, complement fixation, and perhaps even placental transfer. *Id.* Table II summarizes the use of certain Fc fusions known in the art.

Table II
Fc Fusion with Therapeutic Proteins

| Form of Fc | Fusion partner | Therapeutic implications | Reference |
|---|---|---|---|
| IgG1 | N-terminus of CD30-L | Hodgkin's disease; anaplastic lymphoma; T-cell leukemia | U.S. Patent No. 5,480,981 |
| Murine Fcγ2a | IL-10 | anti-inflammatory; transplant rejection | Zheng et al., 1995, J. Immunol. 154:5590-600 |
| IgG1 | TNF receptor | septic shock | Fisher et al., 1996, N. Engl. J. Med. 334:1697-1702; Van Zee et al., 1996, J. Immunol. 156:2221-30 |
| IgG, IgA, IgM, or IgE (excluding the first domain) | TNF receptor | inflammation, autoimmune disorders | U.S. Patent No. 5,808,029 |
| IgG1 | CD4 receptor | AIDS | Capon et al., 1989, Nature 337: 525-31 |
| IgG1, IgG3 | N-terminus of IL-2 | anti-cancer, antiviral | Harvill et al., 1995,Immunotech. 1:95-105 |
| IgG1 | C-terminus of OPG | osteoarthritis; bone density | WO 97/23614 |
| IgG1 | N-terminus of leptin | anti-obesity | PCT/US 97/23183, filed December 11, 1997 |
| Human Ig Cγ1 | CTLA-4 | autoimmune disorders | Linsley, 1991, J. Exp. Med., 174:561-69 |

In one example, a human IgG hinge, CH2, and CH3 region may be fused at either the amino-terminus or carboxyl-terminus of the FGF-23 polypeptides using methods known to the skilled artisan. In another example, a human IgG hinge, CH2, and CH3 region may be fused at either the amino-terminus or carboxyl-terminus of an FGF-23 polypeptide fragment (e.g., the predicted extracellular portion of FGF-23 polypeptide).

The resulting FGF-23 fusion polypeptide may be purified by use of a Protein A affinity column. Peptides and proteins fused to an Fc region have been found to exhibit a substantially greater half-life *in vivo* than the unfused counterpart. Also, a fusion to an Fc region allows for dimerization/multimerization of the fusion polypeptide. The Fc region may be a naturally occurring Fc region, or may be altered to improve certain qualities, such as therapeutic qualities, circulation time, or reduced aggregation.

Identity and similarity of related nucleic acid molecules and polypeptides are readily calculated by known methods. Such methods include, but are not limited to those described in Computational Molecular Biology (A.M. Lesk, ed., Oxford University Press 1988); Biocomputing: Informatics and Genome Projects (D.W. Smith, ed., Academic Press 1993); Computer Analysis of Sequence Data (Part 1, A.M. Griffin and H.G. Griffin, eds., Humana Press 1994); G. von Heinle, Sequence Analysis in Molecular Biology (Academic Press 1987); Sequence Analysis Primer (M. Gribskov and J. Devereux, eds., M. Stockton Press 1991); and Carillo et al., 1988, SIAM J. Applied Math., 48:1073.

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., 1984, Nucleic Acids Res. 12:387; Genetics Computer Group, University of Wisconsin, Madison, WI), BLASTP, BLASTN, and FASTA (Altschul et al., 1990, J. Mol. Biol. 215:403-10). The BLASTX program is publicly available from the National Center for Biotechnology Information (NCBI) and other sources (Altschul et al., BLAST Manual (NCB NLM NIH, Bethesda, MD); Altschul *et al.,* 1990, *supra*). The well-known Smith Waterman algorithm may also be used to determine identity.

Certain alignment schemes for aligning two amino acid sequences may result in the matching of only a short region of the two sequences, and this small aligned region may have very high sequence identity even though there is no significant relationship between the two full-length sequences. Accordingly, in a preferred embodiment, the selected alignment method (GAP program) will result in an alignment that spans at least 50 contiguous amino acids of the claimed polypeptide.

For example, using the computer algorithm GAP (Genetics Computer Group, University of Wisconsin, Madison, WI), two polypeptides for which the percent sequence identity is to be determined are aligned for optimal matching of their respective amino acids (the "matched span," as determined by the algorithm). A gap opening penalty (which is calculated as 3X the average diagonal; the "average diagonal" is the average of the diagonal of the comparison matrix being used; the "diagonal" is the score or number assigned to each perfect amino acid match by the particular comparison matrix) and a gap extension penalty (which is usually 0.1X the gap opening penalty), as well as a comparison matrix such as PAM 250 or BLOSUM 62 are used in conjunction with the algorithm. A standard comparison matrix is also used by the algorithm (*see* Dayhoff *et al., 5 Atlas of Protein Sequence and Structure* (Supp. 3 1978)(PAM250 comparison matrix); Henikoff et al., 1992, Proc. Natl. Acad. Sci USA 89:10915-19 (BLOSUM 62 comparison matrix)).

Preferred parameters for polypeptide sequence comparison include the following:

Algorithm: Needleman and Wunsch, 1970, J. Mol. Biol. 48:443-53;
Comparison matrix: BLOSUM 62 (Henikoff *et al., supra*);
Gap Penalty: 12
Gap Length Penalty: 4
Threshold of Similarity: 0

The GAP program is useful with the above parameters. The aforementioned parameters are the default parameters for polypeptide comparisons (along with no penalty for end gaps) using the GAP algorithm.

Preferred parameters for nucleic acid molecule sequence comparison include the following:

Algorithm: Needleman and Wunsch, *supra;*
Comparison matrix: matches = +10, mismatch = 0
Gap Penalty: 50
Gap Length Penalty: 3

The GAP program is also useful with the above parameters. The aforementioned parameters are the default parameters for nucleic acid molecule comparisons.

Other exemplary algorithms, gap opening penalties, gap extension penalties, comparison matrices, and thresholds of similarity may be used, including those set forth in the Program Manual, Wisconsin Package, Version 9, September, 1997. The particular choices to be made will be apparent to those of skill in the art and will depend on the specific comparison to be made, such as DNA-to-DNA, protein-to-protein, protein-to-DNA; and additionally, whether the comparison is between given pairs of sequences (in which case GAP or BestFit are generally preferred) or between one sequence and a large database of sequences (in which case FASTA or BLASTA are preferred).

Nucleic Acid Molecules

The nucleic acid molecules encoding a polypeptide comprising the amino acid sequence of an FGF-23 polypeptide can readily be obtained in a variety of ways including, without limitation, chemical synthesis, cDNA or genomic library screening, expression library screening, and/or PCR amplification of cDNA.

Recombinant DNA methods used herein are generally those set forth in Sambrook et al., Molecular Cloning: A Laboratory Manual (Cold Spring Harbor Laboratory Press, 1989) and/or Current Protocols in Molecular Biology (Ausubel et al., eds., Green Publishers Inc. and Wiley and Sons 1994). The invention provides for nucleic acid molecules as described herein and methods for obtaining such molecules.

Where a gene encoding the amino acid sequence of an FGF-23 polypeptide has been identified from one species, all or a portion of that gene may be used as a probe to identify orthologs or related genes from the same species. The probes or primers may be used to screen cDNA libraries from various tissue sources believed to express the FGF-23 polypeptide. In addition, part or all of a nucleic acid molecule having the sequence as set forth in SEQ ID NO: 1 may be used to screen a genomic library to identify and isolate a gene encoding the amino acid sequence of an FGF-23 polypeptide. Typically, conditions of moderate or high stringency will be employed for screening to minimize the number of false positives obtained from the screening.

Nucleic acid molecules encoding the amino acid sequence of FGF-23 polypeptides may also be identified by expression cloning which employs the detection of positive clones based upon a property of the expressed protein. Typically, nucleic acid libraries are screened by the binding an antibody or other binding partner (*e.g.*, receptor or ligand) to cloned proteins that are expressed and displayed on a host cell surface. The antibody or binding partner is modified with a detectable label to identify those cells expressing the desired clone.

Recombinant expression techniques conducted in accordance with the descriptions set forth below may be followed to produce these polynucleotides and to express the encoded polypeptides. For example, by inserting a nucleic acid sequence that encodes the amino acid sequence of an FGF-23 polypeptide into an appropriate vector, one skilled in the art can readily produce large quantities of the desired nucleotide sequence. The sequences can then be used to generate detection probes or amplification primers. Alternatively, a polynucleotide encoding the amino acid sequence of an FGF-23 polypeptide can be inserted into an expression vector. By introducing the expression vector into an appropriate host, the encoded FGF-23 polypeptide may be produced in large amounts.

Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA encoding the amino acid sequence of an FGF-23 polypeptide, are then added to the cDNA along with a polymerase such as *Taq* polymerase, and the polymerase amplifies the cDNA region between the two primers.

Another means of preparing a nucleic acid molecule encoding the amino acid sequence of an FGF-23 polypeptide is chemical synthesis using methods well known to the skilled artisan such as those described by Engels et al., 1989, Angew. Chem. Intl. Ed. 28:716-34. These methods include, *inter alia,* the phosphotriester, phosphoramidite, and H-phosphonate methods for nucleic acid synthesis. A preferred method for such chemical synthesis is polymer-supported synthesis using standard phosphoramidite chemistry. Typically, the DNA encoding the amino acid sequence of an FGF-23 polypeptide will be several hundred nucleotides in length. Nucleic acids larger than about 100 nucleotides can be synthesized as several fragments using these methods. The fragments can then be ligated together to form the full-length nucleotide sequence of an FGF-23 gene. Usually, the DNA fragment encoding the amino-terminus of the polypeptide will have an ATG, which encodes a methionine residue. This methionine may or may not be present on the mature form of the FGF-23 polypeptide, depending on whether the polypeptide produced in the host cell is designed to be secreted from that cell. Other methods known to the skilled artisan may be used as well.

In certain embodiments, nucleic acid variants contain codons which have been altered for optimal expression of an FGF-23 polypeptide in a given host cell. Particular codon alterations will depend upon the FGF-23 polypeptide and host cell selected for expression. Such "codon optimization" can be carried out by a variety of methods, for example, by selecting codons which are preferred for use in highly expressed genes in a given host cell. Computer algorithms which incorporate codon frequency tables such as "Eco_high.Cod" for codon preference of highly expressed bacterial genes may be used and are provided by the University of Wisconsin Package Version 9.0 (Genetics Computer Group, Madison, WI). Other useful codon frequency tables include "Celegans_high.cod," "Celegans_low.cod," "Drosophila_ high.cod," "Human_high.cod," "Maize_high.cod," and "Yeast_high.cod."

In some cases, it may be desirable to prepare nucleic acid molecules encoding FGF-23 polypeptide variants.

Nucleic acid molecules encoding variants may be produced using site directed mutagenesis, PCR amplification, or other appropriate methods, where the primer(s) have the desired point mutations (*see* Sambrook *et al., supra,* and Ausubel *et al., supra,* for descriptions of mutagenesis techniques). Chemical synthesis using methods described by Engels *et al., supra,* may also be used to prepare such variants. Other methods known to the skilled artisan may be used as well.

Vectors and Host Cells

A nucleic acid molecule encoding the amino acid sequence of an FGF-23 polypeptide is inserted into an appropriate expression vector using standard ligation techniques. The vector is typically selected to be functional in the particular host cell employed (*i.e.,* the vector is compatible with the host cell machinery such that amplification of the gene and/or expression of the gene can occur). A nucleic acid molecule encoding the amino acid sequence of an FGF-23 polypeptide may be amplified/expressed in prokaryotic, yeast, insect (baculovirus systems) and/or eukaryotic host cells. Selection of the host cell will depend in part on whether an FGF-23 polypeptide is to be post-translationally modified (*e.g.,* glycosylated and/or phosphorylated). If so, yeast, insect, or mammalian host cells are preferable. For a review of expression vectors, *see* Meth. Enz., vol. 185 (D.V. Goeddel, ed., Academic Press 1990).

Typically, expression vectors used in any of the host cells will contain sequences for plasmid maintenance and for cloning and expression of exogenous nucleotide sequences. Such sequences, collectively referred to as "flanking sequences" in certain embodiments will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, i.e., an oligonucleotide molecule located at the 5' or 3' end of the FGF-23 polypeptide coding sequence; the oligonucleotide sequence encodes polyHis (such as hexaHis), or another "tag" such as FLAG, HA (hemaglutinin influenza virus), or *myc* for which commercially available antibodies exist. This tag is typically fused to the polypeptide upon expression of the polypeptide, and can serve as a means for affinity purification of the FGF-23 polypeptide from the host cell. Affinity purification can be accomplished, for example, by column chromatography using antibodies against the tag as an affinity matrix. Optionally, the tag can subsequently be removed from the purified FGF-23 polypeptide by various means such as using certain peptidases for cleavage.

Flanking sequences may be homologous (*i.e.*, from the same species and/or strain as the host cell), heterologous (*i.e.*, from a species other than the host cell species or strain), hybrid (*i.e.,* a combination of flanking sequences from more than one source), or synthetic, or the flanking sequences may be native sequences which normally function to regulate FGF-23 polypeptide expression. As such, the source of a flanking sequence may be any prokaryotic or eukaryotic organism, any vertebrate or invertebrate organism, or any plant, provided that the flanking sequence is functional in, and can be activated by, the host cell machinery.

Flanking sequences useful in the vectors of this invention may be obtained by any of several methods well known in the art. Typically, flanking sequences useful herein - other than the FGF-23 gene flanking sequences - will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Where all or only a portion of the flanking sequence is known, it may be obtained using PCR and/or by screening a genomic library with a suitable oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen® column chromatography (Chatsworth, CA), or other methods known to the skilled artisan. The selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. Amplification of the vector to a certain copy number can, in some cases, be important for the optimal expression of an FGF-23 polypeptide. If the vector of choice does not contain an origin of replication site, one may be chemically synthesized based on a known sequence, and ligated into the vector. For example, the origin of replication from the plasmid pBR322 (New England Biolabs, Beverly, MA) is suitable for most gram-negative bacteria and various origins (*e.g.*, SV40, polyoma, adenovirus, vesicular stomatitus virus (VSV), or papillomaviruses such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding region and serves to terminate transcription. Usually, a transcription termination sequence in prokaryotic cells is a G-C rich fragment followed by a poly-T sequence. While the sequence is easily cloned from a library or even purchased commercially as part of a vector, it can also be readily synthesized using methods for nucleic acid synthesis such as those described herein.

A selectable marker gene element encodes a protein necessary for the survival and growth of a host cell grown in a selective culture medium. Typical selection marker genes encode proteins that (a) confer resistance to antibiotics or other toxins, *e.g.*, ampicillin, tetracycline, or kanamycin for prokaryotic host cells; (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene that will be expressed. Amplification is the process wherein genes that are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure wherein only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the DNA that encodes an FGF-23 polypeptide. As a result, increased quantities of FGF-23 polypeptide are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of an FGF-23 polypeptide to be expressed. The Shine-Dalgarno sequence is varied but is typically a polypurine (*i.e.,* having a high A-G content). Many Shine-Dalgarno sequences have been identified, each of which can be readily synthesized using methods set forth herein and used in a prokaryotic vector.

A leader, or signal, sequence may be used to direct an FGF-23 polypeptide out of the host cell. Typically, a nucleotide sequence encoding the signal sequence is positioned in the coding region of an FGF-23 nucleic acid molecule, or directly at the 5' end of an FGF-23 polypeptide coding region. Many signal sequences have been identified, and any of those that are functional in the selected host cell may be used in conjunction with an FGF-23 nucleic acid molecule. Therefore, a signal sequence may be homologous (naturally occurring) or heterologous to the FGF-23 nucleic acid molecule. Additionally, a signal sequence may be chemically synthesized using methods described herein. In most cases, the secretion of an FGF-23 polypeptide from the host cell via the presence of a signal peptide will result in the removal of the signal peptide from the secreted FGF-23 polypeptide. The signal sequence may be a component of the vector, or it may be a part of an FGF-23 nucleic acid molecule that is inserted into the vector.

Included within the scope of this invention is the use of either a nucleotide sequence encoding a native FGF-23 polypeptide signal sequence joined to an FGF-23 polypeptide coding region or a nucleotide sequence encoding a heterologous signal sequence joined to an FGF-23 polypeptide coding region. The heterologous signal sequence selected should be one that is recognized and processed, i.e., cleaved by a signal peptidase, by the host cell. For prokaryotic host cells that do not recognize and process the native FGF-23 polypeptide signal sequence, the signal sequence is substituted by a prokaryotic signal sequence selected, for example, from the group of the alkaline phosphatase, penicillinase, or heat-stable enterotoxin II leaders. For yeast secretion, the native FGF-23 polypeptide signal sequence may be substituted by the yeast invertase, alpha factor, or acid phosphatase leaders. In mammalian cell expression the native signal sequence is satisfactory, although other mammalian signal sequences may be suitable.

In some cases, such as where glycosylation is desired in a eukaryotic host cell expression system, one may manipulate the various presequences to improve glycosylation or yield. For example, one may alter the peptidase cleavage site of a particular signal peptide, or add pro-sequences, which also may affect glycosylation. The final protein product may have, in the -1 position (relative to the first amino acid of the mature protein) one or more additional amino acids incident to expression, which may not have been totally removed. For example, the final protein product may have one or two amino acid residues found in the peptidase cleavage site, attached to the amino-terminus. Alternatively, use of some enzyme cleavage sites may result in a slightly truncated form of the desired FGF-23 polypeptide, if the enzyme cuts at such area within the mature polypeptide.

In many cases, transcription of a nucleic acid molecule is increased by the presence of one or more introns in the vector; this is particularly true where a polypeptide is produced in eukaryotic host cells, especially mammalian host cells. The introns used may be naturally occurring within the FGF-23 gene especially where the gene used is a full-length genomic sequence or a fragment thereof. Where the intron is not naturally occurring within the gene (as for most cDNAs), the intron may be obtained from another source. The position of the intron with respect to flanking sequences and the FGF-23 gene is generally important, as the intron must be transcribed to be effective. Thus, when an FGF-23 cDNA molecule is being transcribed, the preferred position for the intron is 3' to the transcription start site and 5' to the poly-A transcription termination sequence. Preferably, the intron or introns will be located on one side or the other (*i.e.*, 5' or 3') of the cDNA such that it does not interrupt the coding sequence. Any intron from any source, including viral, prokaryotic and eukaryotic (plant or animal) organisms, may be used to practice this invention, provided that it is compatible with the host cell into which it is inserted. Also included herein are synthetic introns. Optionally, more than one intron may be used in the vector.

The expression and cloning vectors of the present invention will typically contain a promoter that is recognized by the host organism and operably linked to the molecule encoding the FGF-23 polypeptide. Promoters are untranscribed sequences located upstream (*i.e.*, 5') to the start codon of a structural gene (generally within about 100 to 1000 bp) that control the transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A suitable promoter is operably linked to the DNA encoding FGF-23 polypeptide by removing the promoter from the source DNA by restriction enzyme digestion and inserting the desired promoter sequence into the vector. The native FGF-23 promoter sequence may be used to direct amplification and/or expression of an FGF-23 nucleic acid molecule. A heterologous promoter is preferred, however, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; alkaline phosphatase; a tryptophan (trp) promoter system; and hybrid promoters such as the tac promoter. Other known bacterial promoters are also suitable. Their sequences have been published, thereby enabling one skilled in the art to ligate them to the desired DNA sequence, using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus, retroviruses, hepatitis-B virus and most preferably Simian Virus 40 (SV40). Other suitable mammalian promoters include heterologous mammalian promoters, for example, heat-shock promoters and the actin promoter.

Additional promoters which may be of interest in controlling FGF-23 gene expression include, but are not limited to: the SV40 early promoter region (Bemoist and Chambon, 1981, Nature 290:304-10); the CMV promoter; the promoter contained in the 3' long terminal repeat of Rous sarcoma virus (Yamamoto, et al., 1980, Cell 22:787-97); the herpes thymidine kinase promoter (Wagner et al., 1981, Proc. Natl. Acad. Sci. U.S.A. 78:1444-45); the regulatory sequences of the metallothionine gene (Brinster et al., 1982, Nature 296:39-42); prokaryotic expression vectors such as the beta-lactamase promoter (Villa-Kamaroff et al., 1978, Proc. Natl. Acad. Sci. U.S.A., 75:3727-31); or the tac promoter (DeBoer et al., 1983, Proc. Natl. Acad. Sci. U.S.A., 80:21-25). Also of interest are the following animal transcriptional control regions, which exhibit tissue specificity and have been utilized in transgenic animals: the elastase I gene control region which is active in pancreatic acinar cells (Swift et al., 1984, Cell 38:639-46; Ornitz et al., 1986, Cold Spring Harbor Symp. Quant. Biol. 50:399-409 (1986); MacDonald, 1987, Hepatology 7:425-515); the insulin gene control region which is active in pancreatic beta cells (Hanahan, 1985, Nature 315:115-22); the immunoglobulin gene control region which is active in lymphoid cells (Grosschedl et al., 1984, Cell 38:647-58; Adames et al., 1985, Nature 318:533-38; Alexander et al., 1987, Mol. Cell. Biol., 7:1436-44); the mouse mammary tumor virus control region which is active in testicular, breast, lymphoid and mast cells (Leder et al., 1986, Cell 45:485-95); the albumin gene control region which is active in liver (Pinkert et al., 1987, Genes and Devel. 1:268-76); the alpha-feto-protein gene control region which is active in liver (Krumlauf et al., 1985, Mol. Cell. Biol., 5:1639-48; Hammer et al., 1987, Science 235:53-58); the alpha 1-antitrypsin gene control region which is active in the liver (Kelsey et al., 1987, Genes and Devel. 1:161-71); the beta-globin gene control region which is active in myeloid cells (Mogram et al., 1985, Nature 315:338-40; Kollias et al., 1986, Cell 46: 89-94); the myelin basic protein gene control region which is active in oligodendrocyte cells in the brain (Readhead et al., 1987, Cell 48:703-12); the myosin light chain-2 gene control region which is active in skeletal muscle (Sani, 1985, Nature 314:283-86); and the gonadotropic releasing hormone gene control region which is active in the hypothalamus (Mason et al., 1986, Science 234:1372-78).

An enhancer sequence may be inserted into the vector to increase the transcription of a DNA encoding an FGF-23 polypeptide of the present invention by higher eukaryotes. Enhancers are cis-acting elements of DNA, usually about 10-300 bp in length, that act on the promoter to increase transcription. Enhancers are relatively orientation and position independent. They have been found 5' and 3' to the transcription unit. Several enhancer sequences available from mammalian genes are known (*e.g.*, globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters.

While an enhancer may be spliced into the vector at a position 5' or 3' to an FGF-23 nucleic acid molecule, it is typically located at a site 5' from the promoter.

Expression vectors of the invention may be constructed from a starting vector such as a commercially available vector. Such vectors may or may not contain all of the desired flanking sequences. Where one or more of the flanking sequences described herein are not already present in the vector, they may be individually obtained and ligated into the vector. Methods used for obtaining each of the flanking sequences are well known to one skilled in the art.

Preferred vectors for practicing this invention are those which are compatible with bacterial, insect, and mammalian host cells. Such vectors include, *inter alia,* pCRII, pCR3, and pcDNA3.1 (Invitrogen, San Diego, CA), pBSII (Stratagene, La Jolla, CA), pET15 (Novagen, Madison, WI), pGEX (Pharmacia Biotech, Piscataway, NJ), pEGFP-N2 (Clontech, Palo Alto, CA), pETL (BlueBacII, Invitrogen), pDSR-alpha (PCT Pub. No. WO 90/14363) and pFastBacDual (Gibco-BRL, Grand Island, NY).

Additional suitable vectors include, but are not limited to, cosmids, plasmids, or modified viruses, but it will be appreciated that the vector system must be compatible with the selected host cell. Such vectors include, but are not limited to plasmids such as Bluescript® plasmid derivatives (a high copy number ColE1-based phagemid, Stratagene Cloning Systems, La Jolla CA), PCR cloning plasmids designed for cloning Taq-amplified PCR products (*e.g.,* TOPO™ TA Cloning® Kit, PCR2.1® plasmid derivatives, Invitrogen, Carlsbad, CA), and mammalian, yeast or virus vectors such as a baculovirus expression system (pBacPAK plasmid derivatives, Clontech, Palo Alto, CA).

After the vector has been constructed and a nucleic acid molecule encoding an FGF-23 polypeptide has been inserted into the proper site of the vector, the completed vector may be inserted into a suitable host cell for amplification and/or polypeptide expression. The transformation of an expression vector for an FGF-23 polypeptide into a selected host cell may be accomplished by well known methods including methods such as transfection, infection, calcium chloride, electroporation, microinjection, lipofection, DEAE-dextran method, or other known techniques. The method selected will in part be a function of the type of host cell to be used. These methods and other suitable methods are well known to the skilled artisan, and are set forth, for example, in Sambrook *et al., supra.*

Host cells may be prokaryotic host cells (such as *E. coli*) or eukaryotic host cells (such as a yeast, insect, or vertebrate cell). The host cell, when cultured under appropriate conditions, synthesizes an FGF-23 polypeptide which can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity (such as glycosylation or phosphorylation) and ease of folding into a biologically active molecule.

A number of suitable host cells are known in the art and many are available from the American Type Culture Collection (ATCC), Manassas, VA. Examples include, but are not limited to, mammalian cells, such as Chinese hamster ovary cells (CHO), CHO DHFR(-) cells (Urlaub et al., 1980, Proc. Natl. Acad. Sci. US.A. 97:4216-20), human embryonic kidney (HEK) 293 or 293T cells, or 3T3 cells. The selection of suitable mammalian host cells and methods for transformation, culture, amplification, screening, product production, and purification are known in the art. Other suitable mammalian cell lines, are the monkey COS-1 and COS-7 cell lines, and the CV-1 cell line. Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines. Each of these cell lines is known by and available to those skilled in the art of protein expression.

Similarly useful as host cells suitable for the present invention are bacterial cells. For example, the various strains of *E. coli* (*e.g.,* HB101, DH5α, DH10, and MC1061) are well-known as host cells in the field of biotechnology. Various strains of *B. subtilis, Pseudomonas spp.,* other *Bacillus spp., Streptomyces spp.,* and the like may also be employed in this method.

Many strains of yeast cells known to those skilled in the art are also available as host cells for the expression of the polypeptides of the present invention. Preferred yeast cells include, for example, *Saccharomyces cerivisae* and *Pichia pastoris.*

Additionally, where desired, insect cell systems may be utilized in the methods of the present invention. Such systems are described, for example, in Kitts et al., 1993, Biotechniques, 14:810-17; Lucklow, 1993, Curr. Opin. Biotechnol. 4:564-72; and Lucklow et al., 1993, J. Virol., 67:4566-79. Preferred insect cells are Sf-9 and Hi5 (Invitrogen).

One may also use transgenic animals to express glycosylated FGF-23 polypeptides. For example, one may use a transgenic milk-producing animal (a cow or goat, for example) and obtain the present glycosylated polypeptide in the animal milk. One may also use plants to produce FGF-23 polypeptides, however, in general, the glycosylation occurring in plants is different from that produced in mammalian cells, and may result in a glycosylated product which is not suitable for human therapeutic use.

Polypeptide Production

Host cells comprising an FGF-23 polypeptide expression vector may be cultured using standard media well known to the skilled artisan. The media will usually contain all nutrients necessary for the growth and survival of the cells. Suitable media for culturing *E. coli* cells include, for example, Luria Broth (LB) and/or Terrific Broth (TB). Suitable media for culturing eukaryotic cells include Roswell Park Memorial Institute medium 1640 (RPMI 1640), Minimal Essential Medium (MEM) and/or Dulbecco's Modified Eagle Medium (DMEM), all of which may be supplemented with serum and/or growth factors as necessary for the particular cell line being cultured. A suitable medium for insect cultures is Grace's medium supplemented with yeastolate, lactalbumin hydrolysate, and/or fetal calf serum as necessary.

Typically, an antibiotic or other compound useful for selective growth of transfected or transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline, and neomycin.

The amount of an FGF-23 polypeptide produced by a host cell can be evaluated using standard methods known in the art. Such methods include, without limitation, Western blot analysis, SDS-polyacrylamide gel electrophoresis, non-denaturing gel electrophoresis, High Performance Liquid Chromatography (HPLC) separation, immunoprecipitation, and/or activity assays such as DNA binding gel shift assays.

If an FGF-23 polypeptide has been designed to be secreted from the host cells, the majority of polypeptide may be found in the cell culture medium. If however, the FGF-23 polypeptide is not secreted from the host cells, it will be present in the cytoplasm and/or the nucleus (for eukaryotic host cells) or in the cytosol (for gram-negative bacteria host cells).

For an FGF-23 polypeptide situated in the host cell cytoplasm and/or nucleus (for eukaryotic host cells) or in the cytosol (for bacterial host cells), the intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation.

If an FGF-23 polypeptide has formed inclusion bodies in the cytosol, the inclusion bodies can often bind to the inner and/or outer cellular membranes and thus will be found primarily in the pellet material after centrifugation. The pellet material can then be treated at pH extremes or with a chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The solubilized FGF-23 polypeptide can then be analyzed using gel electrophoresis, immunoprecipitation, or the like. If it is desired to isolate the FGF-23 polypeptide, isolation may be accomplished using standard methods such as those described herein and in Marston et al., 1990, Meth. Enz., 182:264-75.

In some cases, an FGF-23 polypeptide may not be biologically active upon isolation. Various methods for "refolding" or converting the polypeptide to its tertiary structure and generating disulfide linkages can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridges. Some of the commonly used redox couples include cysteine/cystamine, glutathione (GSH)/dithiobis GSH, cupric chloride, dithiothreitol(DTT)/dithiane DTT, and 2-2-mercaptoethanol(bME)/dithio-b(ME). In many instances, a cosolvent may be used or may be needed to increase the efficiency of the refolding, and the more common reagents used for this purpose include glycerol, polyethylene glycol of various molecular weights, arginine and the like.

If inclusion bodies are not formed to a significant degree upon expression of an FGF-23 polypeptide, then the polypeptide will be found primarily in the supernatant after centrifugation of the cell homogenate. The polypeptide may be further isolated from the supernatant using methods such as those described herein.

The purification of an FGF-23 polypeptide from solution can be accomplished using a variety of techniques. If the polypeptide has been synthesized such that it contains a tag such as Hexahistidine (FGF-23 polypeptide/hexaHis) or other small peptide such as FLAG (Eastman Kodak Co., New Haven, CT) or *myc* (Invitrogen, Carlsbad, CA) at either its carboxyl- or amino-terminus, it may be purified in a one-step process by passing the solution through an affinity column where the column matrix has a high affinity for the tag.

For example, polyhistidine binds with great affinity and specificity to nickel. Thus, an affinity column of nickel (such as the Qiagen® nickel columns) can be used for purification of FGF-23 polypeptide/polyHis. *See, e.g.,* Current Protocols in Molecular Biology § 10.11.8 (Ausubel et al., eds., Green Publishers Inc. and Wiley and Sons 1993).

Additionally, FGF-23 polypeptides may be purified through the use of a monoclonal antibody that is capable of specifically recognizing and binding to an FGF-23 polypeptide.

Other suitable procedures for purification include, without limitation, affinity chromatography, immunoaffinity chromatography, ion exchange chromatography, molecular sieve chromatography, HPLC, electrophoresis (including native gel electrophoresis) followed by gel elution, and preparative isoelectric focusing ("Isoprime" machine/technique, Hoefer Scientific, San Francisco, CA). In some cases, two or more purification techniques may be combined to achieve increased purity.

FGF-23 polypeptides may also be prepared by chemical synthesis methods (such as solid phase peptide synthesis) using techniques known in the art such as those set forth by Merrifield et al., 1963, J. Am. Chem. Soc. 85: 2149; Houghten et al., 1985, Proc Natl Acad. Sci. USA 82:5132; and Stewart and Young, Solid Phase Peptide Synthesis (Pierce Chemical Co. 1984). Such polypeptides may be synthesized with or without a methionine on the amino-terminus. Chemically synthesized FGF-23 polypeptides may be oxidized using methods set forth in these references to form disulfide bridges. Chemically synthesized FGF-23 polypeptides are expected to have comparable biological activity to the corresponding FGF-23 polypeptides produced recombinantly or purified from natural sources, and thus may be used interchangeably with a recombinant or natural FGF-23 polypeptide.

Another means of obtaining FGF-23 polypeptide is via purification from biological samples such as source tissues and/or fluids in which the FGF-23 polypeptide is naturally found. Such purification can be conducted using methods for protein purification as described herein. The presence of the FGF-23 polypeptide during purification may be monitored, for example, using an antibody prepared against recombinantly produced FGF-23 polypeptide or peptide fragments thereof.

A number of additional methods for producing nucleic acids and polypeptides are known in the art, and the methods can be used to produce polypeptides having specificity for FGF-23 polypeptide. *See, e.g.,* Roberts et al., 1997, Proc. Natl. Acad. Sci. U.S.A. 94:12297-303, which describes the production of fusion proteins between an mRNA and its encoded peptide. *See also,* Roberts, 1999, Curr. Opin. Chem. Biol. 3:268-73. Additionally, U.S. Patent No. 5,824,469 describes methods for obtaining oligonucleotides capable of carrying out a specific biological function. The procedure involves generating a heterogeneous pool of oligonucleotides, each having a 5' randomized sequence, a central preselected sequence, and a 3' randomized sequence. The resulting heterogeneous pool is introduced into a population of cells that do not exhibit the desired biological function. Subpopulations of the cells are then screened for those that exhibit a predetermined biological function. From that subpopulation, oligonucleotides capable of carrying out the desired biological function are isolated.

U.S. Patent Nos. 5,763,192; 5,814,476; 5,723,323; and 5,817,483 describe processes for producing peptides or polypeptides. This is done by producing stochastic genes or fragments thereof, and then introducing these genes into host cells which produce one or more proteins encoded by the stochastic genes. The host cells are then screened to identify those clones producing peptides or polypeptides having the desired activity.

Another method for producing peptides or polypeptides is described in PCT/US98/20094 (WO99/15650) filed by Athersys, Inc. Known as "Random Activation of Gene Expression for Gene Discovery" (RAGE-GD), the process involves the activation of endogenous gene expression or over-expression of a gene by *in situ* recombination methods. For example, expression of an endogenous gene is activated or increased by integrating a regulatory sequence into the target cell which is capable of activating expression of the gene by non-homologous or illegitimate recombination. The target DNA is first subjected to radiation, and a genetic promoter inserted. The promoter eventually locates a break at the front of a gene, initiating transcription of the gene. This results in expression of the desired peptide or polypeptide.

It will be appreciated that these methods can also be used to create comprehensive FGF-23 polypeptide expression libraries, which can subsequently be used for high throughput phenotypic screening in a variety of assays, such as biochemical assays, cellular assays, and whole organism assays (*e.g.*, plant, mouse, etc.).

Synthesis

It will be appreciated by those skilled in the art that the nucleic acid and polypeptide molecules described herein may be produced by recombinant and other means.

Selective Binding Agents

The term "selective binding agent" refers to a molecule that has specificity for one or more FGF-23 polypeptides. Suitable selective binding agents include, but are not limited to, antibodies and derivatives thereof, polypeptides, and small molecules. Suitable selective binding agents may be prepared using methods known in the art. An exemplary FGF-23 polypeptide selective binding agent of the present invention is capable of binding a certain portion of the FGF-23 polypeptide thereby inhibiting the binding of the polypeptide to an FGF-23 polypeptide receptor.

Selective binding agents such as antibodies and antibody fragments that bind FGF-23 polypeptides are within the scope of the present invention. The antibodies may be polyclonal including monospecific polyclonal; monoclonal (MAbs); recombinant; chimeric; humanized, such as CDR-grafted; human; single chain; and/or bispecific; as well as fragments; variants; or derivatives thereof. Antibody fragments include those portions of the antibody that bind to an epitope on the FGF-23 polypeptide. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Polyclonal antibodies directed toward an FGF-23 polypeptide generally are produced in animals (*e.g.,* rabbits or mice) by means of multiple subcutaneous or intraperitoneal injections of FGF-23 polypeptide and an adjuvant. It may be useful to conjugate an FGF-23 polypeptide to a carrier protein that is immunogenic in the species to be immunized, such as keyhole limpet hemocyanin, serum, albumin, bovine thyroglobulin, or soybean trypsin inhibitor. Also, aggregating agents such as alum are used to enhance the immune response. After immunization, the animals are bled and the serum is assayed for anti-FGF-23 antibody titer.

Monoclonal antibodies directed toward FGF-23 polypeptides are produced using any method that provides for the production of antibody molecules by continuous cell lines in culture. Examples of suitable methods for preparing monoclonal antibodies include the hybridoma methods of Kohler et al., 1975, Nature 256:495-97 and the human B-cell hybridoma method (Kozbor, 1984, J. Immunol. 133:3001; Brodeur et al., Monoclonal Antibody Production Techniques and Applications 51-63 (Marcel Dekker, Inc., 1987). Also provided by the invention are hybridoma cell lines that produce monoclonal antibodies reactive with FGF-23 polypeptides.

Monoclonal antibodies of the invention may be modified for use as therapeutics. One embodiment is a "chimeric" antibody in which a portion of the heavy (H) and/or light (L) chain is identical with or homologous to a corresponding sequence in antibodies derived from a particular species or belonging to a particular antibody class or subclass, while the remainder of the chain(s) is/are identical with or homologous to a corresponding sequence in antibodies derived from another species or belonging to another antibody class or subclass. Also included are fragments of such antibodies, so long as they exhibit the desired biological activity. *See* U.S. Patent No. 4,816,567; Morrison et al., 1985, Proc. Natl. Acad. Sci. 81:6851-S5.

In another embodiment, a monoclonal antibody of the invention is a "humanized" antibody. Methods for humanizing non-human antibodies are well known in the art. *See* U.S. Patent Nos. 5,585,089 and 5,693,762. Generally, a humanized antibody has one or more amino acid residues introduced into it from a source that is non-human. Humanization can be performed, for example, using methods described in the art (Jones et al., 1986, Nature 321:522-25; Riechmann et al., 1998, Nature 332:323-27; Verhoeyen et al., 1988, Science 239:1534-36), by substituting at least a portion of a rodent complementarity-determining region (CDR) for the corresponding regions of a human antibody.

Also encompassed by the invention are human antibodies that bind FGF-23 polypeptides. Using transgenic animals (*e.g.*, mice) that are capable of producing a repertoire of human antibodies in the absence of endogenous immunoglobulin production such antibodies are produced by immunization with an FGF-23 polypeptide antigen (*i.e.,* having at least 6 contiguous amino acids), optionally conjugated to a carrier. *See, e.g.,* Jakobovits et al., 1993, Proc. Natl. Acad. Sci. 90:2551-55; Jakobovits et al., 1993, Nature 362:255-58; Bruggermann et al., 1993, Year in Immuno. 7: 33. In one method, such transgenic animals are produced by incapacitating the endogenous loci encoding the heavy and light immunoglobulin chains therein, and inserting loci encoding human heavy and light chain proteins into the genome thereof. Partially modified animals, that is those having less than the full complement of modifications, are then cross-bred to obtain an animal having all of the desired immune system modifications. When administered an immunogen, these transgenic animals produce antibodies with human (rather than, *e.g.,* murine) amino acid sequences, including variable regions which are immunospecific for these antigens. *See* PCT App. Nos. PCT/US96/05928 and PCT/US93/06926. Additional methods are described in U.S. Patent No. 5,545,807, PCT App. Nos. PCT/US91/245 and PCT/GB89/01207, and in European Patent Nos. 546073B1 and 546073A1. Human antibodies can also be produced by the expression of recombinant DNA in host cells or by expression in hybridoma cells as described herein.

In an alternative embodiment, human antibodies can also be produced from phage-display libraries (Hoogenboom et al., 1991, J. Mol. Biol. 227:381; Marks et al., 1991, J. Mol. Biol. 222:581). These processes mimic immune selection through the display of antibody repertoires on the surface of filamentous bacteriophage, and subsequent selection of phage by their binding to an antigen of choice. One such technique is described in PCT App. No. PCT/US98/17364, which describes the isolation of high affinity and functional agonistic antibodies for MPL- and msk-receptors using such an approach.

Chimeric, CDR grafted, and humanized antibodies are typically produced by recombinant methods. Nucleic acids encoding the antibodies are introduced into host cells and expressed using materials and procedures described herein. In a preferred embodiment, the antibodies are produced in mammalian host cells, such as CHO cells. Monoclonal (*e.g.,* human) antibodies may be produced by the expression of recombinant DNA in host cells or by expression in hybridoma cells as described herein.

The anti-FGF-23 antibodies of the invention may be employed in any known assay method, such as competitive binding assays, direct and indirect sandwich assays, and immunoprecipitation assays (Sola, Monoclonal Antibodies: A Manual of Techniques 147-158 (CRC Press, Inc., 1987)) for the detection and quantitation of FGF-23 polypeptides. The antibodies will bind FGF-23 polypeptides with an affinity that is appropriate for the assay method being employed:

For diagnostic applications, in certain embodiments, anti-FGF-23 antibodies may be labeled with a detectable moiety. The detectable moiety can be any one that is capable of producing, either directly or indirectly, a detectable signal. For example, the detectable moiety may be a radioisotope, such as $^3$H, $^{14}$C, $^{32}$P, $^{35}$S, $^{125}$I, $^{99}$Tc, $^{111}$In, or $^{67}$Ga; a fluorescent or chemiluminescent compound, such as fluorescein isothiocyanate, rhodamine, or luciferin; or an enzyme, such as alkaline phosphatase, β-galactosidase, or horseradish peroxidase (Bayer, et al., 1990, Meth. Enz. 184:138-63).

Competitive binding assays rely on the ability of a labeled standard (*e.g.*, an FGF-23 polypeptide, or an immunologically reactive portion thereof) to compete with the test sample analyte (an FGF-23 polypeptide) for binding with a limited amount of anti-FGF-23 antibody. The amount of an FGF-23 polypeptide in the test sample is inversely proportional to the amount of standard that becomes bound to the antibodies. To facilitate determining the amount of standard that becomes bound, the antibodies typically are insolubilized before or after the competition, so that the standard and analyte that are bound to the antibodies may conveniently be separated from the standard and analyte which remain unbound.

Sandwich assays typically involve the use of two antibodies, each capable of binding to a different immunogenic portion, or epitope, of the protein to be detected and/or quantitated. In a sandwich assay, the test sample analyte is typically bound by a first antibody which is immobilized on a solid support, and thereafter a second antibody binds to the analyte, thus forming an insoluble three-part complex. *See, e.g.,* U.S. Patent No. 4,376,110. The second antibody may itself be labeled with a detectable moiety (direct sandwich assays) or may be measured using an anti-immunoglobulin antibody that is labeled with a detectable moiety (indirect sandwich assays). For example, one type of sandwich assay is an enzyme-linked immunosorbent assay (ELISA), in which case the detectable moiety is an enzyme.

The selective binding agents, including anti-FGF-23 antibodies, are also useful for *in vivo* imaging. An antibody labeled with a detectable moiety may be administered to an animal, preferably into the bloodstream, and the presence and location of the labeled antibody in the host assayed. The antibody may be labeled with any moiety that is detectable in an animal, whether by nuclear magnetic resonance, radiology, or other detection means known in the art.

Selective binding agents of the invention, including antibodies, may be used as therapeutics. These therapeutic agents are generally agonists or antagonists, in that they either enhance or reduce, respectively, at least one of the biological activities of an FGF-23 polypeptide. In one embodiment, antagonist antibodies of the invention are antibodies or binding fragments thereof which are capable of specifically binding to an FGF-23 polypeptide and which are capable of inhibiting or eliminating the functional activity of an FGF-23 polypeptide *in vivo* or *in vitro.* In preferred embodiments, the selective binding agent, *e.g.,* an antagonist antibody, will inhibit the functional activity of an FGF-23 polypeptide by at least about 50%, and preferably by at least about 80%. In another embodiment, the selective binding agent may be an anti-FGF-23 polypeptide antibody that is capable of interacting with an FGF-23 polypeptide binding partner (a ligand or receptor) thereby inhibiting or eliminating FGF-23 polypeptide activity *in vitro* or *in vivo.* Selective binding agents, including agonist and antagonist anti-FGF-23 polypeptide antibodies, are identified by screening assays that are well known in the art.

The invention also relates to a kit comprising FGF-23 selective binding agents (such as antibodies) and other reagents useful for detecting FGF-23 polypeptide levels in biological samples. Such reagents may include a detectable label, blocking serum, positive and negative control samples, and detection reagents.

Microarrays

It will be appreciated that DNA microarray technology can be utilized in accordance with the present invention. DNA microarrays are miniature, high-density arrays of nucleic acids positioned on a solid support, such as glass. Each cell or element within the array contains numerous copies of a single nucleic acid species that acts as a target for hybridization with a complementary nucleic acid sequence (*e.g.,* mRNA). In expression profiling using DNA microarray technology, mRNA is first extracted from a cell or tissue sample and then converted enzymatically to fluorescently labeled cDNA. This material is hybridized to the microarray and unbound cDNA is removed by washing. The expression of discrete genes represented on the array is then visualized by quantitating the amount of labeled cDNA that is specifically bound to each target nucleic acid molecule. In this way, the expression of thousands of genes can be quantitated in a high throughput, parallel manner from a single sample of biological material.

This high throughput expression profiling has a broad range of applications with respect to the FGF-23 molecules of the invention, including, but not limited to: the identification and validation of FGF-23 disease-related genes as targets for therapeutics; molecular toxicology of related FGF-23 molecules and inhibitors thereof; stratification of populations and generation of surrogate markers for clinical trials; and enhancing related FGF-23 polypeptide small molecule drug discovery by aiding in the identification of selective compounds in high throughput screens.

Chemical Derivatives

Chemically modified derivatives of FGF-23 polypeptides may be prepared by one skilled in the art, given the disclosures described herein. FGF-23 polypeptide derivatives are modified in a manner that is different - either in the type or location of the molecules naturally attached to the polypeptide. Derivatives may include molecules formed by the deletion of one or more naturally-attached chemical groups. The polypeptide comprising the amino acid sequence of SEQ ID NO: 2, or other FGF-23 polypeptide, may be modified by the covalent attachment of one or more polymers. For example, the polymer selected is typically water-soluble so that the protein to which it is attached does not precipitate in an aqueous environment, such as a physiological environment. Included within the scope of suitable polymers is a mixture of polymers. Preferably, for therapeutic use of the end-product preparation, the polymer will be pharmaceutically acceptable.

The polymers each may be of any molecular weight and may be branched or unbranched. The polymers each typically have an average molecular weight of between about 2 kDa to about 100 kDa (the term "about" indicating that in preparations of a water-soluble polymer, some molecules will weigh more, some less, than the stated molecular weight). The average molecular weight of each polymer is preferably between about 5 kDa and about 50 kDa, more preferably between about 12 kDa and about 40 kDa and most preferably between about 20 kDa and about 35 kDa.

Suitable water-soluble polymers or mixtures thereof include, but are not limited to, N-linked or O-linked carbohydrates, sugars, phosphates, polyethylene glycol (PEG) (including the forms of PEG that have been used to derivatize proteins, including mono-($C_1$-$C_{10}$), alkoxy-, or aryloxy-polyethylene glycol), monomethoxy-polyethylene glycol, dextran (such as low molecular weight dextran of, for example, about 6 kD), cellulose, or other carbohydrate based polymers, poly-(N-vinyl pyrrolidone) polyethylene glycol, propylene glycol homopolymers, polypropylene oxide/ethylene oxide co-polymers, polyoxyethylated polyols (*e.g.*, glycerol), and polyvinyl alcohol. Also encompassed by the present invention are bifunctional crosslinking molecules which may be used to prepare covalently attached FGF-23 polypeptide multimers.

In general, chemical derivatization may be performed under any suitable condition used to react a protein with an activated polymer molecule. Methods for preparing chemical derivatives of polypeptides will generally comprise the steps of: (a) reacting the polypeptide with the activated polymer molecule (such as a reactive ester or aldehyde derivative of the polymer molecule) under conditions whereby the polypeptide comprising the amino acid sequence of SEQ ID NO: 2, or other FGF-23 polypeptide, becomes attached to one or more polymer molecules, and (b) obtaining the reaction products. The optimal reaction conditions will be determined based on known parameters and the desired result. For example, the larger the ratio of polymer molecules to protein, the greater the percentage of attached polymer molecule. In one embodiment, the FGF-23 polypeptide derivative may have a single polymer molecule moiety at the amino-terminus. *See, e.g.,* U.S. Patent No. 5,234,784.

The pegylation of a polypeptide may be specifically carried out using any of the pegylation reactions known in the art. Such reactions are described, for example, in the following references: Francis et al., 1992, Focus on Growth Factors 3:4-10; European Patent Nos. 0154316 and 0401384; and U.S. Patent No. 4,179,337. For example, pegylation may be carried out via an acylation reaction or an alkylation reaction with a reactive polyethylene glycol molecule (or an analogous reactive water-soluble polymer) as described herein. For the acylation reactions, a selected polymer should have a single reactive ester group. For reductive alkylation, a selected polymer should have a single reactive aldehyde group. A reactive aldehyde is, for example, polyethylene glycol propionaldehyde, which is water stable, or mono $C_1$-$C_{10}$ alkoxy or aryloxy derivatives thereof *(see* U.S. Patent No. 5,252,714).

In another embodiment, FGF-23 polypeptides may be chemically coupled to biotin. The biotin/FGF-23 polypeptide molecules are then allowed to bind to avidin, resulting in tetravalent avidin/biotin/FGF-23 polypeptide molecules. FGF-23 polypeptides may also be covalently coupled to dinitrophenol (DNP) or trinitrophenol (TNP) and the resulting conjugates precipitated with anti-DNP or anti-TNP-IgM to form decameric conjugates with a valency of 10.

Generally, conditions that may be alleviated or modulated by the administration of the present FGF-23 polypeptide derivatives include those described herein for FGF-23 polypeptides. However, the FGF-23 polypeptide derivatives disclosed herein may have additional activities, enhanced or reduced biological activity, or other characteristics, such as increased or decreased half-life, as compared to the non-derivatized molecules.

Genetically Engineered Non-Human Animals

Additionally included within the scope of the present invention are non-human animals such as mice, rats, or other rodents; rabbits, goats, sheep, or other farm animals, in which the genes encoding native FGF-23 polypeptide have been disrupted (*i.e.,* "knocked out") such that the level of expression of FGF-23 polypeptide is significantly decreased or completely abolished. Such animals may be prepared using techniques and methods such as those described in U.S. Patent No. 5,557,032.

The present invention further includes non-human animals such as mice, rats, or other rodents; rabbits, goats, sheep, or other farm animals, in which either the native form of an FGF-23 gene for that animal or a heterologous FGF-23 gene is over-expressed by the animal, thereby creating a "transgenic" animal. Such transgenic animals may be prepared using well known methods such as those described in U.S. Patent No 5,489,743 and PCT Pub. No. WO 94/28122.

The present invention further includes non-human animals in which the promoter for one or more of the FGF-23 polypeptides of the present invention is either activated or inactivated (e.g., by using homologous recombination methods) to alter the level of expression of one or more of the native FGF-23 polypeptides.

These non-human animals may be used for drug candidate screening. In such screening, the impact of a drug candidate on the animal may be measured. For example, drug candidates may decrease or increase the expression of the FGF-23 gene. In certain embodiments, the amount of FGF-23 polypeptide that is produced may be measured after the exposure of the animal to the drug candidate. Additionally, in certain embodiments, one may detect the actual impact of the drug candidate on the animal. For example, over-expression of a particular gene may result in, or be associated with, a disease or pathological condition. In such cases, one may test a drug candidate's ability to decrease expression of the gene or its ability to prevent or inhibit a pathological condition. In other examples, the production of a particular metabolic product such as a fragment of a polypeptide, may result in, or be associated with, a disease or pathological condition. In such cases, one may test a drug candidate's ability to decrease the production of such a metabolic product or its ability to prevent or inhibit a pathological condition.

Assaying for Other Modulators of FGF-23 Polypeptide Activity

In some situations, it may be desirable to identify molecules that are modulators, i.e., agonists or antagonists, of the activity of FGF-23 polypeptide. Natural or synthetic molecules that modulate FGF-23 polypeptide may be identified using one or more screening assays, such as those described herein. Such molecules may be administered either in an *ex vivo* manner or in an *in vivo* manner by injection, or by oral delivery, implantation device, or the like.

"Test molecule" refers to a molecule that is under evaluation for the ability to modulate (*i.e.*, increase or decrease) the activity of an FGF-23 polypeptide. Most commonly, a test molecule will interact directly with an FGF-23 polypeptide. However, it is also contemplated that a test molecule may also modulate FGF-23 polypeptide activity indirectly, such as by affecting FGF-23 gene expression, or by binding to an FGF-23 polypeptide binding partner (e.g., receptor or ligand). In one embodiment, a test molecule will bind to an FGF-23 polypeptide with an affinity constant of at least about $10^{-6}$ M, preferably about $10^{-8}$ M, more preferably about $10^{-9}$ M, and even more preferably about $10^{-10}$ M.

Methods for identifying compounds that interact with FGF-23 polypeptides are encompassed by the present invention. In certain embodiments, an FGF-23 polypeptide is incubated with a test molecule under conditions that permit the interaction of the test molecule with an FGF-23 polypeptide, and the extent of the interaction is measured. The test molecule can be screened in a substantially purified form or in a crude mixture.

In certain embodiments, an FGF-23 polypeptide agonist or antagonist may be a protein, peptide, carbohydrate, lipid, or small molecular weight molecule that interacts with FGF-23 polypeptide to regulate its activity. Molecules which regulate FGF-23 polypeptide expression include nucleic acids which are complementary to nucleic acids encoding an FGF-23 polypeptide, or are complementary to nucleic acids sequences which direct or control the expression of FGF-23 polypeptide, and which act as anti-sense regulators of expression.

Once a test molecule has been identified as interacting with an FGF-23 polypeptide, the molecule may be further evaluated for its ability to increase or decrease FGF-23 polypeptide activity. The measurement of the interaction of a test molecule with FGF-23 polypeptide may be carried out in several formats, including cell-based binding assays, membrane binding assays, solution-phase assays, and immunoassays. In general, a test molecule is incubated with an FGF-23 polypeptide for a specified period of time, and FGF-23 polypeptide activity is determined by one or more assays for measuring biological activity.

The interaction of test molecules with FGF-23 polypeptides may also be assayed directly using polyclonal or monoclonal antibodies in an immunoassay. Alternatively, modified forms of FGF-23 polypeptides containing epitope tags as described herein may be used in solution and immunoassays.

In the event that FGF-23 polypeptides display biological activity through an interaction with a binding partner (e.g., a receptor or a ligand), a variety of *in vitro* assays may be used to measure the binding of an FGF-23 polypeptide to the corresponding binding partner (such as a selective binding agent, receptor, or ligand). These assays may be used to screen test molecules for their ability to increase or decrease the rate and/or the extent of binding of an FGF-23 polypeptide to its binding partner. In one assay, an FGF-23 polypeptide is immobilized in the wells of a microtiter plate. Radiolabeled FGF-23 polypeptide binding partner (for example, iodinated FGF-23 polypeptide binding partner) and a test molecule can then be added either one at a time (in either order) or simultaneously to the wells. After incubation, the wells can be washed and counted for radioactivity, using a scintillation counter, to determine the extent to which the binding partner bound to the FGF-23 polypeptide. Typically, a molecule will be tested over a range of concentrations, and a series of control wells lacking one or more elements of the test assays can be used for accuracy in the evaluation of the results. An alternative to this method involves reversing the "positions" of the proteins, *i.e.,* immobilizing FGF-23 polypeptide binding partner to the microtiter plate wells, incubating with the test molecule and radiolabeled FGF-23 polypeptide, and determining the extent of FGF-23 polypeptide binding. *See, e.g.,* Current Protocols in Molecular Biology, chap. 18 (Ausubel et al., eds., Green Publishers Inc. and Wiley and Sons 1995).

As an alternative to radiolabeling, an FGF-23 polypeptide or its binding partner may be conjugated to biotin, and the presence of biotinylated protein can then be detected using streptavidin linked to an enzyme, such as horse radish peroxidase (HRP) or alkaline phosphatase (AP), which can be detected colorometrically, or by fluorescent tagging of streptavidin. An antibody directed to an FGF-23 polypeptide or to an FGF-23 polypeptide binding partner, and which is conjugated to biotin, may also be used for purposes of detection following incubation of the complex with enzyme-linked streptavidin linked to AP or HRP.

A FGF-23 polypeptide or an FGF-23 polypeptide binding partner can also be immobilized by attachment to agarose beads, acrylic beads, or other types of such inert solid phase substrates. The substrate-protein complex can be placed in a solution containing the complementary protein and the test compound. After incubation, the beads can be precipitated by centrifugation, and the amount of binding between an FGF-23 polypeptide and its binding partner can be assessed using the methods described herein. Alternatively, the substrate-protein complex can be immobilized in a column with the test molecule and complementary protein passing through the column. The formation of a complex between an FGF-23 polypeptide and its binding partner can then be assessed using any of the techniques described herein (*e.g.*, radiolabelling or antibody binding).

Another *in vitro* assay that is useful for identifying a test molecule which increases or decreases the formation of a complex between an FGF-23 polypeptide binding protein and an FGF-23 polypeptide binding partner is a surface plasmon resonance detector system such as the BIAcore assay system (Pharmacia, Piscataway, NJ). The BIAcore system is utilized as specified by the manufacturer. This assay essentially involves the covalent binding of either FGF-23 polypeptide or an FGF-23 polypeptide binding partner to a dextran-coated sensor chip that is located in a detector. The test compound and the other complementary protein can then be injected, either simultaneously or sequentially, into the chamber containing the sensor chip. The amount of complementary protein that binds can be assessed based on the change in molecular mass that is physically associated with the dextran-coated side of the sensor chip, with the change in molecular mass being measured by the detector system.

In some cases, it may be desirable to evaluate two or more test compounds together for their ability to increase or decrease the formation of a complex between an FGF-23 polypeptide and an FGF-23 polypeptide binding partner. In these cases, the assays set forth herein can be readily modified by adding such additional test compound(s) either simultaneously with, or subsequent to, the first test compound. The remainder of the steps in the assay are as set forth herein.

*In vitro* assays such as those described herein may be used advantageously to screen large numbers of compounds for an effect on the formation of a complex between an FGF-23 polypeptide and FGF-23 polypeptide binding partner. The assays may be automated to screen compounds generated in phage display, synthetic peptide, and chemical synthesis libraries.

Compounds which increase or decrease the formation of a complex between an FGF-23 polypeptide and an FGF-23 polypeptide binding partner may also be screened in cell culture using cells and cell lines expressing either FGF-23 polypeptide or FGF-23 polypeptide binding partner. Cells and cell lines may be obtained from any mammal, but preferably will be from human or other primate, canine, or rodent sources. The binding of an FGF-23 polypeptide to cells expressing FGF-23 polypeptide binding partner at the surface is evaluated in the presence or absence of test molecules, and the extent of binding may be determined by, for example, flow cytometry using a biotinylated antibody to an FGF-23 polypeptide binding partner. Cell culture assays can be used advantageously to further evaluate compounds that score positive in protein binding assays described herein.

Cell cultures can also be used to screen the impact of a drug candidate. For example, drug candidates may decrease or increase the expression of the FGF-23 gene. In certain embodiments, the amount of FGF-23 polypeptide or an FGF-23 polypeptide fragment that is produced may be measured after exposure of the cell culture to the drug candidate. In certain embodiments, one may detect the actual impact of the drug candidate on the cell culture. For example, the over-expression of a particular gene may have a particular impact on the cell culture. In such cases, one may test a drug candidate's ability to increase or decrease the expression of the gene or its ability to prevent or inhibit a particular impact on the cell culture. In other examples, the production of a particular metabolic product such as a fragment of a polypeptide, may result in, or be associated with, a disease or pathological condition. In such cases, one may test a drug candidate's ability to decrease the production of such a metabolic product in a cell culture.

Internalizing Proteins

The *tat* protein sequence (from HIV) can be used to internalize proteins into a cell. *See, e.g.,* Falwell et al., 1994, Proc. Natl. Acad. Sci. U.S.A. 91:664-68. For example, an 11 amino acid sequence (Y-G-R-K-K-R-R-Q-R-R-R; SEQ ID NO: 40) of the HIV tat protein (termed the "protein transduction domain," or TAT PDT) has been described as mediating delivery across the cytoplasmic membrane and the nuclear membrane of a cell. *See* Schwarze et al., 1999, Science 285:1569-72; and Nagahara et al., 1998, Nat. Med. 4:1449-52. In these procedures, FITC-constructs (FITC-labeled G-G-G-G-Y-G-R-K-K-R-R-Q-R-R-R; SEQ ID NO: 41), which penetrate tissues following intraperitoneal administration, are prepared, and the binding of such constructs to cells is detected by fluorescence-activated cell sorting (FACS) analysis. Cells treated with a *tat*-β-gal fusion protein will demonstrate β-gal activity. Following injection, expression of such a construct can be detected in a number of tissues, including liver, kidney, lung, heart, and brain tissue. It is believed that such constructs undergo some degree of unfolding in order to enter the cell, and as such, may require a refolding following entry into the cell.

It will thus be appreciated that the *tat* protein sequence may be used to internalize a desired polypeptide into a cell. For example, using the *tat* protein sequence, an FGF-23 antagonist (such as an anti-FGF-23 selective binding agent, small molecule, soluble receptor, or antisense oligonucleotide) can be administered intracellularly to inhibit the activity of an FGF-23 molecule. As used herein, the term "FGF-23 molecule" refers to both FGF-23 nucleic acid molecules and FGF-23 polypeptides as defined herein. Where desired, the FGF-23 protein itself may also be internally administered to a cell using these procedures. *See also,* Straus, 1999, Science 285:1466-67.

Cell Source Identification Using FGF-23 Polypeptide

In accordance with certain embodiments of the invention, it may be useful to be able to determine the source of a certain cell type associated with an FGF-23 polypeptide. For example, it may be useful to determine the origin of a disease or pathological condition as an aid in selecting an appropriate therapy. In certain embodiments, nucleic acids encoding an FGF-23 polypeptide can be used as a probe to identify cells described herein by screening the nucleic acids of the cells with such a probe. In other embodiments, one may use anti-FGF-23 polypeptide antibodies to test for the presence of FGF-23 polypeptide in cells, and thus, determine if such cells are of the types described herein.

FGF-23 Polypeptide Compositions and Administration

Therapeutic compositions are within the scope of the present invention. Such FGF-23 polypeptide pharmaceutical compositions may comprise a therapeutically effective amount of an FGF-23 polypeptide or an FGF-23 nucleic acid molecule in admixture with a pharmaceutically or physiologically acceptable formulation agent selected for suitability with the mode of administration. Pharmaceutical compositions may comprise a therapeutically effective amount of one or more FGF-23 polypeptide selective binding agents in admixture with a pharmaceutically or physiologically acceptable formulation agent selected for suitability with the mode of administration.

Acceptable formulation materials preferably are nontoxic to recipients at the dosages and concentrations employed.

The pharmaceutical composition may contain formulation materials for modifying, maintaining, or preserving, for example, the pH, osmolarity, viscosity, clarity, color, isotonicity, odor, sterility, stability, rate of dissolution or release, adsorption, or penetration of the composition. Suitable formulation materials include, but are not limited to, amino acids (such as glycine, glutamine, asparagine, arginine, or lysine), antimicrobials, antioxidants (such as ascorbic acid, sodium sulfite, or sodium hydrogen-sulfite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton; tromethamine; lecithin; cholesterol or tyloxapal), stability enhancing agents (such as sucrose or sorbitol), tonicity enhancing agents (such as alkali metal halides - preferably sodium or potassium chloride - or mannitol sorbitol), delivery vehicles, diluents, excipients and/or pharmaceutical adjuvants. *See* Remington's Pharmaceutical Sciences (18th Ed., A.R. Gennaro, ed., Mack Publishing Company 1990.

The optimal pharmaceutical composition will be determined by a skilled artisan depending upon, for example, the intended route of administration, delivery format, and desired dosage. *See, e.g., Remington's Pharmaceutical Sciences, supra.* Such compositions may influence the physical state, stability, rate of *in vivo* release, and rate of *in vivo* clearance of the FGF-23 molecule.

The primary vehicle or carrier in a pharmaceutical composition may be either aqueous or non-aqueous in nature. For example, a suitable vehicle or carrier for injection may be water, physiological saline solution, or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute. In one embodiment of the present invention, FGF-23 polypeptide compositions may be prepared for storage by mixing the selected composition having the desired degree of purity with optional formulation agents (*Remington's Pharmaceutical Sciences, supra*) in the form of a lyophilized cake or an aqueous solution. Further, the FGF-23 polypeptide product may be formulated as a lyophilizate using appropriate excipients such as sucrose.

The FGF-23 polypeptide pharmaceutical compositions can be selected for parenteral delivery. Alternatively, the compositions may be selected for inhalation or for delivery through the digestive tract, such as orally. The preparation of such pharmaceutically acceptable compositions is within the skill of the art.

The formulation components are present in concentrations that are acceptable to the site of administration. For example, buffers are used to maintain the composition at physiological pH or at a slightly lower pH, typically within a pH range of from about 5 to about 8.

When parenteral administration is contemplated, the therapeutic compositions for use in this invention may be in the form of a pyrogen-free, parenterally acceptable, aqueous solution comprising the desired FGF-23 molecule in a pharmaceutically acceptable vehicle. A particularly suitable vehicle for parenteral injection is sterile distilled water in which an FGF-23 molecule is formulated as a sterile, isotonic solution, properly preserved. Yet another preparation can involve the formulation of the desired molecule with an agent, such as injectable microspheres, bio-erodible particles, polymeric compounds (such as polylactic acid or polyglycolic acid), beads, or liposomes, that provides for the controlled or sustained release of the product which may then be delivered via a depot injection. Hyaluronic acid may also be used, and this may have the effect of promoting sustained duration in the circulation. Other suitable means for the introduction of the desired molecule include implantable drug delivery devices.

In one embodiment, a pharmaceutical composition may be formulated for inhalation. For example, FGF-23 polypeptide may be formulated as a dry powder for inhalation. FGF-23 polypeptide or nucleic acid molecule inhalation solutions may also be formulated with a propellant for aerosol delivery. In yet another embodiment, solutions may be nebulized. Pulmonary administration is further described in PCT Pub. No. WO 94/20069, which describes the pulmonary delivery of chemically modified proteins.

It is also contemplated that certain formulations may be administered orally. In one embodiment of the present invention, FGF-23 polypeptides that are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the FGF-23 polypeptide. Diluents, flavorings, low melting point waxes, vegetable oils, lubricants, suspending agents, tablet disintegrating agents, and binders may also be employed.

Another pharmaceutical composition may involve an effective quantity of FGF-23 polypeptides in a mixture with non-toxic excipients that are suitable for the manufacture of tablets. By dissolving the tablets in sterile water, or another appropriate vehicle, solutions can be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional FGF-23 polypeptide pharmaceutical compositions will be evident to those skilled in the art, including formulations involving FGF-23 polypeptides in sustained- or controlled-delivery formulations. Techniques for formulating a variety of other sustained- or controlled-delivery means, such as liposome carriers, bio-erodible microparticles or porous beads and depot injections, are also known to those skilled in the art. *See, e.g.,* PCT/US93/00829, which describes the controlled release of porous polymeric microparticles for the delivery of pharmaceutical compositions.

Additional examples of sustained-release preparations include semipermeable polymer matrices in the form of shaped articles, e.g. films, or microcapsules. Sustained release matrices may include polyesters, hydrogels, polylactides (U.S. Patent No. 3,773,919 and European Patent No. 058481), copolymers of L-glutamic acid and gamma ethyl-L-glutamate (Sidman et al., 1983, Biopolymers 22:547-56), poly(2-hydroxyethyl-methacrylate) (Langer et al., 1981, J. Biomed. Mater. Res. 15:167-277 and Langer, 1982, Chem. Tech. 12:98-105), ethylene vinyl acetate (Langer *et al., supra*) or poly-D(-)-3-hydroxybutyric acid (European Patent No. 133988). Sustained-release compositions may also include liposomes, which can be prepared by any of several methods known in the art. *See, e.g.,* Eppstein et al., 1985, Proc. Natl. Acad. Sci. USA 82:3688-92; and European Patent Nos. 036676, 088046, and 143949.

The FGF-23 pharmaceutical composition to be used for *in vivo* administration typically must be sterile. This may be accomplished by filtration through sterile filtration membranes. Where the composition is lyophilized, sterilization using this method may be conducted either prior to, or following, lyophilization and reconstitution. The composition for parenteral administration may be stored in lyophilized form or in a solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or as a dehydrated or lyophilized powder. Such formulations may be stored either in a ready-to-use form or in a form (*e.g.*, lyophilized) requiring reconstitution prior to administration.

In a specific embodiment, the present invention is directed to kits for producing a single-dose administration unit. The kits may each contain both a first container having a dried protein and a second container having an aqueous formulation. Also included within the scope of this invention are kits containing single and multi-chambered pre-filled syringes (*e.g.*, liquid syringes and lyosyringes).

The effective amount of an FGF-23 pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the FGF-23 molecule is being used, the route of administration, and the size (body weight, body surface, or organ size) and condition (the age and general health) of the patient. Accordingly, the clinician may titer the dosage and modify the route of administration to obtain the optimal therapeutic effect. A typical dosage may range from about 0.1 µg/kg to up to about 100 mg/kg or more, depending on the factors mentioned above. In other embodiments, the dosage may range from 0.1 µg/kg up to about 100 mg/kg; or 1 µg/kg up to about 100 mg/kg; or 5 µg/kg up to about 100 mg/kg.

The frequency of dosing will depend upon the pharmacokinetic parameters of the FGF-23 molecule in the formulation being used. Typically, a clinician will administer the composition until a dosage is reached that achieves the desired effect. The composition may therefore be administered as a single dose, as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. Appropriate dosages may be ascertained through use of appropriate dose-response data.

The route of administration of the pharmaceutical composition is in accord with known methods, *e.g.*, orally; through injection by intravenous, intraperitoneal, intracerebral (intraparenchymal), intracerebroventricular, intramuscular, intraocular, intraarterial, intraportal, or intralesional routes; by sustained release systems; or by implantation devices. Where desired, the compositions may be administered by bolus injection or continuously by infusion, or by implantation device.

Alternatively or additionally, the composition may be administered locally via implantation of a membrane, sponge, or other appropriate material onto which the desired molecule has been absorbed or encapsulated. Where an implantation device is used, the device may be implanted into any suitable tissue or organ, and delivery of the desired molecule may be via diffusion, timed-release bolus, or continuous administration.

In some cases, it may be desirable to use FGF-23 polypeptide pharmaceutical compositions in an *ex vivo* manner. In such instances, cells, tissues, or organs that have been removed from the patient are exposed to FGF-23 polypeptide pharmaceutical compositions after which the cells, tissues, or organs are subsequently implanted back into the patient.

In other cases, an FGF-23 polypeptide can be delivered by implanting certain cells that have been genetically engineered, using methods such as those described herein, to express and secrete the FGF-23 polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semi-permeable polymeric enclosures or membranes that allow the release of the protein product(s) but prevent the destruction of the cells by the patient's immune system or by other detrimental factors from the surrounding tissues.

As discussed herein, it may be desirable to treat isolated cell populations (such as stem cells, lymphocytes, red blood cells, chondrocytes, neurons, and the like) with one or more FGF-23 polypeptides. This can be accomplished by exposing the isolated cells to the polypeptide directly, where it is in a form that is permeable to the cell membrane.

Additional embodiments of the present invention relate to cells and methods (*e.g.*, homologous recombination and/or other recombinant production methods) for both the *in vitro* production of therapeutic polypeptides and for the production and delivery of therapeutic polypeptides by gene therapy or cell therapy. Homologous and other recombination methods may be used to modify a cell that contains a normally transcriptionally-silent FGF-23 gene, or an under-expressed gene, and thereby produce a cell which expresses therapeutically efficacious amounts of FGF-23 polypeptides.

Homologous recombination is a technique originally developed for targeting genes to induce or correct mutations in transcriptionally active genes. Kucherlapati, 1989, Prog. in Nucl. Acid Res. & Mol. Biol. 36:301. The basic technique was developed as a method for introducing specific mutations into specific regions of the mammalian genome (Thomas et al., 1986, Cell 44:419-28; Thomas and Capecchi, 1987, Cell 51:503-12; Doetschman et al., 1988, Proc. Natl. Acad. Sci. U.S.A. 85:8583-87) or to correct specific mutations within defective genes (Doetschman et al., 1987, Nature 330: 576-78). Exemplary homologous recombination techniques are described in U.S. Patent No. 5,272,071; European Patent Nos. 9193051 and 505500; PCT/US90/07642, and PCT Pub No. WO 91/09955).

Through homologous recombination, the DNA sequence to be inserted into the genome can be directed to a specific region of the gene of interest by attaching it to targeting DNA. The targeting DNA is a nucleotide sequence that is complementary (homologous) to a region of the genomic DNA. Small pieces of targeting DNA that are complementary to a specific region of the genome are put in contact with the parental strand during the DNA replication process. It is a general property of DNA that has been inserted into a cell to hybridize, and therefore, recombine with other pieces of endogenous DNA through shared homologous regions. If this complementary strand is attached to an oligonucleotide that contains a mutation or a different sequence or an additional nucleotide, it too is incorporated into the newly synthesized strand as a result of the recombination. As a result of the proofreading function, it is possible for the new sequence of DNA to serve as the template. Thus, the transferred DNA is incorporated into the genome.

Attached to these pieces of targeting DNA are regions of DNA that may interact with or control the expression of an FGF-23 polypeptide, *e.g.*, flanking sequences. For example, a promoter/enhancer element, a suppressor, or an exogenous transcription modulatory element is inserted in the genome of the intended host cell in proximity and orientation sufficient to influence the transcription of DNA encoding the desired FGF-23 polypeptide. The control element controls a portion of the DNA present in the host cell genome. Thus, the expression of the desired FGF-23 polypeptide may be achieved not by transfection of DNA that encodes the FGF-23 gene itself, but rather by the use of targeting DNA (containing regions of homology with the endogenous gene of interest) coupled with DNA regulatory segments that provide the endogenous gene sequence with recognizable signals for transcription of an FGF-23 gene.

In an exemplary method, the expression of a desired targeted gene in a cell (*i.e.,* a desired endogenous cellular gene) is altered via homologous recombination into the cellular genome at a preselected site, by the introduction of DNA which includes at least a regulatory sequence, an exon, and a splice donor site. These components are introduced into the chromosomal (genomic) DNA in such a manner that this, in effect, results in the production of a new transcription unit (in which the regulatory sequence, the exon, and the splice donor site present in the DNA construct are operatively linked to the endogenous gene). As a result of the introduction of these components into the chromosomal DNA, the expression of the desired endogenous gene is altered.

Altered gene expression, as described herein, encompasses activating (or causing to be expressed) a gene which is normally silent (unexpressed) in the cell as obtained, as well as increasing the expression of a gene which is not expressed at physiologically significant levels in the cell as obtained. The embodiments further encompass changing the pattern of regulation or induction such that it is different from the pattern of regulation or induction that occurs in the cell as obtained, and reducing (including eliminating) the expression of a gene which is expressed in the cell as obtained.

One method by which homologous recombination can be used to increase, or cause, FGF-23 polypeptide production from a cell's endogenous FGF-23 gene involves first using homologous recombination to place a recombination sequence from a site-specific recombination system (*e.g.*, Cre/loxP, FLP/FRT) (Sauer, 1994, Curr. Opin. Biotechnol., 5:521-27; Sauer, 1993, Methods Enzymol., 225:890-900) upstream of (*i.e.,* 5' to) the cell's endogenous genomic FGF-23 polypeptide coding region. A plasmid containing a recombination site homologous to the site that was placed just upstream of the genomic FGF-23 polypeptide coding region is introduced into the modified cell line along with the appropriate recombinase enzyme. This recombinase causes the plasmid to integrate, via the plasmid's recombination site, into the recombination site located just upstream of the genomic FGF-23 polypeptide coding region in the cell line (Baubonis and Sauer, 1993, Nucleic Acids Res. 21:2025-29; O'Gorman et al., 1991, Science 251:1351-55). Any flanking sequences known to increase transcription (e.g., enhancer/promoter, intron, translational enhancer), if properly positioned in this plasmid, would integrate in such a manner as to create a new or modified transcriptional unit resulting in *de novo* or increased FGF-23 polypeptide production from the cell's endogenous FGF-23 gene.

A further method to use the cell line in which the site specific recombination sequence had been placed just upstream of the cell's endogenous genomic FGF-23 polypeptide coding region is to use homologous recombination to introduce a second recombination site elsewhere in the cell line's genome. The appropriate recombinase enzyme is then introduced into the two-recombination-site cell line, causing a recombination event (deletion, inversion, and translocation) (Sauer, 1994, Curr. Opin. Biotechnol., 5:521-27; Sauer, 1993, Methods Enzymol., 225:890-900) that would create a new or modified transcriptional unit resulting in *de novo* or increased FGF-23 polypeptide production from the cell's endogenous FGF-23 gene.

An additional approach for increasing, or causing, the expression of FGF-23 polypeptide from a cell's endogenous FGF-23 gene involves increasing, or causing, the expression of a gene or genes (*e.g.*, transcription factors) and/or decreasing the expression of a gene or genes (*e.g.*, transcriptional repressors) in a manner which results in *de novo* or increased FGF-23 polypeptide production from the cell's endogenous FGF-23 gene. This method includes the introduction of a non-naturally occurring polypeptide (*e.g.*, a polypeptide comprising a site specific DNA binding domain fused to a transcriptional factor domain) into the cell such that *de novo* or increased FGF-23 polypeptide production from the cell's endogenous FGF-23 gene results.

The present invention further relates to DNA constructs useful in the method of altering expression of a target gene. In certain embodiments, the exemplary DNA constructs comprise: (a) one or more targeting sequences, (b) a regulatory sequence, (c) an exon, and (d) an unpaired splice-donor site. The targeting sequence in the DNA construct directs the integration of elements (a) - (d) into a target gene in a cell such that the elements (b) - (d) are operatively linked to sequences of the endogenous target gene. In another embodiment, the DNA constructs comprise: (a) one or more targeting sequences, (b) a regulatory sequence, (c) an exon, (d) a splice-donor site, (e) an intron, and (f) a splice-acceptor site, wherein the targeting sequence directs the integration of elements (a) - (f) such that the elements of (b) - (f) are operatively linked to the endogenous gene. The targeting sequence is homologous to the preselected site in the cellular chromosomal DNA with which homologous recombination is to occur. In the construct, the exon is generally 3' of the regulatory sequence and the splice-donor site is 3' of the exon.

If the sequence of a particular gene is known, such as the nucleic acid sequence of FGF-23 polypeptide presented herein, a piece of DNA that is complementary to a selected region of the gene can be synthesized or otherwise obtained, such as by appropriate restriction of the native DNA at specific recognition sites bounding the region of interest. This piece serves as a targeting sequence upon insertion into the cell and will hybridize to its homologous region within the genome. If this hybridization occurs during DNA replication, this piece of DNA, and any additional sequence attached thereto, will act as an Okazaki fragment and will be incorporated into the newly synthesized daughter strand of DNA. The present invention, therefore, includes nucleotides encoding an FGF-23 polypeptide, which nucleotides may be used as targeting sequences.

FGF-23 polypeptide cell therapy, *e.g.*, the implantation of cells producing FGF-23 polypeptides, is also contemplated. This embodiment involves implanting cells capable of synthesizing and secreting a biologically active form of FGF-23 polypeptide. Such FGF-23 polypeptide-producing cells can be cells that are natural producers of FGF-23 polypeptides or may be recombinant cells whose ability to produce FGF-23 polypeptides has been augmented by transformation with a gene encoding the desired FGF-23 polypeptide or with a gene augmenting the expression of FGF-23 polypeptide. Such a modification may be accomplished by means of a vector suitable for delivering the gene as well as promoting its expression and secretion. In order to minimize a potential immunological reaction in patients being administered an FGF-23 polypeptide, as may occur with the administration of a polypeptide of a foreign species, it is preferred that the natural cells producing FGF-23 polypeptide be of human origin and produce human FGF-23 polypeptide. Likewise, it is preferred that the recombinant cells producing FGF-23 polypeptide be transformed with an expression vector containing a gene encoding a human FGF-23 polypeptide.

Implanted cells may be encapsulated to avoid the infiltration of surrounding tissue. Human or non-human animal cells may be implanted in patients in biocompatible, semipermeable polymeric enclosures or membranes that allow the release of FGF-23 polypeptide, but that prevent the destruction of the cells by the patient's immune system or by other detrimental factors from the surrounding tissue. Alternatively, the patient's own cells, transformed to produce FGF-23 polypeptides *ex vivo*, may be implanted directly into the patient without such encapsulation.

Techniques for the encapsulation of living cells are known in the art, and the preparation of the encapsulated cells and their implantation in patients may be routinely accomplished. For example, Baetge *et al.* (PCT Pub. No. WO 95/05452 and PCT/US94/09299) describe membrane capsules containing genetically engineered cells for the effective delivery of biologically active molecules. The capsules are biocompatible and are easily retrievable. The capsules encapsulate cells transfected with recombinant DNA molecules comprising DNA sequences coding for biologically active molecules operatively linked to promoters that are not subject to down-regulation *in vivo* upon implantation into a mammalian host. The devices provide for the delivery of the molecules from living cells to specific sites within a recipient. In addition, *see* U.S. Patent Nos. 4,892,538; 5,011,472; and 5,106,627. A system for encapsulating living cells is described in PCT Pub. No. WO 91/10425 (Aebischer *et al.*). *See also,* PCT Pub. No. WO 91/10470 (Aebischer *et al.*); Winn et al., 1991, Exper. Neurol. 113:322-29; Aebischer et al., 1991, Exper. Neurol. 111:269-75; and Tresco et al., 1992, ASAIO 38:17-23.

*In vivo* and *in vitro* gene therapy delivery of FGF-23 polypeptides is also envisioned. One example of a gene therapy technique is to use the FGF-23 gene (either genomic DNA, cDNA, and/or synthetic DNA) encoding an FGF-23 polypeptide which may be operably linked to a constitutive or inducible promoter to form a "gene therapy DNA construct." The promoter may be homologous or heterologous to the endogenous FGF-23 gene, provided that it is active in the cell or tissue type into which the construct will be inserted. Other components of the gene therapy DNA construct may optionally include DNA molecules designed for site-specific integration (*e.g.*, endogenous sequences useful for homologous recombination), tissue-specific promoters, enhancers or silencers, DNA molecules capable of providing a selective advantage over the parent cell, DNA molecules useful as labels to identify transformed cells, negative selection systems, cell specific binding agents (as, for example, for cell targeting), cell-specific internalization factors, transcription factors enhancing expression from a vector, and factors enabling vector production.

A gene therapy DNA construct can then be introduced into cells (either *ex vivo* or *in vivo*) using viral or non-viral vectors. One means for introducing the gene therapy DNA construct is by means of viral vectors as described herein. Certain vectors, such as retroviral vectors, will deliver the DNA construct to the chromosomal DNA of the cells, and the gene can integrate into the chromosomal DNA. Other vectors will function as episomes, and the gene therapy DNA construct will remain in the cytoplasm.

In yet other embodiments, regulatory elements can be included for the controlled expression of the FGF-23 gene in the target cell. Such elements are turned on in response to an appropriate effector. In this way, a therapeutic polypeptide can be expressed when desired. One conventional control means involves the use of small molecule dimerizers or rapalogs to dimerize chimeric proteins which contain a small molecule-binding domain and a domain capable of initiating a biological process, such as a DNA-binding protein or transcriptional activation protein (see PCT Pub. Nos. WO 96/41865, WO 97/31898, and WO 97131899). The dimerization of the proteins can be used to initiate transcription of the transgene.

An alternative regulation technology uses a method of storing proteins expressed from the gene of interest inside the cell as an aggregate or cluster. The gene of interest is expressed as a fusion protein that includes a conditional aggregation domain that results in the retention of the aggregated protein in the endoplasmic reticulum. The stored proteins are stable and inactive inside the cell. The proteins can be released, however, by administering a drug (e.g., small molecule ligand) that removes the conditional aggregation domain and thereby specifically breaks apart the aggregates or clusters so that the proteins may be secreted from the cell. See Aridor et al., 2000, Science 287:816-17 and Rivera et al., 2000, Science 287:826-30.

Other suitable control means or gene switches include, but are not limited to, the systems described herein. Mifepristone (RU486) is used as a progesterone antagonist. The binding of a modified progesterone receptor ligand-binding domain to the progesterone antagonist activates transcription by forming a dimer of two transcription factors that then pass into the nucleus to bind DNA. The ligand-binding domain is modified to eliminate the ability of the receptor to bind to the natural ligand. The modified steroid hormone receptor system is further described in U.S. Patent No. 5,364,791 and PCT Pub. Nos. WO 96/40911 and WO 97/10337.

Yet another control system uses ecdysone (a fruit fly steroid hormone) which binds to and activates an ecdysone receptor (cytoplasmic receptor). The receptor then translocates to the nucleus to bind a specific DNA response element (promoter from ecdysone-responsive gene). The ecdysone receptor includes a transactivation domain, DNA-binding domain, and ligand-binding domain to initiate transcription. The ecdysone system is further described in U.S. Patent No. 5,514,578 and PCT Pub. Nos. WO 97/38117, WO 96/37609, and WO 93/03162.

Another control means uses a positive tetracycline-controllable transactivator. This system involves a mutated tet repressor protein DNA-binding domain (mutated tet R-4 amino acid changes which resulted in a reverse tetracycline-regulated transactivator protein, i.e., it binds to a tet operator in the presence of tetracycline) linked to a polypeptide which activates transcription. Such systems are described in U.S. Patent Nos. 5,464,758, 5,650,298, and 5,654,168.

Additional expression control systems and nucleic acid constructs are described in U.S. Patent Nos. 5,741,679 and 5,834,186, to Innovir Laboratories Inc.

In vivo gene therapy may be accomplished by introducing the gene encoding FGF-23 polypeptide into cells via local injection of an FGF-23 nucleic acid molecule or by other appropriate viral or non-viral delivery vectors. Hefti, 1994, Neurobiology 25:1418-35. For example, a nucleic acid molecule encoding an FGF-23 polypeptide may be contained in an adeno-associated virus (AAV) vector for delivery to the targeted cells (see, e.g., Johnson, PCT Pub. No. WO 95/34670; PCT App. No. PCT/US95/07178). The recombinant AAV genome typically contains AAV inverted terminal repeats flanking a DNA sequence encoding an FGF-23 polypeptide operably linked to functional promoter and polyadenylation sequences.

Alternative suitable viral vectors include, but are not limited to, retrovirus, adenovirus, herpes simplex virus, lentivirus, hepatitis virus, parvovirus, papovavirus, poxvirus, alphavirus, coronavirus, rhabdovirus, paramyxovirus, and papilloma virus vectors. U.S. Patent No. 5,672,344 describes an in vivo viral-mediated gene transfer system involving a recombinant neurotrophic HSV-1 vector. U.S. Patent No. 5,399,346 provides examples of a process for providing a patient with a therapeutic protein by the delivery of human cells which have been treated in vitro to insert a DNA segment encoding a therapeutic protein. Additional methods and materials for the practice of gene therapy techniques are described in U.S. Patent Nos. 5,631,236 (involving adenoviral vectors), 5,672,510 (involving retroviral vectors), 5,635,399 (involving retroviral vectors expressing cytokines).

Nonviral delivery methods include, but are not limited to, liposome-mediated transfer, naked DNA delivery (direct injection), receptor-mediated transfer (ligand-DNA complex), electroporation, calcium phosphate precipitation, and microparticle bombardment (e.g., gene gun). Gene therapy materials and methods may also include inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, and transcription factors to enhance expression by a vector as well as methods of vector manufacture. Such additional methods and materials for the practice of gene therapy techniques are described in U.S. Patent Nos. 4,970,154 (involving electroporation techniques), 5,679,559 (describing a lipoprotein-containing system for gene delivery), 5,676,954 (involving liposome carriers), 5,593,875 (describing methods for calcium phosphate transfection), and 4,945,050 (describing a process wherein biologically active particles are propelled at cells at a speed whereby the particles penetrate the surface of the cells and become incorporated into the interior of the cells), and PCT Pub. No. WO 96/40958 (involving nuclear ligands).

It is also contemplated that FGF-23 gene therapy or cell therapy can further include the delivery of one or more additional polypeptide(s) in the same or a different cell(s). Such cells may be separately introduced into the patient, or the cells may be contained in a single implantable device, such as the encapsulating membrane described above, or the cells may be separately modified by means of viral vectors.

A means to increase endogenous FGF-23 polypeptide expression in a cell via gene therapy is to insert one or more enhancer elements into the FGF-23 polypeptide promoter, where the enhancer elements can serve to increase transcriptional activity of the FGF-23 gene. The enhancer elements used will be selected based on the tissue in which one desires to activate the gene - enhancer elements known to confer promoter activation in that tissue will be selected. For example, if a gene encoding an FGF-23 polypeptide is to be "turned on" in T-cells, the *lck* promoter enhancer element may be used. Here, the functional portion of the transcriptional element to be added may be inserted into a fragment of DNA containing the FGF-23 polypeptide promoter (and optionally, inserted into a vector and/or 5' and/or 3' flanking sequences) using standard cloning techniques. This construct, known as a "homologous recombination construct," can then be introduced into the desired cells either *ex vivo* or *in vivo.*

Gene therapy also can be used to decrease FGF-23 polypeptide expression by modifying the nucleotide sequence of the endogenous promoter. Such modification is typically accomplished via homologous recombination methods. For example, a DNA molecule containing all or a portion of the promoter of the FGF-23 gene selected for inactivation can be engineered to remove and/or replace pieces of the promoter that regulate transcription. For example, the TATA box and/or the binding site of a transcriptional activator of the promoter may be deleted using standard molecular biology techniques; such deletion can inhibit promoter activity thereby repressing the transcription of the corresponding FGF-23 gene. The deletion of the TATA box or the transcription activator binding site in the promoter may be accomplished by generating a DNA construct comprising all or the relevant portion of the FGF-23 polypeptide promoter (from the same or a related species as the FGF-23 gene to be regulated) in which one or more of the TATA box and/or transcriptional activator binding site nucleotides are mutated via substitution, deletion and/or insertion of one or more nucleotides. As a result, the TATA box and/or activator binding site has decreased activity or is rendered completely inactive. This construct, which also will typically contain at least about 500 bases of DNA that correspond to the native (endogenous) 5' and 3' DNA sequences adjacent to the promoter segment that has been modified, may be introduced into the appropriate cells (either *ex vivo* or *in vivo*) either directly or via a viral vector as described herein. Typically, the integration of the construct into the genomic DNA of the cells will be via homologous recombination, where the 5' and 3' DNA sequences in the promoter construct can serve to help integrate the modified promoter region via hybridization to the endogenous chromosomal DNA.

## Therapeutic Uses

FGF-23 nucleic acid molecules, polypeptides, and agonists and antagonists thereof can be used to treat, diagnose, ameliorate, or prevent a number of diseases, disorders, or conditions, including those recited herein.

FGF-23 polypeptide agonists and antagonists include those molecules which regulate FGF-23 polypeptide activity and either increase or decrease at least one activity of the mature form of the FGF-23 polypeptide. Agonists or antagonists may be co-factors, such as a protein, peptide, carbohydrate, lipid, or small molecular weight molecule, which interact with FGF-23 polypeptide and thereby regulate its activity. Potential polypeptide agonists or antagonists include antibodies that react with either soluble or membrane-bound forms of FGF-23 polypeptides that comprise part or all of the extracellular domains of the said proteins. Molecules that regulate FGF-23 polypeptide expression typically include nucleic acids encoding FGF-23 polypeptide that can act as anti-sense regulators of expression.

The FGF-23 nucleic acid molecules, polypeptides, and agonists and antagonists thereof of the present invention are useful for the same purposes for which members of the FGF family of polypeptides are known to be useful. Thus, the FGF-23 polypeptides of this invention are potent mitogens for a variety of cells of the mesodermal, ectodermal, and endodermal origin, including fibroblasts, corneal and vascular endothelial cells, granulocytes, adrenal cortical cells, chondrocytes, myoblasts, vascular smooth muscle cells, lens epithelial cells, melanocytes, keratinocytes, oligodendrocytes, astrocytes, osteoblasts, and hematopoietic cells. Included among these biological activities are the ability to stimulate the proliferation of vascular endothelial cells and to enable endothelial cells to penetrate the basement membrane. Consistent with these properties, the FGF-23 polypeptides of this invention may stimulate angiogenesis and promote wound healing (*i.e.*, facilitate the repair or replacement of damages of diseased tissue resulting from bums, traumatic injuries, surgery, or ulcers). These polypeptides may also induce mesoderm formation and modulate the differentiation of neuronal cells, adipocytes, and skeletal muscle cells. The polypeptides may also be employed to prevent or ameliorate skin aging due to sun exposure by stimulating keratinocyte growth. Further, the polypeptides of this invention may be employed to maintain organs before transplantation or for supporting cultures of primary cells and tissues. In addition, these polypeptides may be utilized to prevent hair loss since FGF family members activate hair-forming cells and promote melanocyte growth. They may also be used to stimulate the growth and differentiation of hematopoietic cells and bone marrow cells when used in combination with other cytokines.

FGF-23 has been linked with a human autosomal dominant genetic disease, hypophosphatemic rickets (ADHR) (The ADHR Consortium, 2000, Nature Genetics 26:345-48). Accordingly, the FGF-23 nucleic acid molecules, polypeptides, and agonists and antagonists thereof of the present invention may be used to treat, diagnose, ameliorate, or prevent ADHR.

The FGF-23 gene has been shown to be most closely related to human FGF-21 (Yamashita et al., 2000, Biochem. Biophys. Res. Commun. 277:494-98), a gene which is expressed most abundantly in the liver and at lower levels in the thymus (Nishimura et al., 2000, Biochim. Biophys. Acta 1492:203-06). Accordingly, FGF-23 nucleic acid molecules, polypeptides, and agonists and antagonists thereof may be used to treat, diagnose, ameliorate, or prevent diseases, disorders, or conditions involving the liver or thymus.

A non-exclusive list of other diseases, disorders, or conditions which may be treated, diagnosed, ameliorated, or prevented with the FGF-23 nucleic acid molecules, polypeptides, and agonists and antagonists thereof of the present invention include: dermal wounds, epidermalysis bullosa, male pattern alopecia, gastric ulcer, duodenal ulcer, erosive gastritis, esophagitis, esophageal reflux disease, inflammatory bowel disease, radiation- or chemotherapy-induced gut toxicity, hyaline membrane disease, necrosis of the respiratory epithelium, emphysema, pulmonary inflammation, pulmonary fibrosis, hepatic cirrhosis, fulminant liver failure, viral hepatitis, and diabetes.

Agonists or antagonists of FGF-23 polypeptide function may be used (simultaneously or sequentially) in combination with one or more cytokines, growth factors, antibiotics, anti-inflammatories, and/or chemotherapeutic agents as is appropriate for the condition being treated.

Other diseases caused by or mediated by undesirable levels of FGF-23 polypeptides are encompassed within the scope of the invention. Undesirable levels include excessive levels of FGF-23 polypeptides and sub-normal levels of FGF-23 polypeptides.

Uses of FGF-23 Nucleic Acids and Polypeptides

Nucleic acid molecules of the invention (including those that do not themselves encode biologically active polypeptides) may be used to map the locations of the FGF-23 gene and related genes on chromosomes. Mapping may be done by techniques known in the art, such as PCR amplification and *in situ* hybridization.

FGF-23 nucleic acid molecules (including those that do not themselves encode biologically active polypeptides), may be useful as hybridization probes in diagnostic assays to test, either qualitatively or quantitatively, for the presence of an FGF-23 nucleic acid molecule in mammalian tissue or bodily fluid samples.

Other methods may also be employed where it is desirable to inhibit the activity of one or more FGF-23 polypeptides. Such inhibition may be effected by nucleic acid molecules that are complementary to and hybridize to expression control sequences (triple helix formation) or to FGF-23 mRNA. For example, antisense DNA or RNA molecules, which have a sequence that is complementary to at least a portion of an FGF-23 gene can be introduced into the cell. Anti-sense probes may be designed by available techniques using the sequence of the FGF-23 gene disclosed herein. Typically, each such antisense molecule will be complementary to the start site (5' end) of each selected FGF-23 gene. When the antisense molecule then hybridizes to the corresponding FGF-23 mRNA, translation of this mRNA is prevented or reduced. Anti-sense inhibitors provide information relating to the decrease or absence of an FGF-23 polypeptide in a cell or organism.

Alternatively, gene therapy may be employed to create a dominant-negative inhibitor of one or more FGF-23 polypeptides. In this situation, the DNA encoding a mutant polypeptide of each selected FGF-23 polypeptide can be prepared and introduced into the cells of a patient using either viral or non-viral methods as described herein. Each such mutant is typically designed to compete with endogenous polypeptide in its biological role.

In addition, an FGF-23 polypeptide, whether biologically active or not, may be used as an immunogen, that is, the polypeptide contains at least one epitope to which antibodies may be raised. Selective binding agents that bind to an FGF-23 polypeptide (as described herein) may be used for *in vivo* and *in vitro* diagnostic purposes, including, but not limited to, use in labeled form to detect the presence of FGF-23 polypeptide in a body fluid or cell sample. The antibodies may also be used to prevent, treat, or diagnose a number of diseases and disorders, including those recited herein. The antibodies may bind to an FGF-23 polypeptide so as to diminish or block at least one activity characteristic of an FGF-23 polypeptide, or may bind to a polypeptide to increase at least one activity characteristic of an FGF-23 polypeptide (including by increasing the pharmacokinetics of the FGF-23 polypeptide).

The FGF-23 polypeptides of the present invention can be used to clone FGF-23 polypeptide receptors, using an expression cloning strategy. Radiolabeled ([125]Iodine) FGF-23 polypeptide or affinity/activity-tagged FGF-23 polypeptide (such as an Fc fusion or an alkaline phosphatase fusion) can be used in binding assays to identify a cell type or cell line or tissue that expresses FGF-23 polypeptide receptors. RNA isolated from such cells or tissues can be converted to cDNA, cloned into a mammalian expression vector, and transfected into mammalian cells (such as COS or 293 cells) to create an expression library. A radiolabeled or tagged FGF-23 polypeptide can then be used as an affinity ligand to identify and isolate from this library the subset of cells that express the FGF-23 polypeptide receptors on their surface.

DNA can then be isolated from these cells and transfected into mammalian cells to create a secondary expression library in which the fraction of cells expressing FGF-23 polypeptide receptors is many-fold higher than in the original library. This enrichment process can be repeated iteratively until a single recombinant clone containing an FGF-23 polypeptide receptor is isolated. Isolation of the FGF-23 polypeptide receptors is useful for identifying or developing novel agonists and antagonists of the FGF-23 polypeptide signaling pathway. Such agonists and antagonists include soluble FGF-23 polypeptide receptors, anti-FGF-23 polypeptide receptor antibodies, small molecules, or antisense oligonucleotides, and they may be used for treating, preventing, or diagnosing one or more of the diseases or disorders described herein.

According (Acc.) to a 1st aspect it is provided

an isolated nucleic acid molecule comprising a nucleotide sequence selected from the group consisting of:

(a) the nucleotide sequence as set forth in SEQ ID NO: 1;
(b) the nucleotide sequence of the DNA insert in ATCC Deposit No. PTA-1617;
(c) a nucleotide sequence encoding the polypeptide as set forth in SEQ ID NO: 2;
(d) a nucleotide sequence which hybridizes under moderately or highly stringent conditions to the complement of any of (a) - (c); and
(e) a nucleotide sequence complementary to any of (a) - (c).

Acc. to a 2nd aspect it is provided

an isolated nucleic acid molecule comprising a nucleotide sequence selected from the group consisting of:

(a) a nucleotide sequence encoding a polypeptide which is at least about 70 percent identical to the polypeptide as set forth in SEQ ID NO: 2, wherein the encoded polypeptide has an activity of the polypeptide set forth in SEQ ID NO: 2;
(b) a nucleotide sequence encoding an allelic variant or splice variant of the nucleotide sequence as set forth in SEQ ID NO: 1, the nucleotide sequence of the DNA insert in ATCC Deposit No. PTA-1617, or (a);
(c) a region of the nucleotide sequence of SEQ ID NO: 1, the DNA insert in ATCC Deposit No. PTA-1617, (a), or (b) encoding a polypeptide fragment of at least about 25 amino acid residues, wherein the polypeptide fragment has an activity of the encoded polypeptide as set forth in SEQ ID NO: 2, or is antigenic;
(d) a region of the nucleotide sequence of SEQ ID NO: 1, the DNA insert in ATCC Deposit No. PTA-1617, or any of (a) - (c) comprising a fragment of at least about 16 nucleotides;
(e) a nucleotide sequence which hybridizes under moderately or highly stringent condition to the complement of any of (a) - (d); and
(f) a nucleotide sequence complementary to any of (a) - (d).

Acc. to a 3rd aspect it is provided

an isolated nucleic acid molecule comprising a nucleotide sequence selected from the group consisting of:

(a) a nucleotide sequence encoding a polypeptide as set forth in SEQ ID NO: 2 with at least one conservative amino acid substitution, wherein the encoded polypeptide has an activity of the polypeptide set forth in SEQ ID NO: 2;
(b) a nucleotide sequence encoding a polypeptide as set forth in SEQ ID NO: 2 with at least one amino acid insertion, wherein the encoded polypeptide has an activity of the polypeptide set forth in SEQ ID NO: 2;
(c) a nucleotide sequence encoding a polypeptide as set forth in SEQ ID NO: 2 with at least one amino acid deletion, wherein the encoded polypeptide has an activity of the polypeptide set forth in SEQ ID NO: 2;
(d) a nucleotide sequence encoding a polypeptide as set forth in SEQ ID NO: 2 which has a C- and/or N- terminal truncation, wherein the encoded polypeptide has an activity of the polypeptide set forth in SEQ ID NO: 2;
(e) a nucleotide sequence encoding a polypeptide as set forth in SEQ ID NO: 2 with at least one modification selected from the group consisting of amino acid substitutions, amino acid insertions, amino acid deletions, C-terminal truncation, and N-terminal truncation, wherein the encoded polypeptide has an activity of the polypeptide set forth in SEQ ID NO: 2;
(f) a nucleotide sequence of any of (a) - (e) comprising a fragment of at least about 16 nucleotides;
(g) a nucleotide sequence which hybridizes under moderately or highly stringent conditions to the complement of any of (a) - (f); and
(h) a nucleotide sequence complementary to any of (a) - (e).

Acc. to a 4th aspect it is provided

a vector comprising the nucleic acid molecule of any of the inventions.

Acc. to a 5th aspect it is provided

a host cell comprising the vector of the invention.

In a preferred embodiment it is provided

a host cell of the invention that is a eukaryotic cell.

In a preferred embodiment it is provided
a host cell of the invention that is a prokaryotic cell.

Acc. to a 6th aspect it is provided
a process of producing an FGF-23 polypeptide comprising culturing the host cell of the invention under suitable conditions to express the polypeptide, and optionally isolating the polypeptide from the culture.

Acc. to a 7th aspect it is provided
a polypeptide produced by the process of the invention.

In a preferred embodiment it is provided
a process of the invention wherein the nucleic acid molecule comprises promoter DNA other than the promoter DNA for the native FGF-23 polypeptide operatively linked to the DNA encoding the FGF-23 polypeptide.

Acc. to an 8th aspect it is provided
an isolated nucleic acid molecule according to the invention, wherein the percent identity is determined using a computer program selected from the group consisting of GAP, BLASTN, FASTA, BLASTA, BLASTX, BestFit, and the Smith-Waterman algorithm.

Acc. to a 9th aspect it is provided
a process for determining whether a compound inhibits FGF-23 polypeptide activity or FGF-23 polypeptide production comprising exposing a cell according to the invention to the compound and measuring FGF-23 polypeptide activity or FGF-23 polypeptide production in said cell.

Acc. to a 10th aspect it is provided
an isolated polypeptide comprising the amino acid sequence selected from the group consisting of:

(a) the amino acid sequence as set forth in SEQ ID NO: 2; and
(b) the amino acid sequence encoded by the DNA insert in ATCC Deposit No. PTA-1617.

Acc. to an 11th aspect it is provided
an isolated polypeptide comprising the amino acid sequence selected from the group consisting of:

(a) the amino acid sequence as set forth in SEQ ID NO: 3, optionally further comprising an ammo-terminal methionine;
(b) an amino acid sequence for an ortholog of SEQ ID NO: 2;
(c) an amino acid sequence which is at least about 70 percent identical to the amino acid sequence of SEQ ID NO: 2, wherein the polypeptide has an activity of the polypeptide set forth in SEQ ED NO: 2;
(d) a fragment of the amino acid sequence set forth in SEQ ID NO: 2 comprising at least about 25 amino acid residues, wherein the fragment has an activity of the polypeptide set forth in SEQ ID NO: 2, or is antigenic; and
(e) an amino acid sequence for an allelic variant or splice variant of the amino acid sequence as set forth in SEQ ID NO: 2, the amino acid sequence encoded by the DNA insert in ATCC Deposit No. PTA-1617, or (a) - (c).

Acc. to a 12th aspect it is provided
an isolated polypeptide comprising the amino acid sequence selected from the group consisting of:

(a) the amino acid sequence as set forth in SEQ ID NO: 2 with at least one conservative amino acid substitution, wherein the polypeptide has an activity of the polypeptide set forth in SEQ ID NO: 2;
(b) the amino acid sequence as set forth in SEQ ID NO: 2 with at least one amino acid insertion, wherein the polypeptide has an activity of the polypeptide set forth in SEQ ID NO: 2;
(c) the amino acid sequence as set forth in SEQ ID NO: 2 with at least one amino acid deletion, wherein the polypeptide has an activity of the polypeptide set forth in SEQ ID NO: 2;
(d) the amino acid sequence as set forth in SEQ ID NO; 2 which has a C- and/or N- terminal truncation, wherein the polypeptide has an activity of the polypeptide set forth in SEQ ID NO: 2; and
(e) the amino acid sequence as set forth in SEQ ID NO: 2 with at least one modification selected from the group consisting of amino acid substitutions, amino acid insertions, amino acid deletions, C-terminal truncation, and N-terminal truncation, wherein the polypeptide has an activity of the polypeptide set forth in SEQ ID NO: 2.

It is provided
an isolated polypeptide encoded by the nucleic acid molecule of the invention wherein the polypeptide has an activity of the polypeptide set forth in SEQ ID NO: 2.

Acc. to a 14th aspect it is provided
an isolated polypeptide according to the invention wherein the percent identity is determined using a computer program selected from the group consisting of GAP, BLASTP, FASTA, BLASTA, BLASTX, BestFit, and the Smith-Waterman algorithm.

Acc. to a 15th aspect it is provided

A selective binding agent or fragment thereof which specifically binds the polypeptide of any of the invention.

Acc. to a 16th aspect it is provided

a selective binding agent or fragment thereof of the invention that specifically binds the polypeptide comprising the amino acid sequence as set forth in SEQ ID NO: 2, or a fragment thereof.

In a preferred embodiment it is provided

a selective binding agent of the invention that is an antibody or fragment thereof.

In a preferred embodiment if is provided

a selective binding agent of the invention that is a humanized antibody.

In a preferred embodiment it is provided

a selective binding agent of the invention that is a human antibody or fragment thereof.

In a preferred embodiment it is provided

a selective binding agent of the invention that is a polyclonal antibody or fragment thereof.

In a preferred embodiment it is provided

a selective binding agent of the invention that is a monoclonal antibody or fragment thereof.

In a preferred embodiment it is provided

a selective binding agent of the invention that is a chimeric antibody or fragment thereof.

In a preferred embodiment it is provided

a selective binding agent of the invention that is a CDR-grafted antibody or fragment thereof.

In a preferred embodiment it is provided

a selective binding agent of the invention that is an antiidiotypic antibody or fragment thereof.

In a preferred embodiment it is provided

a selective binding agent of the invention that is a variable region fragment.

Acc. to a 17th aspect it is provided

a variable region fragment of the invention that is a Fab or a Fab' fragment.

Acc. to an 18th aspect it is provided

a selective binding agent or fragment thereof comprising at least one complementarity determining region with specificity for a polypeptide having the amino acid sequence of SEQ ID NO: 2.

In a preferred embodiment it is provided

a selective binding agent of the invention that is bound to a detectable label.

In a preferred embodiment it is provided

a selective binding agent of the invention that antagonizes FGF-23 polypeptide biological activity.

Acc. to a 19th aspect it is provided

a method for treating, preventing, or ameliorating an FGF-23 polypeptide-related disease, condition, or disorder comprising administering to a patient an effective amount of a selective binding agent according to the invention.

Acc. to a 20th aspect it is provided

a selective binding agent produced by immunizing an animal with a polypeptide comprising an amino acid sequence of SEQ ED NO: 2.

Acc. to a 21st aspect it is provided

a hybridoma which produces a selective binding agent which is capable of binding a polypeptide according to the invention.

Acc. to a 22nd aspect it is provided

a method of detecting or quantitating the amount of FGF-23 polypeptide using the anti-FGF-23 antibody or fragment of the invention.

Acc. to a 23rd aspect it is provided

a composition comprising the polypeptide of the invention and a pharmaceutically acceptable formulation agent.

In a preferred embodiment it is provided

a composition of the invention, wherein the pharmaceutically acceptable formulation agent is a carrier, adjuvant, solubilizer, stabilizer, or antioxidant.

In a preferred embodiment it is provided

a composition of the invention wherein the polypeptide comprises the amino acid sequence as set forth in SEQ ID NO: 3.

Acc. to a 24th aspect it is provided

a polypeptide comprising a derivative of the polypeptide of the invention.

In a preferred embodiment it is provided

a polypeptide of Claim 40 that is covalently modified with a water-soluble polymer.

In a preferred embodiment it is provided

a polypeptide of the invention wherein the water-soluble polymer is selected from the group consisting of polyethylene glycol, monomethoxy-polyethylene glycol, dextran, cellulose, poly-(N-vinyl pyrrolidone) polyethylene glycol, propylene glycol homopolymers, polypropylene oxide/ethylene oxide copolymers, polyoxyethylated polyols, and polyvinyl alcohol.

Acc. to a 25th aspect it is provided
a composition comprising a nucleic acid molecule of any of the invention and a pharmaceutically acceptable formulation agent.

In a preferred embodiment it is provided
a composition of the invention, wherein said nucleic acid molecule is contained in a viral vector.

Acc. to a 26th aspect it is provided
a viral vector comprising a nucleic acid molecule to the invention.

Acc. to a 27th aspect it is provided
a fusion polypeptide comprising the polypeptide of the invention fused to a heterologous amino acid sequence.

In a preferred embodiment it is provided
a fusion polypeptide of the invention, wherein the heterologous amino acid sequence is an IgG constant domain or fragment thereof.

Acc. to a 28th aspect it is provided
a method for treating, preventing, or ameliorating a medical condition comprising administering to a patient the polypeptide of the invention or the polypeptide encoded by the nucleic acid of the invention.

Acc. to a 29th aspect it is provided
a method for treating, preventing or ameliorating a medical condition comprising administering to a patient an agonist or antagonist of the biological activity of the polypeptide of the invention or of the polypeptide encoded by the nucleic acid of the invention.

In a preferred embodiment it is provided
a method of the invention wherein the medical condition being treated, prevented, or ameliorated is autosomal dominant hypophosphatemic rickets (ADHR).

Acc. to a 30th aspect it is provided
a method of diagnosing a pathological condition or a susceptibility to a pathological condition in a subject comprising;

(a) determining the presence or amount of expression of the polypeptide of the invention or the polypeptide of polypeptide encoded by the nucleic acid molecule of the invention in a sample; and

(b) diagnosing a pathological condition or a susceptibility to a pathological condition based on the presence or amount of expression of the polypeptide.

Acc. to a 31st aspect it is provided
a device, comprising:

(a) a membrane suitable for implantation; and

(b) cells encapsulated within said membrane, wherein said cells secrete a protein of any of the invention; and said membrane is permeable to said protein and impermeable to materials detrimental to said cells.

Acc. to a 32nd aspect it is provided
a method of identifying a compound which binds to an FGF-23 polypeptide comprising

(a) contacting the polypeptide of any of the invention, with a compound; and

(b) determining the extent of binding of the FGF-23 polypeptide to the compound.

In a preferred embodiment it is provided
a method of the invention further comprising determining the activity of the polypeptide when bound to the compound.

Acc. to a 33rd aspect it is provided
a method of modulating levels of a polypeptide in an animal comprising administering to the animal the nucleic acid molecule of any of Claims 1, 2, or 3.

Acc. to a 34th aspect it is provided
a transgenic non-human mammal comprising the nucleic acid molecule of any of Claims 1, 2, or 3.

Acc. to a 35th aspect it is provided
a process for determining whether a compound inhibits FGF-23 polypeptide activity or FGF-23 polypeptide production comprising exposing a transgenic mammal according to Claim 56 to the compound, and measuring FGF-23 polypeptide activity or FGF-23 polypeptide production in said mammal.

A deposit of cDNA encoding human FGF-23 polypeptide, subcloned into the pGEM-t vector, and having Accession No. PTA-1617, was made with the American Type Culture Collection, 10801 University Boulevard, Manassas, VA 20110-2209 on March 31, 2000.

The following examples are intended for illustration purposes only, and should not be construed as limiting the scope of the invention in any way.

Example 1: Cloning of the Human FGF-23 Polypeotide Gene

To isolate cDNA sequences encoding human FGF-23 polypeptide,homology-based BLAST searches of a human genomic database were performed.

A putative coding sequence sharing homology with the Fibroblast Growth Factor (FGF) family was identified in a human genomic clone (GenBank accession no. AC008012). The putative coding sequence consisted of three potential exons separated by introns of 6.6 kb and 1.87 kb. This sequence was used to design gene specific oligonucleotides for the identification of cDNA sources and the generation of cDNA clones, using various PCR strategies.

**[0324]** [0323] A number of cDNA libraries were analyzed in amplification reactions containing 10 pmol each of the amplimers (5'-C-T-A-T-C-C-C-A-A-T-G-C-C-T-C-C-C-C-A-C-T-G-3'; SEQ ID NO: 42, and 5'-C-G-C-C-C-C-T-G-A-C-C-A-C-C-C-C-T-A-A-T-G-3'; SEQ ID NO: 43) and Ready-To-Go PCR beads (Pharmacia, Piscataway, NJ), in a total reaction volume of 250 μl. Reactions were performed at 95°C for 5 minutes for one cycle; 95°C for 30 seconds, 68°C for 15 seconds, and 72°C for 1 minute for 35 cycles; and 72°C for 7 minutes for one cycle. A PCR product of the expected size (616 bp) was identified in a number of cDNA libraries, including libraries derived from colon tumor T25 (random primed), fetal mesentery (oligo-dT primed), fetal gall bladder (random primed), and fetal heart (oligo-dT primed). The PCR product generated from the fetal mesentery cDNA library was subcloned using a TopoTA 4.0 cloning kit (Invitrogen) and four clones were sequenced to verify that the clones contained the predicted FGF-23 cDNA sequence. The fetal mesentery cDNA library was selected for further amplification experiments to isolate full-length cDNA sequences encoding FGF-23 polypeptide.

**[0325]** [0324] The fetal mesentery cDNA library was prepared as follows. Total RNA was extracted from human fetal mesentery using standard RNA extraction procedures and poly-A$^+$ RNA was selected from this total RNA using standard procedures. Oligo-dT primed cDNA was synthesized from this poly-A$^+$ RNA using the Superscript Plasmid System for cDNA Synthesis and Plasmid Cloning kit (Gibco-BRL), according to the manufacturer's suggested protocols. The resulting cDNA was digested with the restriction endonucleases Sal I and Not I and was then ligated into pSPORT-1. Ligation products were transformed into *E. coli* using standard techniques, and bacterial transformants were selected on culture plates containing ampicillin. The cDNA library consisted of all, or a subset, of these transformants.

**[0326]** [0325] Both 5'RACE and 3'RACE reactions were performed in order to generate the full-length cDNA sequence for FGF-23 polypeptide. To isolate cDNA sequences corresponding to the 5' end of the cDNA sequence for FGF-23 polypeptide, 5'RACE was performed using the Smart RACE cDNA Amplification kit (Clontech), random-primed human fetal mesentery cDNA library in pSPORT1, and the primers 5'-G-T-G-T-G-G-A-A-T-T-G-T-G-A-G-C-G-G-A-T-A-A-C-3' (SEQ ID NO: 44) and 5'-C-T-G-A-T-G-G-G-G-T-G-C-G-C-C-A-T-C-C-A-C-A-3' (SEQ ID NO: 45). Reactions were performed at 94°C for 1 minute for one cycle; 94°C for 5 seconds, 68°C for 10 seconds, and 72°C for 3 minutes for 35 cycles; and 72°C for 7 minutes for one cycle. Nested PCR was performed using a portion of the 5'RACE amplification product (diluted 1/100) and the primers 5'-C-T-A-T-G-A-C-C-A-T-G-A-T-T-A-C-G-C-C-A-A-G-C-3' (SEQ ID NO: 46) and 5'-C-A-T-T-C-T-T-G-T-G-G-A-T-C-T-G-C-A-G-G-T-G-G-T-3' (SEQ ID NO: 47). Nested PCR Reactions were performed at 94°C for 5 minutes for one cycle; 94°C for 15 seconds, 68°C for 15 seconds, and 72°C for 3 minutes for 30 cycles; and 72°C for 7 minutes for one cycle. The amplification products were analyzed by agarose gel electrophoresis and a prominent PCR product of 200 bp was isolated and subcloned using the TopoTA 4.0 cloning kit. Sequencing analysis of isolated clones indicated that the 5' PCR product did not extend the known sequence.

**[0327]** [0326] Further 5'RACE experiments were performed to isolate additional cDNA sequences corresponding to the 5' end of the cDNA sequence for FGF-23 polypeptide. Additional 5'RACE experiments were performed using the Advantage-2 PCR kit (Clontech), a Marathon™ human heart cDNA library (Clontech), and the primers 5'-C-T-G-A-T-G-G-G-G-T-G-C-G-C-C-A-T-C-C-A-C-A-3' (SEQ ID NO: 45) and AP1 (Clontech). Reactions were performed at 94°C for 30 seconds for one cycle and 94°C for 5 seconds and 68°C for 4 minutes for 30 cycles. Nested PCR was performed using a portion of the 5'RACE amplification product (diluted 1/100) and the primers 5'-C-A-T-T-C-T-T-G-T-G-G-A-T-C-T-G-C-A-G-G-T-G-G-T-3' (SEQ ID NO: 47) and AP2 (Clontech). Nested PCR Reactions were performed at 94°C for 30 seconds for one cycle and 94°C for 30 seconds, 68°C for 4 minutes for 30 cycles. The amplification products were analyzed by agarose gel electrophoresis and the most prominent PCR product (350 bp) was isolated and subcloned using the TopoTA 4.0 cloning kit. Sequencing analysis of isolated clones indicated that the 5' PCR product extended the known sequence by approximately 143 bp.

**[0328]** [0327] To isolate cDNA sequences corresponding to the 3' end of the cDNA sequence for FGF-23 polypeptide, 3'RACE was performed using the Smart RACE cDNA Amplification kit (Clontech), random-primed human fetal mesentery cDNA library in pSPORT1, and the primers 5'-C-G-G-C-C-T-C-C-T-G-T-T-C-A-C-A-G-G-A-G-C-T-C-3' (SEQ ID NO: 48) and 5'-C-G-G-G-C-C-T-C-T-T-C-G-C-T-A-T-T-A-C-G-C-3' (SEQ ID NO: 49). Reactions were performed at 94°C for 1 minute for one cycle; 94°C for 5 seconds, 68°C for 10 seconds, and 72°C for 3 minutes for 35 cycles; and 72°C for 7 minutes for one cycle. Nested PCR was performed using a portion of the 5'RACE amplification product (diluted 1/100) and the primers 5'-G-C-G-C-C-G-A-G-G-A-C-A-A-C-A-G-C-C-C-G-A-3' (SEQ ID NO: 50) and 5'-T-G-G-C-G-A-A-A-G-G-G-G-G-A-T-G-T-G-C-T-G-3' (SEQ ID NO: 51). Nested PCR Reactions were performed at 94°C for 5 minutes for one cycle; 94°C for 15 seconds, 68°C for 15 seconds, and 72°C for 3 minutes for 30 cycles; and 72°C for 7 minutes for one cycle. The amplification products were analyzed by agarose gel electrophoresis and a prominent PCR product of 650 bp was isolated and subcloned using the TopoTA 4.0 cloning kit. Sequencing analysis of isolated clones indicated that the 3' PCR product extended the known sequence by 433 bp, including the poly-A region.

**[0329]** [0328] A contiguous sequence that appears to contain the full-length open reading frame for the FGF-23 gene was generated using the sequence derived from the initial PCR amplification and the 5' and 3'RACE amplifications. Sequence analysis of this consensus sequence indicated that the FGF-23 gene comprises a 753 bp open reading frame encoding a protein of 251 amino acids (Figures 1A-1B).

**[0330]** [0329] Sequence analysis also revealed that FGF-23 polypeptide shares homology with the Fibroblast Growth Factor (FGF) family. Figures 2A-2G illustrate the amino acid sequence alignment of human FGF-1 (hu FGF-1; SEQ ID NO: 4), human FGF-2 (hu FGF-2; SEQ ID NO: 5), human FGF-3 (hu FGF-3; SEQ ID NO: 6), human FGF-4 (hu FGF-4; SEQ ID NO: 7), human FGF-5 (hu FGF-5; SEQ ID NO: 8), human FGF-6 (hu FGF-6; SEQ ID NO: 9), human FGF-7 (hu FGF-7; SEQ ID NO: 10), human FGF-8 (hu FGF-8; SEQ ID NO: 11), human FGF-9 (hu FGF-9; SEQ ID NO: 12), human FGF-10 (hu FGF-10; SEQ ID NO: 13), human FGF-11 (hu FGF-11; SEQ ID NO: 14), human FGF-12 (hu FGF-12; SEQ ID NO: 15), human FGF-13 (hu FGF-13; SEQ ID NO: 16), human FGF-14 (hu FGF-14; SEQ ID NO: 17), human FGF-16 (hu FGF-16; SEQ ID NO: 18), human FGF-17 (hu FGF-17; SEQ ID NO: 19), human FGF-18 (hu FGF-18; SEQ ID NO: 20), human FGF-19 (hu FGF-19; SEQ ID NO: 21), human FGF-23 (hu FGF-23; SEQ ID NO: 22), murine FGF-1 (mu FGF-1; SEQ ID NO: 23), murine FGF-2 (mu FGF-2; SEQ ID NO: 24), murine FGF-3 (mu FGF-3; SEQ ID NO: 25), murine FGF-4 (mu FGF-4; SEQ ID NO: 26), murine FGF-5 (mu FGF-5; SEQ ID NO: 27), murine FGF-6 (mu FGF-6; SEQ ID NO: 28), murine FGF-7 (mu FGF-7; SEQ ID NO: 29), murine FGF-8 (mu FGF-8; SEQ ID NO: 30), murine FGF-9 (mu FGF-9; SEQ ID NO: 31), murine FGF-10 (mu FGF-10; SEQ ID NO: 32), murine FGF-11 (mu FGF-11; SEQ ID NO: 33), murine FGF-12 (mu FGF-12; SEQ ID NO: 34), murine FGF-13 (mu FGF-13; SEQ ID NO: 35), murine FGF-14 (mu FGF-14; SEQ ID NO: 36), murine FGF-15 (mu FGF-15; SEQ ID NO: 37), rat FGF-16 (rat FGF-16; SEQ ID NO: 38), murine FGF-17 (mu FGF-17; SEQ ID NO: 39).

**[0331]** [0330] From the amino acid sequence analysis shown in Figures 2A-2G, the FGF-23 gene appears to be closely related to murine FGF-15 and human FGF-19. The regionally restricted pattern of FGF-15 expression in the developing nervous system suggests that FGF-15 may play an important role in regulating cell division and patterning within specific regions of the embryonic brain, spinal cord, and sensory organs (McWhirter et al., 1997, Development 124:3221-32). Accordingly, FGF-23 nucleic acid molecules, polypeptides, and agonists and antagonists thereof may be useful for the diagnosis or treatment of diseases involving the developing nervous system. Human FGF-19, which is expressed in fetal cartilage, skin, and retina, adult gall bladder, and a colon adenocarcinoma cell line, maps to a region of chromosome 11 that is associated with an osteoporosis-pseudoglioma syndrome of skeletal and retinal defects (Xie et al., 1999, Cytokine 11:729-35). Accordingly, FGF-23 nucleic acid molecules, polypeptides, and agonists and antagonists thereof may be useful for the diagnosis or treatment of diseases involving the skeletal system or retina.

**[0332]** [0331] The FGF-23 gene has been shown to be most closely related to human FGF-21 (Yamashita et al., 2000, Biochem. Biophys. Res. Commun. 277:494-98), a gene which is expressed most abundantly in the liver and at lower levels in the thymus (Nishimura et al., 2000, Biochim. Biophys. Acta 1492:203-06). Accordingly, FGF-23 nucleic acid molecules, polypeptides, and agonists and antagonists thereof may be useful for the diagnosis or treatment of diseases involving the liver or thymus.

Example 2: FGF-23 mRNA Expression

**[0333]** [0332] The expression of FGF-23 was analyzed by RT-PCR. Total RNA was prepared from various human fetal tissues using standard techniques. Template and primer mixtures were prepared using 2 μg of total RNA and 50 ng of random primer (Gibco-BRL) in a volume of 12 μl. The mixtures were heated to 70°C for 10 minutes and then chilled on ice. Reverse transcription was performed by adding 4 μl of 5X first strand buffer (Gibco-BRL), 2 μl of 0.1 M DTT, and 1 μl of 10 mM dNTPs to the template-primer mixture, warming the reaction mixture to 37°C for 2 minutes, adding 1 μl of Superscript II RT (Gibco-BRL), and then incubating the reaction mixture at 37°C for 1 hour.

**[0334]** [0333] Differences in RNA concentration and cDNA conversion efficiency were normalized by performing control PCR amplifications on each cDNA sample using primers specific for glyceraldehyde-3-phosphate dehydrogenase (G3PDH), a gene expected to be expressed at about the same level in all of the tissues to be examined. Control PCR amplifications were performed using the amplimers 5'-T-C-C-A-C-C-A-C-C-C-T-G-T-T-G-C-T-G-T-A-G-3' (SEQ ID NO:

52) and 5'-G-A-C-C-A-CA-G-T-C-C-A-T-G-C-C-A-T-C-A-C-T-3' (SEQ ID NO: 53) and Ready-To-Go PCR Beads (Amersham Pharmacia Biotech, Piscataway, NJ). Reactions were performed at 95°C for 1 minute for one cycle; 92°C for 30 seconds, 55°C for 45 seconds, and 72°C for 1 minute for 25 cycles; and 72°C for 5 minutes for one cycle. Control reaction products were analyzed on 2% agarose gels, the relative intensities of the control products were estimated, and the concentration of cDNA samples was adjusted so that the cDNA samples would generate G3PDH bands of equal intensity. FGF-23 expression analysis was carried out using concentration-normalized cDNA samples.

**[0335]** [0334] The expression of FGF-23 was analyzed in PCR amplifications containing the amplimers 5'-C-T-A-T-C-C-CA-A-T-G-C-C-T-C-C-C-C-A-C-T-G-3' (SEQ ID NO: 54) and 5'-C-G-C-C-C-C-T-G-A-C-C-A-C-C-C-C-T-A-A-T-G-3' (SEQ ID NO: 55) and Ready-To-Go PCR Beads. Reactions were performed at 95°C for 5 minutes for one cycle; 95°C for 30 seconds, 68°C for 30 seconds, and 72°C for 1 minute for 30 cycles; and 72°C for 7 minutes for one cycle. PCR products were analyzed on 2% agarose gels and the relative intensities of the PCR products were estimated (using the faintest PCR product as a baseline). The results of this analysis are shown in Table III.

Table III
Relative FGF-23 Expression

| Tissue | Relative Expression Level |
| --- | --- |
| Spinal cord | +++ |
| Bladder | +++ |
| Adrenal | + |
| Bone | +/- |
| Placenta | ++ |
| Intestine | ++++ |
| Mesentery | ++ |
| Lung | ++ |
| Thymus | +/- |
| Pancreas | + |
| Cord Blood | +++ |
| Uterus | +/- |
| Heart | ++ |
| Testes | +++ |
| Eye | - |

**[0336]** [0335] FGF-23 mRNA expression is analyzed on Northern blots. Multiple human tissue Northern blots (Clontech) are probed with a suitable restriction fragment isolated from a human FGF-23 polypeptide cDNA clone. The probe is labeled with $^{32}$P-dCTP using standard techniques.

**[0337]** [0336] Northern blots are prehybridized for 2 hours at 42°C in hybridization solution (5X SSC, 50% deionized formamide, 5X Denhardt's solution, 0.5% SDS, and 100 mg/ml denatured salmon sperm DNA) and then hybridized at 42°C overnight in fresh hybridization solution containing 5 ng/ml of the labeled probe. Following hybridization, the filters are washed twice for 10 minutes at room temperature in 2X SSC and 0.1% SDS, and then twice for 30 minutes at 65°C in 0.1X SSC and 0.1% SDS. The blots are then exposed to autoradiography.

**[0338]** [0337] The expression of FGF-23 mRNA in normal adult mouse tissue and in 3-week-old high expressing and non-expressing transgenic mouse tissue (see Example 5) was localized by *in situ* hybridization. Normal embryonic and adult mouse tissues were immersion fixed, embedded in paraffin, and sectioned at 5 μm. *In situ* hybridization was performed using standard techniques. Sectioned tissues were hybridized overnight at 60°C in hybridization solution containing a $^{33}$P-labeled antisense riboprobe complementary to the human FGF-23 gene. The riboprobe was obtained by *in vitro* transcription of a clone containing human FGF-23 cDNA sequences using standard techniques.

**[0339]** [0338] Following hybridization, sections were treated with RNaseA to digest unhybridized probe and washed in 0.1X SSC at 55°C for 30 minutes. Sections were then immersed in NTB-2 emulsion (Kodak, Rochester, NY), exposed for 3 weeks at 4°C, developed, and counterstained with hematoxylin and eosin. Tissue morphology and hybridization signal were simultaneously analyzed by darkfield and standard illumination for brain, gastrointestinal system (parotid, submandibular, and sublingual glands; esophagus; stomach; duodenum; jejunum; ileum; proximal and distal colon; liver; and pancreas), cardiopulmonary system (heart, lung, trachea, and blood vessels); hematolymphoid system (lymph nodes, spleen, thymus, and bone marrow), urinary system (kidney and bladder), endocrine system (adrenal gland, thyroid gland, and pituitary gland), reproductive system (testis, prostrate, and glands; ovary, uterus, and oviduct; placenta); and musculoskeletal system (bone, skeletal muscle, skin, and adipose tissue). Normal mouse embryos at E18 and E14.5 with placenta were also analyzed by *in situ* hybridization.

**[0340]** [0339] A low to moderate, diffuse expression of FGF-23 was detected throughout the tissues of normal adult mouse. An RNAase protection assay was performed on a limited sample of mouse tissues in which a diffuse signal was detected by *in situ* hybridization, to determine whether this signal due to non-specific binding. The results of the RNAase protection assay indicated that FGF-23 is expressed only weakly in the heart and brain, and not at all in liver, thyroid/ parathyroid, and stomach. These results suggest that the diffuse signal detected by *in situ* hybridization is mostly due to non-specific binding, especially in epithelial cell types. Since the RNAase protection assay indicated that FGF-23 is weakly expressed in the heart and brain, the low signal observed in these tissues by *in situ* hybridization may be real. In the brain, generally low expression was seen in most neurons including areas of the thalamus, caudate putamen, septum, and hypothalamus. Moderate labeling, however, was noted in the hippocampal granule and pyramidal cells, the neocortex (Figure 3), and the piriform cortex. Low expression was found in the ependyma and choroid plexus. Both cardiac and skeletal muscle also exhibited low levels of FGF-23 expression. In cardiac muscle, low diffuse signal was evident throughout the left and right ventricles with a somewhat greater signal in the atrium (Figure 3). A low diffuse signal was also found in skeletal muscle.

**[0341]** [0340] No expression was found in either the E14.5 or the E18 mouse embryos.

**[0342]** [0341] Neither of the 3-week-old transgenic mouse littermates showed the diffuse non-specific signal that was detected in normal adult mouse. In the non-expressing transgenic mouse, strong FGF-23 expression was detected in scattered cells in the lymph nodes (Figure 4), thymic medulla (Figure 4), and bone (Figure 4). While positive identification of the labeled cells was not possible, the expression in bone seemed to be in the mesenchymal cells scattered in the lacunae and trabeculae in the bones of the hindlimb, vertebrae, ribs, and nasal cavities. In the high expressing transgenic mouse, the distribution of labeled cells was more widespread. In the liver, strong FGF-23 expression was found throughout the hepatocytes (Figure 5). Strong labeling was also detected in scattered cells of the thymic medulla (Figure 5), as was observed in the non-expressing transgenic mouse. However, in the high expressing transgenic mouse, well-labeled cells were also found in the red pulp of the spleen (Figure 5), smooth muscle adjoining the prostate gland (Figure 6), and striated muscle of the jaw (Figure 6). Strong expression was also detected in a few identified megakaryocytes in the bone marrow and in numerous chondrocytes in the hindlimb and in the vertebrae (Figure 6).


Example 3: Production of FGF-23 Polypeptides


A. Expression of FGF-23 Polypeptides in Bacteria

**[0343]** [0342] PCR is used to amplify template DNA sequences encoding an FGF-23 polypeptide using primers corresponding to the 5' and 3' ends of the sequence. The amplified DNA products may be modified to contain restriction enzyme sites to allow for insertion into expression vectors. PCR products are gel purified and inserted into expression vectors using standard recombinant DNA methodology. An exemplary vector, such as pAMG21 (ATCC no. 98113) containing the lux promoter and a gene encoding kanamycin resistance is digested with Bam HI and Nde I for directional cloning of inserted DNA. The ligated mixture is transformed into an *E. coli* host strain by electroporation and transformants are selected for kanamycin resistance. Plasmid DNA from selected colonies is isolated and subjected to DNA sequencing to confirm the presence of the insert.

**[0344]** [0343] Transformed host cells are incubated in 2xYT medium containing 30 $\mu$g/mL kanamycin at 30°C prior to induction. Gene expression is induced by the addition of N-(3-oxohexanoyl)-dl-homoserine lactone to a final concentration of 30 ng/mL followed by incubation at either 30°C or 37°C for six hours. The expression of FGF-23 polypeptide is evaluated by centrifugation of the culture, resuspension and lysis of the bacterial pellets, and analysis of host cell proteins by SDS-polyacrylamide gel electrophoresis.

**[0345]** [0344] Inclusion bodies containing FGF-23 polypeptide are purified as follows. Bacterial cells are pelleted by centrifugation and resuspended in water. The cell suspension is lysed by sonication and pelleted by centrifugation at 195,000 xg for 5 to 10 minutes. The supernatant is discarded, and the pellet is washed and transferred to a homogenizer. The pellet is homogenized in 5 mL of a Percoll solution (75% liquid Percoll and 0.15 M NaCl) until uniformly suspended and then diluted and centrifuged at 21,600 xg for 30 minutes. Gradient fractions containing the inclusion bodies are recovered and pooled. The isolated inclusion bodies are analyzed by SDS-PAGE.

**[0346]** [0345] A single band on an SDS polyacrylamide gel corresponding to *E. coli*-produced FGF-23 polypeptide is excised from the gel, and the N-terminal amino acid sequence is determined essentially as described by Matsudaira et al., 1987, J. Biol. Chem. 262:10-35.

B. Expression of FGF-23 Polypeptide in Mammalian Cells

**[0347]** [0346] PCR is used to amplify template DNA sequences encoding an FGF-23 polypeptide using primers corresponding to the 5' and 3' ends of the sequence. The amplified DNA products may be modified to contain restriction enzyme sites to allow for insertion into expression vectors. PCR products are gel purified and inserted into expression vectors using standard recombinant DNA methodology. An exemplary expression vector, pCEP4 (Invitrogen, Carlsbad, CA), that contains an Epstein-Barr virus origin of replication, may be used for the expression of FGF-23 polypeptides in 293-EBNA-1 cells. Amplified and gel purified PCR products are ligated into pCEP4 vector and introduced into 293-EBNA cells by lipofection. The transfected cells are selected in 100 $\mu$g/mL hygromycin and the resulting drug-resistant cultures are grown to confluence. The cells are then cultured in serum-free media for 72 hours. The conditioned media is removed and FGF-23 polypeptide expression is analyzed by SDS-PAGE.

**[0348]** [0347] FGF-23 polypeptide expression may be detected by silver staining. Alternatively, FGF-23 polypeptide is produced as a fusion protein with an epitope tag, such as an IgG constant domain or a FLAG epitope, which may be detected by Western blot analysis using antibodies to the peptide tag.

**[0349]** [0348] FGF-23 polypeptides may be excised from an SDS-polyacrylamide gel, or FGF-23 fusion proteins are purified by affinity chromatography to the epitope tag, and subjected to N-terminal amino acid sequence analysis as described herein.

C. Purification of FGF-23 Polypeptide from Mammalian Cells

**[0350]** [0349] FGF-23 polypeptide expression constructs are introduced into 293 EBNA or CHO cells using either a lipofection or calcium phosphate protocol.

**[0351]** [0350] To conduct functional studies on the FGF-23 polypeptides that are produced, large quantities of conditioned media are generated from a pool of hygromycin selected 293 EBNA clones. The cells are cultured in 500 cm Nunc Triple Flasks to 80% confluence before switching to serum-free media a week prior to harvesting the media. Conditioned media is harvested and frozen at -20°C until the protein is to be purified.

**[0352]** [0351] Conditioned media is purified by affinity chromatography as described below. The media is thawed and then passed through a 0.2 $\mu$m filter. A Protein G column is equilibrated with PBS at pH 7.0, and then loaded with the filtered media. The column is washed with PBS until the absorbance at $A_{280}$ reaches a baseline. FGF-23 polypeptide is eluted from the column with 0.1 M Glycine-HCl at pH 2.7 and immediately neutralized with 1 M Tris-HCl at pH 8.5. Fractions containing FGF-23 polypeptide are pooled, dialyzed in PBS, and stored at -70°C.

**[0353]** [0352] For Factor Xa cleavage of the human FGF-23 polypeptide-Fc fusion polypeptide, affinity chromatography-purified protein is dialyzed in 50 mM Tris-HCl, 100 mM NaCl, 2 mM $CaCl_2$ at pH 8.0. The restriction protease Factor Xa is added to the dialyzed protein at 1/100 (w/w) and the sample digested overnight at room temperature.

Example 4: Production of Anti-FGF-23 Polypeptide Antibodies

**[0354]** [0353] Antibodies to FGF-23 polypeptides may be obtained by immunization with purified protein or with FGF-23 peptides produced by biological or chemical synthesis. Suitable procedures for generating antibodies include those described in Hudson and Bay, Practical Immunology (2nd ed., Blackwell Scientific Publications).

**[0355]** [0354] In one procedure for the production of antibodies, animals (typically mice or rabbits) are injected with an FGF-23 antigen (such as an FGF-23 polypeptide), and those with sufficient serum titer levels as determined by ELISA are selected for hybridoma production. Spleens of immunized animals are collected and prepared as single cell suspensions from which splenocytes are recovered. The splenocytes are fused to mouse myeloma cells (such as Sp2/0-Ag14 cells), are first incubated in DMEM with 200 U/mL penicillin, 200 $\mu$g/mL streptomycin sulfate, and 4 mM glutamine, and are then incubated in HAT selection medium (hypoxanthine, aminopterin, and thymidine). After selection, the tissue culture supernatants are taken from each fusion well and tested for anti- FGF-23 antibody production by ELISA.

**[0356]** [0355] Alternative procedures for obtaining anti-FGF-23 antibodies may also be employed, such as the immunization of transgenic mice harboring human Ig loci for production of human antibodies, and the screening of synthetic antibody libraries, such as those generated by mutagenesis of an antibody variable domain.

Example 5: Expression of FGF-23 Polypeptide in Transgenic Mice

**[0357]** [0356] To assess the biological activity of FGF-23 polypeptide, a construct encoding FGF-23 polypeptide under the control of the ApoE promoter (TH00-026) was prepared. The expression of the FGF-23 gene was expected to cause pathological changes in the transgenic mice that would be informative as to the function of FGF-23 polypeptide.

**[0358]** [0357] A distinctive phenotype was produced in 3-week-old BDF 1 mice following transfer of the TH00-026 construct in that litters had an unusually high number of runts. While not all of the expressor mice were runted and some of the non-expressing littermates were runted, the proportion of runts was higher among the expressor mice. All runts, including a number of the non-expressing runts, were taken down before weaning for examination. The skull of all expressing mice was shortened and more rounded, with the lower jaw developing properly. As a result, all expressing mice had protruding lower teeth. This condition is obvious by external examination and radiographic evaluation. In addition, the two highest expressing mice were found to have low serum phosphorous and low serum calcium levels. However, other signs of rickets - such as inadequate mineralization, overgrowth of epiphyseal cartilage, deranged organization of cartilage, and overgrowth of capillaries (Pathologic Basis of Disease (Cotran ed., 1994)) - were not observed. Bone morphology in expressing runts was no different than that in non-expressing runts, and both types of runts differed from non-runt littermates.

**[0359]** [0358] Partial hepatectomy was performed on 22 DNA positive mice and 4 DNA negative mice. All hepatectomized mice were bled and serum calcium, phosphorous, and alkaline phosphatase measurements were performed. The animals were also examined for the protruding lower teeth phenotype. The groups of mice that were evaluated included: controls, macroscopically phenotypic expressors, non-phenotypic high expressors, and moderate expressors. All phenotypic mice were found to be strong or very strong expressors. Serum calcium levels were somewhat lower in the phenotypic mice than in the other groups, but this difference was not statistically significant. However, serum phosphorous levels were found to be significantly lower in the phenotypic expressors and the non-phenotypic high expressors than in the moderate expressors and the controls. Serum alkaline phosphatase (ALP) was significantly elevated in the phenotypic group versus the controls and moderate expressors (see Table IV). The variability in the phenotype of the expressors may be due to genetic variation in the BDF1 mice, an outbred line that is a cross between the C57/B6 and DBA mouse strains.

Table IV
Serum Calcium, Phospohorous, and ALP in TH00-026 Transgenic Mice

|  | Control | Phenotypic expressor | Non-phenotypic high expressor | Moderate expressor | P value |
|---|---|---|---|---|---|
| Se Ca (mg/dL) | $8.97 \pm 0.26$ | $8.64 \pm 0.27$ | $8.87 \pm 0.32$ | $9.00 \pm 0.27$ | P<0.14 |
| Se P (mg/dL) | $6.67 \pm 0.52$ | $4.96 \pm 1.52$ | $5.22 \pm 1.58$ | $7.03 \pm 0.58$ | P<0.0016 |
| Se ALP (IU) | $126.5 \pm 33$ | $255.4 \pm 75$ | $206.4 \pm 117$ | $146.3 \pm 27$ | P<0.015 |

**[0360]** [0359] While the present invention has been described in terms of the preferred embodiments, it is understood that variations and modifications will occur to those skilled in the art. Therefore, it is intended that the appended claims cover all such equivalent variations that come within the scope of the invention as claimed.

**Claims**

1. An isolated nucleic acid molecule comprising a nucleotide sequence selected from the group consisting of:

   (a) the nucleotide sequence as set forth in SEQ ID NO: 1;
   (b) the nucleotide sequence of the DNA insert in ATCC Deposit No. PTA-1617;
   (c) a nucleotide sequence encoding the polypeptide as set forth in SEQ ID NO: 2;
   (d) a nucleotide sequence which hybridizes under moderately or highly stringent conditions to the complement of any of (a) - (c); and
   (e) a nucleotide sequence complementary to any of (a) - (c).

2. An isolated nucleic acid molecule comprising a nucleotide sequence selected from the group consisting of:

   (a) a nucleotide sequence encoding a polypeptide which is at least about 70 percent identical to the polypeptide as set forth in SEQ ID NO: 2, wherein the encoded polypeptide has an activity of the polypeptide set forth in SEQ ID NO: 2;
   (b) a nucleotide sequence encoding an allelic variant or splice variant of the nucleotide sequence as set forth in SEQ ID NO: 1, the nucleotide sequence of the DNA insert in ATCC Deposit No. PTA-1617, or (a);
   (c) a region of the nucleotide sequence of SEQ ID NO: 1, the DNA insert in ATCC Deposit No. PTA-1617, (a), or (b) encoding a polypeptide fragment of at least about 25 amino acid residues, wherein the polypeptide fragment has an activity of the encoded polypeptide as set forth in SEQ ID NO: 2, or is antigenic;
   (d) a region of the nucleotide sequence of SEQ ID NO: 1, the DNA insert in ATCC Deposit No. PTA-1617, or any of (a) - (c) comprising a fragment of at least about 16 nucleotides;
   (e) a nucleotide sequence which hybridizes under moderately or highly stringent conditions to the complement of any of (a) - (d); and
   (f) a nucleotide sequence complementary to any of (a) - (d).

3. An isolated nucleic acid molecule comprising a nucleotide sequence selected from the group consisting of:

   (a) a nucleotide sequence encoding a polypeptide as set forth in SEQ ID NO: 2 with at least one conservative amino acid substitution, wherein the encoded polypeptide has an activity of the polypeptide set forth in SEQ ID NO: 2;
   (b) a nucleotide sequence encoding a polypeptide as set forth in SEQ ID NO: 2 with at least one amino acid insertion, wherein the encoded polypeptide has an activity of the polypeptide set forth in SEQ ID NO: 2;
   (c) a nucleotide sequence encoding a polypeptide as set forth in SEQ ID NO: 2 with at least one amino acid deletion, wherein the encoded polypeptide has an activity of the polypeptide set forth in SEQ ID NO: 2;
   (d) a nucleotide sequence encoding a polypeptide as set forth in SEQ ID NO: 2 which has a C- and/or N- terminal truncation, wherein the encoded polypeptide has an activity of the polypeptide set forth in SEQ ID NO: 2;
   (e) a nucleotide sequence encoding a polypeptide as set forth in SEQ ID NO: 2 with at least one modification selected from the group consisting of amino acid substitutions, amino acid insertions; amino acid deletions, C-terminal truncation, and N-terminal truncation, wherein the encoded polypeptide has an activity of the polypeptide set forth in SEQ ID NO: 2;
   (f) a nucleotide sequence of any of (a) - (e) comprising a fragment of at least about 16 nucleotides; ,
   (g) a nucleotide sequence which hybridizes under moderately or highly stringent conditions to the complement of any of (a) - (f); and
   (h) a nucleotide sequence complementary to any of (a) - (e).

4. A vector comprising the nucleic acid molecule of any of Claims I, 2, or 3.

5. A host cell comprising the vector of Claim 4.

6. The host cell of Claim 5 that is a eukaryotic cell.

7. The host cell of Claim 5 that is a prokaryotic cell.

8. A process of producing an FGF-23 polypeptide comprising culturing the host cell of Claim 5 under suitable conditions to express the polypeptide, and optionally isolating the polypeptide from the culture.

9. A polypeptide produced by the process of Claim 8.

10. The process of Claim 8, wherein the nucleic acid molecule comprises promoter DNA other than the promoter DNA for the native FGF-23 polypeptide operatively linked to the DNA encoding the FGF-23 polypeptide.

11. The isolated nucleic acid molecule according to Claim 2, wherein the percent identity is determined using a computer program selected from the group consisting of GAP, BLASTN, FASTA, BLASTA, BLASTX, BestFit, and the Smith-Waterman algorithm.

12. A process for determining whether a compound inhibits FGF-23 polypeptide activity or FGF-23 polypeptide production comprising exposing a cell according to any of Claims 5, 6, or 7 to the compound and measuring FGF-23 polypeptide activity or FGF-23 polypeptide production in said cell.

13. An isolated polypeptide comprising the amino acid sequence selected from the group consisting of:

(a) the amino acid sequence as set forth in SEQ ID NO: 2; and
(b) the amino acid sequence encoded by the DNA insert in ATCC Deposit No. PTA-1617.

14. An isolated polypeptide comprising the amino acid sequence selected from the group consisting of:

(a) the amino acid sequence as set forth in SEQ ID NO: 3, optionally further comprising an ammo-terminal methionine;
(b) an amino acid sequence for an ortholog of SEQ ID NO: 2;
(c) an amino acid sequence which is at least about 70 percent identical to the amino acid sequence of SEQ ID NO: 2, wherein the polypeptide has an activity of the polypeptide set forth in SEQ ID NO: 2;
(d) a fragment of the amino acid sequence set forth in SEQ ID NO: 2 comprising at least about 25 amino acid residues, wherein the fragment has an activity of the polypeptide set forth in SEQ ID NO: 2, or is antigenic; and
(e) an amino acid sequence for an allelic variant or splice variant of the amino acid sequence as set forth in SEQ ID NO: 2, the amino acid sequence encoded by the DNA insert in ATCC Deposit No. PTA-1617, or (a) - (c).

15. An isolated polypeptide comprising the amino acid sequence selected from the group consisting of:

(a) the amino acid sequence as set forth in SEQ ID NO: 2 with at least one conservative amino acid substitution, wherein the polypeptide has an activity of the polypeptide set forth in SEQ ID NO: 2;
(b) the amino acid sequence as set forth in SEQ ID NO: 2 with at least one amino acid insertion, wherein the polypeptide has an activity of the polypeptide set forth in SEQ ID NO: 2;
(c) the amino acid sequence as set forth in SEQ ID NO: 2 with at least one amino acid deletion, wherein the polypeptide has an activity of the polypeptide set forth in SEQ ID NO: 2;
(d) the amino acid sequence as set forth in SEQ ID NO: 2 which has a C- and/or N- terminal truncation, wherein the polypeptide has an activity of the polypeptide set forth in SEQ ID NO: 2; and
(e) the amino acid sequence as set forth in SEQ ID NO: 2 with at least one modification selected from the group consisting of amino acid substitutions, amino acid insertions, amino acid deletions, C-tenninal truncation, and N-terminal truncation, wherein the polypeptide has an activity of the polypeptide set forth in SEQ ID NO: 2.

16. An isolated polypeptide encoded by the nucleic acid molecule of any of Claims 1, 2, or 3, wherein the polypeptide has an activity of the polypeptide set forth in SEQ ID NO: 2.

17. The isolated polypeptide according to Claim 14, wherein the percent identity is determined using a computer program selected from the group consisting of GAP, BLASTP, FASTA, BLASTA, BLASTX, DestFit, and the Smith-Waterman algorithm.

18. A selective binding agent or fragment thereof which specifically binds the polypeptide of any of Claims 13, 14, or 15.

19. The selective binding agent or fragment thereof of Claim 18 that specifically binds the polypeptide comprising the amino acid sequence as set forth in SEQ ID NO: 2, or a fragment thereof.

20. The selective binding agent of Claim 18 that is an antibody or fragment thereof.

**21.** The selective binding agent of Claim 18 that is a humanized antibody.

**22.** The selective binding agent of Claim 18 that is a human antibody or fragment thereof.

**23.** The selective binding agent of Claim 18 that is a polyclonal antibody or fragment thereof.

**24.** The selective binding agent Claim 18 that is a monoclonal antibody or fragment thereof.

**25.** The selective binding agent of Claim 18 that is a chimeric antibody or fragment thereof.

**26.** The selective binding agent of Claim 18 that is a CDR-grafted antibody or fragment thereof.

**27.** The selective binding agent of Claim 18 that is an antiidiotypic antibody or fragment thereof.

**28.** The selective binding agent of Claim 18 that is a variable region fragment.

**29.** The variable region fragment of Claim 28 that is a Fab or a Fab' fragment.

**30.** A selective binding agent or fragment thereof comprising at least one complementarity determining region with specificity for a polypeptide having the amino acid sequence of SEQ ID NO: 2.

**31.** The selective binding agent of Claim 18 that is bound to a detectable label.

**32.** The selective binding agent of Claim 18 that antagonizes FGF-23 polypeptide biological activity.

**33.** A method for treating, preventing, or ameliorating an FGF-23 polypeptide-related disease, condition, or disorder comprising administering to a patient an effective amount of a selective binding agent according to Claim 18.

**34.** A selective binding agent produced by immunizing an animal with a polypeptide comprising an amino acid sequence of SEQ ID NO: 2.

**35.** A hybridoma which produces a selective binding agent which is capable of binding a polypeptide according to any of Claims 1, 2, or 3.

**36.** A method of detecting or quantitating the amount of FGF-23 polypeptide using the anti-FGF-23 antibody or fragment of Claim 18.

**37.** A composition comprising the polypeptide of any of Claims 13, 14, or 15, and a pharmaceutically acceptable formulation agent.

**38.** The composition of Claim 37, wherein the pharmaceutically acceptable formulation agent is a carrier, adjuvant, solubilizer, stabilizer, or anti-oxidant.

**39.** The composition of Claim 37, wherein the polypeptide comprises the amino acid sequence as set forth in SEQ ID NO: 3.

**40.** A polypeptide comprising a derivative of the polypeptide of any of Claims 13, 14, or 15.

**41.** The polypeptide of Claim 40 that is covalently modified with a water-soluble polymer.

**42.** The polypeptide of Claim 41, wherein the water-soluble polymer is selected from the group consisting of polyethylene glycol, monomethoxy polyethylene glycol, dextran, cellulose, poly-(N-vinyl pyrrolidone) polyethylene glycol, propylene glycol homopolymers, polypropylene oxide/ethylene oxide copolymers, polyoxyethylated polyols, and polyvinyl alcohol.

**43.** A composition comprising a nucleic acid molecule of any of Claims 1, 2, or 3 and, a pharmaceutically acceptable formulation agent.

**44.** The composition of Claim 43, wherein said nucleic acid molecule is contained in a viral vector.

**45.** A viral vector comprising a nucleic acid molecule of any of Claims 1, 2, or 3.

**46.** A fusion polypeptide comprising the polypeptide of any of Claims 13, 14, or 15 fused to a heterologous amino acid sequence.

**47.** The fusion polypeptide of Claim 46, wherein the heterologous amino acid sequence is an IgG constant domain or fragment thereof.

**48.** Use of the polypeptide of any of claims 13, 14 or 15, or the polypeptide encoded by the nucleic acid of any of claims 1, 2 or 3 for the preparation of a medicament for treating, preventing, or ameliorating a medical condition.

**49.** Use of an agonist or antagonist of the biological activity of the polypeptide of any of claims 12, 14, or 15 or of the polypeptide encoded by the nucleic acid of any of claims 1, 2 or 3 for the preparation of a medicament for treating, preventing, or ameliorating a medical condition.

**50.** The use of either claim 48 or 49 wherein the medical condition being treated, prevented, or ameliorated is autosomal dominant hypophosphatemic rickets (ADHR).

**51.** A method of diagnosing a pathological condition or a susceptibility to a pathological condition in a subject comprising:

(a) determining the presence or amount of expression of the polypeptide of any of Claims 13, 14, or 15, or the polypeptide encoded by the nucleic acid molecule of any of Claims 1, 2, or 3 in a sample; and
(b) diagnosing a pathological condition or a susceptibility to a pathological condition based on the presence or amount of expression of the polypeptide.

**52.** A device, comprising:

(a) a membrane suitable for implantation; and
(b) cells encapsulated within said membrane, wherein said cells secrete a protein of any of Claims 13,14; or 15; and

said membrane is permeable to said protein and impermeable to materials detrimental to said cells.

**53.** A method of identifying a compound which binds to an FGF-23 polypeptide comprising:

(a) contacting the polypeptide of any of Claims 13, 14, or 15 with a compound; and
(b) determining the extent of binding of the FGF-23 polypeptide to the compound.

**54.** The method of Claim 53, further comprising determining the activity of the polypeptide when bound to the compound.

**55.** Use of the nucleic acid molecule of any of claims 1, 2 or 3 for the preparation of a medicament for modulating levels of a polypeptide in an animal.

**56.** A transgenic non-human mammal comprising the nucleic acid molecule of any of Claims 1, 2, or 3.

**57.** An in-vitro method for determining whether a compound inhibits FGF-23 polypeptide activity or FGF-23 polypeptide production in a transgenic mammal according to claim 56, by measuring FGF-23 polypeptide activity or FGF-23 polypeptide activity or FGF-23 polypeptide production in a sample obtained from said mammal, wherein said mammal has previously been exposed to said compound.

# Figure 1A

```
atg ttg ggg gcc cgc ctc agg ctc tgg gtc tgt gcc ttg tgc agc gtc    48
Met Leu Gly Ala Arg Leu Arg Leu Trp Val Cys Ala Leu Cys Ser Val
 1               5                   10                  15

tgc agc atg agc gtc ctc aga gcc tat ccc aat gcc tcc cca ctg ctc    96
Cys Ser Met Ser Val Leu Arg Ala Tyr Pro Asn Ala Ser Pro Leu Leu
                20                  25                  30

ggc tcc agc tgg ggt ggc ctg atc cac ctg tac aca gcc aca gcc agg   144
Gly Ser Ser Trp Gly Gly Leu Ile His Leu Tyr Thr Ala Thr Ala Arg
            35                  40                  45

aac agc tac cac ctg cag atc cac aag aat ggc cat gtg gat ggc gca   192
Asn Ser Tyr His Leu Gln Ile His Lys Asn Gly His Val Asp Gly Ala
        50                  55                  60

ccc cat cag acc atc tac agt gcc ctg atg atc aga tca gag gat gct   240
Pro His Gln Thr Ile Tyr Ser Ala Leu Met Ile Arg Ser Glu Asp Ala
 65                  70                  75                  80

ggc ttt gtg gtg att aca ggt gtg atg agc aga aga tac ctc tgc atg   288
Gly Phe Val Val Ile Thr Gly Val Met Ser Arg Arg Tyr Leu Cys Met
                85                  90                  95

gat ttc aga ggc aac att ttt gga tca cac tat ttc gac ccg gag aac   336
Asp Phe Arg Gly Asn Ile Phe Gly Ser His Tyr Phe Asp Pro Glu Asn
            100                 105                 110

tgc agg ttc caa cac cag acg ctg gaa aac ggg tac gac gtc tac cac   384
Cys Arg Phe Gln His Gln Thr Leu Glu Asn Gly Tyr Asp Val Tyr His
        115                 120                 125

tct cct cag tat cac ttc ctg gtc agt ctg ggc cgg gcg aag aga gcc   432
Ser Pro Gln Tyr His Phe Leu Val Ser Leu Gly Arg Ala Lys Arg Ala
        130                 135                 140

ttc ctg cca ggc atg aac cca ccc ccg tac tcc cag ttc ctg tcc cgg   480
Phe Leu Pro Gly Met Asn Pro Pro Pro Tyr Ser Gln Phe Leu Ser Arg
145                 150                 155                 160

agg aac gag atc ccc cta att cac ttc aac acc ccc ata cca cgg cgg   528
Arg Asn Glu Ile Pro Leu Ile His Phe Asn Thr Pro Ile Pro Arg Arg
                165                 170                 175

cac acc cgg agc gcc gag gac gac tcg gag cgg gac ccc ctg aac gtg   576
His Thr Arg Ser Ala Glu Asp Asp Ser Glu Arg Asp Pro Leu Asn Val
            180                 185                 190

ctg aag ccc cgg gcc cgg atg acc ccg gcc ccg gcc tcc tgt tca cag   624
Leu Lys Pro Arg Ala Arg Met Thr Pro Ala Pro Ala Ser Cys Ser Gln
            195                 200                 205
```

# Figure 1B

```
gag ctc ccg agc gcc gag gac aac agc ccg atg gcc agt gac cca tta    672
Glu Leu Pro Ser Ala Glu Asp Asn Ser Pro Met Ala Ser Asp Pro Leu
    210                 215                 220


ggg gtg gtc agg ggc ggt cga gtg aac acg cac gct ggg gga acg ggc    720
Gly Val Val Arg Gly Gly Arg Val Asn Thr His Ala Gly Gly Thr Gly
225                 230                 235                 240


ccg gaa ggc tgc cgc ccc ttc gcc aag ttc atc                        753
Pro Glu Gly Cys Arg Pro Phe Ala Lys Phe Ile
                245                 250
```

# FIG. 2A

```
hu FGF-1    MA...E....  ..........  ..........  ..........  ........GE
hu FGF-2    MA...A....  ..........  ..........  ..........  ........GS
hu FGF-3    M....G....  ..LIWLL...  ..........  ........LL  SL......L
hu FGF-4    MS........  ..........  .GPGTAAVAL  LPAVL...LA  LLAPWAGRGG
hu FGF-5    MSLSFL....  ..LLLFFSHL  ILSAWAHGEK  R.........  .LAPKGQPGP
hu FGF-6    MALGQK....  ..LFITMSR.  .GAGRLQGTL  WALVF...LG  ILVGMVVPSP
hu FGF-7    MH...K....  ....WILTWI  LPTLLYRS..  ...CF...HI  IC......L
hu FGF-8    MGSPRSALSC  LLLHLLVLCL  QAQ...EGPG  RGPALGRELA  SL........
hu FGF-9    MA...P....  ..LGEVGNYF  GVQD......  ........AV  PF......GN
hu FGF-10   MW...K....  ....WILTHC  ASAFPHLPGC  CCCCF...LL  LF......L
hu FGF-11   MAA.......  .....LASSL  IRQKREVRE.  ..........  ....PGGSRP
hu FGF-12   MAAA......  .....IASSL  IRQKRQARE.  ..........  ....SNSDRV
hu FGF-13   MAAA......  .....IASSL  IRQKRQARE.  ..........  ....R..EKS
hu FGF-14   MAAA......  .....IASGL  IRQKRQARE.  ..........  ....QHWDRP
hu FGF-16   MA........  ....EVGGVF  ASLDWDLHG.  ....F...SS  SL......GN
hu FGF-17   MGAAR.....  .LLPNLTLCL  QLLI......  ........LC  CQTQGEN...
hu FGF-18   MYSAPSACTC  LCLHFLLLCF  QVQVLVA...  ..........  .....EENVD
hu FGF-19   M...RS..GC  VVVHVWI...  LAGLWLA...  ...VAGRPLA  ..........
hu FGF-23   MSVLRA....  ..........  ..........  ..........  ..........
mu FGF-1    MA...E....  ..........  ..........  ..........  ........GE
mu FGF-2    MA...A....  ..........  ..........  ..........  ........SG
mu FGF-3    M....G....  ..LIWLL...  ..........  ........LL  SL......L
mu FGF-4    MA........  ..........  .KRGPTTGTL  LPRVL...LA  LVVALADRGT
mu FGF-5    MSLSLL....  ..FLIFCSHL  IHSAWAHGEK  R.........  .LTPEGQPAP
mu FGF-6    MALGQR....  ..LFITMSR.  .GAGRVQGTL  QALVF...LG  VLVGMVVPSP
mu FGF-7    MR...K....  ....WILTRI  LPTLLYRS..  ...CF...HL  VC......L
mu FGF-8    MGSPRSALSC  LLLHLLVLCL  QAQVRSAAQK  RGPGAGNPAD  TLGQGHEDRP
mu FGF-9    MA...P....  ..LGEVGSYF  GVQD......  ........AV  PF......GN
mu FGF-10   MW...K....  ....WILTHC  ASAFPHLPGC  CCC.F...LL  LF......L
mu FGF-11   MAA.......  .....LASSL  IRQKREVRE.  ..........  ....PGGSRP
mu FGF-12   MAAA......  .....IASSL  IRQKRQARE.  ..........  ....SNSDRV
mu FGF-13   MTAA......  .....IASSL  IRQKRQARE.  ..........  ....R..EKS
mu FGF-14   MAAA......  .....IASGL  IRQKRQARE.  ..........  ....QHWDRP
mu FGF-15   MARKWN..GR  AVARALV...  LATLWLA...  ...VSGRPLA  ..........
rat FGF-16  MA........  ....EVGGVF  ASLDWDLQG.  ....F...SS  SL......GN
mu FGF-17   MYSAPSACTC  LCLHFLLLCF  QVQVLAA...  ..........  .....EENVD
```

## FIG. 2B

```
hu FGF-1    ITTFTA....  .....LT.EK  FN...LPPGN  YKKP......  ..........
hu FGF-2    ITTLPA....  .....LP.ED  GGSGAFPPGH  FKDP......  ..........
hu FGF-3    EPGWPAA...  ..........  .....GPGAR  LR.RDAGGRG  G.VYEHL...
hu FGF-4    AAAPTAPNGT  LEAELER.RW  ESLVALSLAR  LPVAAQPKEA  AVQSGAGDYL
hu FGF-5    AATDRNPIGS  S.SRQSS.SS  AMSSSSASSS  PAASLGSQGS  GLEQSSFQWS
hu FGF-6    AGTR..ANNT  LLD..SR.GW  GTLLSRSRAG  LAGEI....A  GVNWESG.YL
hu FGF-7    VGTISLACND  MTPEQMA.TN  V.NCSSPE..  ........RH  TRSYDYME..
hu FGF-8    F....RAGRE  ........PQ  G.........  ...VSQQHVR  EQSLVTDQLS
hu FGF-9    VPVLPVDSPV  LLSDHLG.QS  EA........  ..GGLPRGPA  VTDLDHLK..
hu FGF-10   VSSVPVTCQA  LGQDMVS.PE  ATN..SSSSS  FSSPSSAGRH  VRSYNHLQ..
hu FGF-11   VSAQRRVCP.  RGTKSLC.QK  QLLILLSKVR  LCGGRPARPD  RGPEPQLK..
hu FGF-12   SASKRRSSPS  KDGRSLC.ER  HVLGVFSKVR  FCSGRKRPVR  RRPEPQLK..
hu FGF-13   NACKCVSSPS  K.GKTSC.DK  NKLNVFSRVK  LFGSKKRR.R  RRPEPQLK..
hu FGF-14   SASRRRSSPS  KN.RGLC.NG  NLVDIFSKVR  IFGLKKRRLR  RQ.DPQLK..
hu FGF-16   VPL..ADSPG  FLNERLG.QI  E.........  ..GKLQRGSP  .TDFAHLK..
hu FGF-17   ..........  ..HPSPNF..  ..........  .....NQYVR  DQGAMTDQLS
hu FGF-18   F.........  ..........  ..........  .....RIHVE  NQTRARDDVS
hu FGF-19   ........FS  DAGPHVHYGW  ..........  ..........  ..........
hu FGF-23   ........YP  NASPLLGSSW  ..........  ..........  ..........
mu FGF-1    ITTFAA....  .....LT.ER  FN...LPLGN  YKKP......  ..........
mu FGF-2    ITSLPA....  .....LP.ED  GGA.AFPPGH  FKDP......  ..........
mu FGF-3    EPSWPTT...  ..........  .....GPGTR  LR.RDAGGRG  G.VYEHL...
mu FGF-4    AA....PNGT  RHAELGH.GW  DGLVARSLAR  LPVAAQPPQA  AVRSGAGDYL
mu FGF-5    P...RNPGDS  SGSRGRS.SA  TFSSSSASSP  VAASPGSQGS  GSEHSSFQWS
mu FGF-6    AGAR..ANGT  LLD..SR.GW  GTLLSRSRAG  LAGEI....S  GVNWESG.YL
mu FGF-7    VGTISLACND  MSPEQTA.TS  V.NCSSPE..  ........RH  TRSYDYME..
mu FGF-8    FGQRSRAGKN  FTNPAPNYPE  EGSKEQRDSV  LPKVTQRHVR  EQSLVTDQLS
mu FGF-9    VPVLPVDSPV  LLNDHLG.QS  EA........  ..GGLPRGPA  VTDLDHLK..
mu FGF-10   VSSFPVTCQA  LGQDMVS.QE  ATNCSSSSSS  FSSPSSAGRH  VRSYNHLQ..
mu FGF-11   VSAQRRVCP.  RGTKSLC.QK  QLLILLSKVR  LCGGRPTRQD  RGPEPQLK..
mu FGF-12   SASKRRSSPS  KDGRSLC.ER  HVLGVFSKVR  FCSGRKRPVR  RRPEPQLK..
mu FGF-13   NACKCVSSPS  K.GKTSC.DK  NKLNVFSRVK  LFGSKKRR.R  RRPBPQLK..
mu FGF-14   SASRRRSSPS  KN.RGLF.NG  NLVDIFSKVR  IFGLKKRRLR  RQ.DPQLK..
mu FGF-15   ...QQSQSVS  DEDPLFLYGW  ..........  ..........  ..........
rat FGF-16  VPL..ADSPG  FLNERLG.QI  E.........  ..GKLQRGSP  .TDFAHLK..
mu FGF-17   F.........  ..........  ..........  .....RIHVE  NQTRARDDVS
```

# FIG. 2C

```
hu FGF-1    ......KLLY CSNG.GH... FLRILPDGTV DGTRDRSDQH IQLQLSAESV
hu FGF-2    ......KRLY CKNG.GF... FLRIHPDGRV DGVREKSDPH IKLQLQAEER
hu FGF-3    GGAPRRRKLY CATK..Y... HLQLHPSGRV NGSL.ENSAY SILEITAVEV
hu FGF-4    LGIKRLRRLY CNVGIGF... HLQALPDGRI GGAHADT.RD SLLELSPVER
hu FGF-5    PSGRRTGSLY CRVGIGF... HLQIYPDGKV NGSHEAN.ML SVLEIFAVSQ
hu FGF-6    VGIKRQRRLY CNVGIGF... HLQVLPDGRI SGTHEEN.PY SLLEISTVER
hu FGF-7    GGDIRVRRLF CRTQ..W... YLRIDKRGKV KGTQEMKNNY NIMEIRTVAV
hu FGF-8    RRLIRTYQLY SRTS.GK... HVQVLANKRI NAMAEDGDPF AKLIVETDTF
hu FGF-9    G.ILRRRQLY CRTG..F... HLEIFPNGTI QGTRKDHSRF GILEFISIAV
hu FGF-10   G.DVRWRKLF SFTK..Y... FLKIEKNGKV SGTKKENCPY SILEITSVEI
hu FGF-11   .GIV..TKLF CRQ..GF... YLQANPDGSI QGTPEDTSSF THFNLIPVGL
hu FGF-12   .GIV..TRLF SQQ..GY... FLQMHPDGTI DGTKDENSDY TLFNLIPVGL
hu FGF-13   .GIV..TKLY SRQ..GY... HLQLQADGTI DGTKDEDSTY TLFNLIPVGL
hu FGF-14   .GIV..TRLY CRQ..GY... YLQMHPDGAL DGTKDDSTNS TLFNLIPVGL
hu FGF-16   G.ILRRRQLY CRTG..F... HLEIFPNGTV HGTRHDHSRF GILEFISLAV
hu FGF-17   RRQIREYQLY SRTS.GK... HVQV.TGRRI SATAEDGNKF AKLIVETDTF
hu FGF-18   RKQLRLYQLY SRTS.GK... HIQVL.GRRI SARGEDGDKY AQLLVETDTF
hu FGF-19   GDPIRLRHLY TSGPHGLSSC FLRIRADGVV DCARGQSA.H SLLEIKAVAL
hu FGF-23   GGLIH...LY TATARN..SY HLQIHKNGHV DGAPHQTI.Y SALMIRSEDA
mu FGF-1    ......KLLY CSNG.GH... FLRILPDGTV DGTRDRSDQH IQLQLSAESA
mu FGF-2    ......KRLY CKNG.GF... FLRIHPDGRV DGVREKSDPH VKLQLQAEER
mu FGF-3    GGAPRRRKLY CATK..Y... HLQLHPSGRV NGSL.ENSAY SILEITAVEV
mu FGF-4    LGLKRLRRLY CNVGIGF... HLQVLPDGRI GGVHADT.RD SLLELSPVQR
mu FGF-5    PSGRRTGSLY CRVGIGF... HLQIYPDGKV NGSHEAS.VL SILEIFAVSQ
mu FGF-6    VGIKRQRRLY CNVGIGF... HLQVPPDGRI SGTHEEN.PY SLLEISTVER
mu FGF-7    GGDIRVRRLF CRTQ..W... YLRIDKRGKV KGTQEMKNSY NIMEIRTVAV
mu FGF-8    RRLIRTYQLY SRTS.GK... HVQVLANKRI NAMAEDGDPF AKLIVETDTF
mu FGF-9    G.ILRRRQLY CRTG..F... HLEIFPNGTI QGTRKDHSRF GILEFISIAV
mu FGF-10   G.DVRWRRLF SFTK..Y... FLTIEKNGKV SGTKNEDCPY SVLEITSVEI
mu FGF-11   .GIV..TKLF CRQ..GF... YLQANPDGSI QGTPEDTSSF THFNLIPVGL
mu FGF-12   .GIV..TRLF SQQ..GY... FLQMHPDGTI DGTKDENSDY TLFNLIPVGL
mu FGF-13   .GIV..TKLY SRQ..GY... HLQLQADGTI DGTKDEDSTY TLFNLIPVGL
mu FGF-14   .GIV..TRLY CRQ..GY... YLQMHPDGAL DGTKDDSTNS TLFNLIPVGL
mu FGF-15   GKITRLQYLY SAGPY.VSNC FLRIRSDGSV DCEEDQNE.R NLLEFRAVAL
rat FGF-16  G.ILRRRQLY CRTG..F... HLEIFPNGTV HGTRHDHSRF GILEFISLAV
mu FGF-17   RKQLRLYQLY SRTS.GK... HIQVL.GRRI SARGEDGDKY AQLLVETDTF
```

# FIG. 2D

```
hu FGF-1    GE.VYIKSTE  TGQYLAMDTD  GLLYGSQTPN  E.ECLFLERL  EENHYNTYIS
hu FGF-2    GV.VSIKGVC  ANRYLAMKED  GRLLASKCVT  D.ECFFFERL  ESNNYNTYRS
hu FGF-3    GI.VAIRGLF  SGRYLAMNKR  GRLYASEHYS  A.ECEFVERI  HELGYNTYAS
hu FGF-4    GV.VSIFGVA  SRFFVAMSSK  GKLYGSPFFT  D.ECTFKEIL  LPNNYNAYES
hu FGF-5    GI.VGIRGVF  SNKFLAMSKK  GKLHASAKFT  D.DCKFRERF  QENSYNTYAS
hu FGF-6    GV.VSLFGVR  SALFVAMNSK  GRLYATPSFQ  E.ECKFRETL  LPNNYNAYES
hu FGF-7    GI.VAIKGVE  SEFYLAMNKE  GKLYAKKECN  E.DCNFKELI  LENHYNTYAS
hu FGF-8    GSRVRVRGAE  TGLYICMNKK  GKLIAKSNGK  GKDCVFTEIV  LENNYTALQN
hu FGF-9    GL.VSIRGVD  SGLYLGMNEK  GELYGSEKLT  Q.ECVFREQF  EENWYNTYSS
hu FGF-10   GV.VAVKAIN  SNYYLAMNKK  GKLYGSKEFN  N.DCKLKERI  EENGYNTYAS
hu FGF-11   RV.VTIQSAK  LGHYMAMNAE  GLLYSSPHFT  A.ECRFKECV  FENYVLYAS
hu FGF-12   RV.VAIQGVK  ASLYVAMNGE  GYLYSSDVFT  P.ECKFKESV  FENYVIYSS
hu FGF-13   RV.VAIQGVQ  TKLYLAMNSE  GYLYTSELFT  P.ECKFKESV  FENYVTYSS
hu FGF-14   RV.VAIQGVK  TGLYIAMNGE  GYLYPSELFT  P.ECKFKESV  FENYVIYSS
hu FGF-16   GL.ISIRGVD  SGLYLGMNER  GELYGSKKLT  R.ECVFREQF  EENWYNTYAS
hu FGF-17   GSRVRIKGAE  SEKYICMNKR  GKLIGKPSGK  SKDCVFTEIV  LENNYTAFQN
hu FGF-18   GSQVRIKGKE  TEFYLCMNRK  GKLVGKPDGT  SKECVFIEKV  LENNYTALMS
hu FGF-19   RT.VAIKGVH  SVRYLCMGAD  GKMQGLLQYS  EEDCAFEEEI  RPDGYNVYRS
hu FGF-23   GF.VVITGVM  SRRYLCMDFR  GNIFGSHYFD  PENCRFQHQT  LENGYDVYHS
mu FGF-1    GE.VYIKGTE  TGQYLAMDTE  GLLYGSQTPN  E.ECLFLERL  EENHYNTYTS
mu FGF-2    GV.VSIKGVC  ANRYLAMKED  GRLLASKCVT  E.ECFFFERL  ESNNYNTYRS
mu FGF-3    GV.VAIKGLF  SGRYLAMNKR  GRLYASDHYN  A.ECEFVERI  HELGYNTYAS
mu FGF-4    GV.VSIFGVA  SRFFVAMSSR  GKLFGVPFFT  D.ECKFKEIL  LPNNYNAYEA
mu FGF-5    GI.VGIRGVF  SNKFLAMSKK  GKLHASAKFT  D.DCKFRERF  QENSYNTYAS
mu FGF-6    GV.VSLFGVK  SALFIAMNSK  GRLYTTPSFH  D.ECKFRETL  LPNNYNAYES
mu FGF-7    GI.VAIKGVE  SEYYLAMNKE  GKLYAKKECN  E.DCNFKELI  LENHYNTYAS
mu FGF-8    GSRVRVRGAE  TGLYICMNKK  GKLIAKSNGK  GKDCVFTEIV  LENNYTALQN
mu FGF-9    GL.VSIRGVD  SGLYLGMNEK  GELYGSEKLT  Q.ECVFREQF  EENWYNTYSS
mu FGF-10   GV.VAVKAIN  SNYYLAMNKK  GKLYGSKEFN  N.DCKLKERI  EENGYNTYAS
mu FGF-11   RV.VTIQSAK  LGHYMAMNAE  GLLYSSPHFT  A.ECRFKECV  FENYVLYAS
mu FGF-12   RV.VAIQGVK  ASLYVAMNGE  GYLYSSDVFT  P.ECKFKESV  FENYVIYSS
mu FGF-13   RV.VAIQGVQ  TKLYLAMNSE.GYLYTSEHFT  P.ECKFKESV  FENYVTYSS
mu FGF-14   RV.VAIQGVK  TGLYIAMNGE  GYLYPSELFT  P.ECKFKESV  FENYVIYSS
mu FGF-15   KT.IAIKDVS  SVRYLCMSAD  GKIYGLIRYS  EEDCTFREEM  DCLGYNQYRS
rat FGF-16  GL.ISIRGVD  SGLYLGMNER  GELFGSKKLT  R.ECVFREQF  EENWYNTYAS
mu FGF-17   GSQVRIKGKE  TEFYLCMNRK  GKLVGKPDGT  SKECVFIEKV  LENNYTALMS
```

89

# FIG. 2E

```
hu FGF-1    KKH...... ........A EKNWFVGLKK NGSCKRG..P RTHYGQKAIL
hu FGF-2    RKY...... ........T ..SWYVALKR TGQYKLG..S KTGPGQKAIL
hu FGF-3    RLYRTVSSTP GARRQ..PSA ERLWYVSVNG KGRPRRG..F KTRRTQKSSL
hu FGF-4    YKYPGM.... .......... ....FIALSK NGKTKKG..N RVSPTMKVTH
hu FGF-5    AIHRTEKTG. .......... .REWYVALNK RGKAKRGCSP RVKPQHISTH
hu FGF-6    DLYQGT.... .......... ....YIALSK YGRVKRG..S KVSPIMTVTH
hu FGF-7    A......... ..KWT..HNG GEM.FVALNQ KGIPVRG..K KTKKEQKTAH
hu FGF-8    AKYEG..... .......... ...WYMAFTR KGRPRKG..S KTRQHQREVH
hu FGF-9    NLYK...... ....H..VDT GRRYYVALNK DGTPREG..T RTKRHQKFTH
hu FGF-10   F......... ..NWQ..HNG RQM.YVALNG KGAPRRG..Q KTRRKNTSAH
hu FGF-11   ALYR...... ....Q..RRS GRAWYLGLDK EGQVMKG..N RVKKTKAAAH
hu FGF-12   TLYR...... ....Q..QES GRAWFLGLNK EGQIMKG..N RVKKTKPSSH
hu FGF-13   MIYR...... ....Q..QQS GRGWYLGLNK EGEIMKG..N HVKKNKPAAH
hu FGF-14   MLYR...... ....Q..QES GRAWFLGLNK EGQAMKG..N RVKKTKPAAH
hu FGF-16   TLYK...... ....H..SDS ERQYYVALNK DGSPREG..Y RTKRHQKFTH
hu FGF-17   ARHEG..... .......... ...WFMAFTR QGRPRQA..S RSRQNQREAH
hu FGF-18   AKYSG..... .......... ...WYVGFTK KGRPRKG..P KTRENQQDVH
hu FGF-19   EKHRLPVSLS SAKQ...... .....RQLYK NRGFLPLSHF ...LPMLPMV
hu FGF-23   PQYHFLVSLG RAKRAFLPGM NPPPYSQFLS RRNEIPLIHF NTPIPRRHTR
mu FGF-1    KKH...... ........A EKNWFVGLKK NGSCKRG..P RTHYGQKAIL
mu FGF-2    RKY...... ........S ..SWYVALKR TGQYKLG..S KTGPGQKAIL
mu FGF-3    RLYRTGSSGP GAQRQ..PGA QRPWYVSVNG KGRPRRG..F KTRRTQKSSL
mu FGF-4    YAYPGM.... .......... ....FMALSK NGRTKKG..N RVSPTMKVTH
mu FGF-5    AIHRTEKTG. .......... .REWYVALNK RGKAKRGCSP RVKPQHVSTH
mu FGF-6    DLYRGT.... .......... ....YIALSK YGRVKRG..S KVSPIMTVTH
mu FGF-7    A......... ..KWT..HSG GEM.FVALNQ KGIPVKG..K KTKKEQKTAH
mu FGF-8    AKYEG..... .......... ...WYMAFTR KGRPRKG..S KTRQHQREVH
mu FGF-9    NLYK...... ....H..VDT GRRYYVALNK DGTPREG..T RTKRHQKFTH
mu FGF-10   F......... ..NWQ..HNG RQM.YVALNG KGAPRRG..Q KTRRKNTSAH
mu FGF-11   ALYR...... ....Q..RRS GRAWYLGLDK EGRVMKG..N RVKKTKAAAH
mu FGF-12   TLYR...... ....Q..QES GRAWFLGLNK EGQIMKG..N RVKKTKPSSH
mu FGF-13   MIYR...... ....Q..QQS GRGWYLGLNK EGEIMKG..N HVKKNKPAAH
mu FGF-14   MLYR...... ....Q..QES GRAWFLGLNK EGQVMKG..N RVKKTKPAAH
mu FGF-15   MKHHLHIIFI QAKP...... .....REQLQ DQ...KPSNF ...IPVFHRS
rat FGF-16  TLYK...... ....H..SDS ERQYYVALNK DGSPREG..Y RTKRHQKFTH
mu FGF-17   AKYSG..... .......... ...WYVGFTK KGRPRKG..P KTRENQQDVH
```

EP 1 947 182 A1

# FIG. 2F

```
hu FGF-1    FLPLPVSSD. .......... .......... .......... ..........
hu FGF-2    FLPMSAKS.. .......... .......... .......... ..........
hu FGF-3    FLPRVLDHRD HEM....VRQ LQSGLPRPPG KGVQPRRRRQ K.QSPDNLEP
hu FGF-4    FLPRL..... .......... .......... .......... ..........
hu FGF-5    FLPRFKQSEQ PEL....SFT VTVPEKKNPP SPIKSK.... ..........
hu FGF-6    FLPRI..... .......... .......... .......... ..........
hu FGF-7    FLPMAI.... .......... .......... .......... ..........
hu FGF-8    FMKRLPRG.. .H........ HTTEQSLRFE FLNYPPFTRS LRGSQRTWAP
hu FGF-9    FLPRPVD... .......... .......... .......... ....PDKV..
hu FGF-10   FLPMVVH... .......... .......... .......... ..........
hu FGF-11   FLPKLLEVAM YQE....PSL HSVPEASPSS PPAP...... ..........
hu FGF-12   FVPKPIEVCM YRE....PSL HEIGEKQGR. ....SRKSS. ..........
hu FGF-13   FLPKPLKVAM YKE....PSL HDLTEFSRSG SGTPTKSRS. ..........
hu FGF-14   FLPKPLEVAM YRE....PSL HDVGETVPKP GVTPSKSTS. ..........
hu FGF-16   FLPRPVD... .......... .......... .......... ....PSKL..
hu FGF-17   FIKRLYQG.. .QL....PFP NHAEKQKQFE FVGSAPTRRT ....KRTRRP
hu FGF-18   FMKRYPKG.. .Q........ ..PELQKPFK YTTVTKRSRR IRPTHPA...
hu FGF-19   PEEPEDLRGH LE........ .......SDM FSSPLETDSM ..DPFGLVTG
hu FGF-23   SAEDDSERDP LNVLKPRARM TPAPASCSQE LPSAEDNSPM ASDPLGVVRG
mu FGF-1    FLPLPVSSD. .......... .......... .......... ..........
mu FGF-2    FLPMSAKS.. .......... .......... .......... ..........
mu FGF-3    FLPRVLGHKD HEM....VRL LQSSQPRAPG EGSQPRQRRQ KKQSPGDHGK
mu FGF-4    FLPRL..... .......... .......... .......... ..........
mu FGF-5    FLPRFKQSEQ PEL....SFT VTVPEKKKPP ..VKPK.... ..........
mu FGF-6    FLPRI..... .......... .......... .......... ..........
mu FGF-7    FLPMAI.... .......... .......... .......... ..........
mu FGF-8    FMKRLPRG.. .H........ HTTEQSLRFE FLNYPPFTRS LRGSQRTWAP
mu FGF-9    FLPRPVD... .......... .......... .......... ....PDKV..
mu FGF-10   FLPMTIQ... .......... .......... .......... ..........
mu FGF-11   FVPKLLEVAM YRE....PSL HSVPETSPSS PPAH...... ..........
mu FGF-12   FVPKPIEVCM YRE....PSL HEIGEKQGR. ....SRKSS. ..........
mu FGF-13   FLPKPLKVAM YKE....PSL HDLTEFSRSG SGTPTKSRS. ..........
mu FGF-14   FLPKPLEVAM YRE....PSL HDVGETVPKA GVTPSKSTS. ..........
mu FGF-15   FFETGD...Q LR........ .......SKM FSLPLESDSM ..DPFRMVED
rat FGF-16  FLPRPVD... .......... .......... .......... ....PSKL..
mu FGF-17   FMKRYPKG.. .Q........ ..AELQKPFK YTTVTKRSRR IRPTHPG...
```

91

## FIG. 2G

```
hu FGF-1     .......... .......... ..
hu FGF-2     .......... .......... ..
hu FGF-3     SHVQASRL.. GSQLEASA.. .H
hu FGF-4     .......... .......... ..
hu FGF-5     IPLSAPRKNT NSVKYRLKFR FG
hu FGF-6     .......... .......... ..
hu FGF-7     .......... .......... .T
hu FGF-8     EP........ .......... .R
hu FGF-9     .......... .PELYKDILS QS
hu FGF-10    .......... .......... .S
hu FGF-11    .......... .......... ..
hu FGF-12    ...GTPTMNG GKVVNQ...D ST
hu FGF-13    ...VSGVLNG GKSMSHN..E ST
hu FGF-14    ...ASAIMNG GKPVNKS..K TT
hu FGF-16    .......... .PSMSRDLFH YR
hu FGF-17    QPL....... .......... .T
hu FGF-18    .......... .......... ..
hu FGF-19    LEA.VRS... .....PSFEK ..
hu FGF-23    GRVNTHAGGT GPEGCRPFAK FI
mu FGF-1     .......... .......... ..
mu FGF-2     .......... .......... ..
mu FGF-3     METLSTRATP STQLHTGGLA VA
mu FGF-4     .......... .......... ..
mu FGF-5     VPLSQPRRSP SPVKYRLKFR FG
mu FGF-6     .......... .......... ..
mu FGF-7     .......... .......... .T
mu FGF-8     EP........ .......... .R
mu FGF-9     .......... .PELYKDILS QS
mu FGF-10    .......... .......... .T
mu FGF-11    .......... .......... ..
mu FGF-12    ...GTPTMNG GKVVNQ...D ST
mu FGF-13    ...VSGVLNG GKSMSHN..E ST
mu FGF-14    ...ASAIMNG GKPVNKC..K TT
mu FGF-15    VDHLVKS... .....PSFQK ..
rat FGF-16   .......... .PSMSRDLFR YR
mu FGF-17    .......... .......... ..
```

## FIG. 3

## FIG. 4

LYMPH NODE (H&E)

LYMPH NODE (ISH)

50 um

cortex

medulla

THYMUS (ISH)

50 um

TIBIA (H&E)

muscle

bone

TIBIA (ISH)

50 um

HEAD (H&E)

nasal epithelium

salivary gland

bone

HEAD (ISH)

50 um

FIG. 5

# FIG. 6

**European Patent Office**

## PARTIAL EUROPEAN SEARCH REPORT

which under Rule 63 of the European Patent Convention shall be considered, for the purposes of subsequent proceedings, as the European search report

**Application Number**

EP 07 01 7423

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/27100 A (GENENTECH INC ;BOTSTEIN DAVID (US); GODDARD AUDREY (US); GURNEY AU) 3 June 1999 (1999-06-03) * the whole document * ----- | 1-57 | INV. C12N15/12 C12N15/62 C12N15/86 C07K14/50 |
| X | DATABASE EMHUM EMBL Heidelberg, Germany; AC/ID AC008012 12 July 1999 (1999-07-12), MUZNY D ET AL.: "Homo sapiens 12p13 BAC RPCI11-388F6" XP002177850 * abstract * ----- | 1-8,10, 11 | C07K16/22 C12Q1/68 A61K9/00 A61K38/18 A01K67/027 G01N33/68 |
| P,X | WO 00/73454 A (GENENTECH INC) 7 December 2000 (2000-12-07) see PRO9828 (SEQ ID NO: 510 and 511) * page 375 - page 378; claims 1-28; figures 323,324; example 175 * * page 381 - page 398 * ----- | 1-57 | |
| P,X | WO 00/60085 A (MILLENNIUM PHARM INC) 12 October 2000 (2000-10-12) * the whole document * ----- -/-- | 1-57 | **TECHNICAL FIELDS SEARCHED (IPC)** C12N C07K C12Q A61K A01K G01N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 13 June 2008 | Oderwald, Harald |

EPO FORM 1503 03.82 (P04E07)

European Patent
Office

**PARTIAL EUROPEAN SEARCH REPORT**

Application Number

EP 07 01 7423

| | **DOCUMENTS CONSIDERED TO BE RELEVANT** | | CLASSIFICATION OF THE APPLICATION (IPC) |
|---|---|---|---|
| Category | Citation of document with indication, where appropriate, of relevant passages | Relevant to claim | |
| E | WO 01/49740 A (ZYMOGENETICS INC) 12 July 2001 (2001-07-12) * the whole document * ----- | 1-57 | |
| | | | TECHNICAL FIELDS SEARCHED (IPC) |

EPO FORM 1503 03.82 (P04C10)

Although claims 33, 48-50 and 55 are directed to a method of treatment of the human/animal body (Art. 53(c) EPC), the search has been carried out and based on the alleged effects of the compound/composition.

Although claim(s) 36 (as far as in vivo methods are concerned) and 51 are directed to a diagnostic method practised on the human/animal body (Art. 53(c) EPC), the search has been carried out and based on the alleged effects of the compound/composition.

-----

Claim(s) searched completely:
1-17

Claim(s) not searched:
18,19,31-36,49,50

Reason for the limitation of the search:

Present claims 18, 19, 31-36, 49 and 50 relate to a product/compound defined by reference to a desirable characteristic or property, namely 'selective binding agent or fragment thereof' or 'agonist or antagonist'.

The claims cover all products/compounds having this characteristic or property, whereas the application provides support within the meaning of Article 84 EPC and/or disclosure within the meaning of Article 83 EPC for only a very limited number of such products/compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the product/compound by reference to a result to be achieved. Again, this lack of clarity in the present case is such as to render a meaningful search over the whole of the claimed scope impossible (Rule 63 EPC and Guidelines B-VIII, 3). Consequently, the search has been carried out for those parts of the claims which appear to be clear, supported and disclosed, namely those parts relating to the products/compounds 'FGF-23 binding antibodies' as mentioned in the description at pages 6, 49-54 and example 4.

**ANNEX TO THE EUROPEAN SEARCH REPORT
ON EUROPEAN PATENT APPLICATION NO.**                    EP 07 01 7423

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2008

| Patent document cited in search report | | Publication date | Patent family member(s) | | Publication date |
|---|---|---|---|---|---|
| WO 9927100 | A | 03-06-1999 | AU | 733222 B2 | 10-05-2001 |
| | | | AU | 1703399 A | 15-06-1999 |
| | | | CA | 2309783 A1 | 03-06-1999 |
| | | | EP | 1032668 A1 | 06-09-2000 |
| | | | JP | 3653469 B2 | 25-05-2005 |
| | | | JP | 2002503446 T | 05-02-2002 |
| | | | NZ | 504425 A | 26-10-2001 |
| WO 0073454 | A | 07-12-2000 | CA | 2383254 A1 | 07-12-2000 |
| | | | EP | 1210418 A1 | 05-06-2002 |
| WO 0060085 | A | 12-10-2000 | AU | 3924300 A | 23-10-2000 |
| WO 0149740 | A | 12-07-2001 | AU | 2759201 A | 16-07-2001 |
| | | | CA | 2396401 A1 | 12-07-2001 |
| | | | EP | 1246843 A1 | 09-10-2002 |
| | | | JP | 2003518944 T | 17-06-2003 |

EPO FORM P0459

For more details about this annex : see Official Journal of the European Patent Office, No. 12/82

# EP 1 947 182 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5480981 A
- US 5808029 A
- WO 9723614 A
- US 9723183 W
- WO 9014363 A
- US 5824469 A
- US 5763192 A
- US 5814476 A
- US 5723323 A
- US 5817483 A
- US 9820094 W
- WO 9915650 A
- US 4816567 A
- US 5585089 A
- US 5693762 A
- US 9605928 W
- US 9306926 W
- US 5545807 A
- US 91245 A
- GB 8901207 W
- EP 546073 B1
- EP 546073 A1
- US 9817364 W
- US 4376110 A
- US 5234784 A
- EP 0154316 A
- EP 0401384 A
- US 4179337 A
- US 5252714 A
- US 5557032 A
- US 5489743 A
- WO 9428122 A
- WO 9420069 A
- US 9300829 W
- US 3773919 A
- EP 058481 A
- EP 133988 A
- EP 036676 A
- EP 088046 A

- EP 143949 A
- US 5272071 A
- EP 9193051 A
- EP 505500 A
- US 9007642 W
- WO 9109955 A
- WO 9505452 A
- US 9409299 W
- US 4892538 A
- US 5011472 A
- US 5106627 A
- WO 9110425 A
- WO 9110470 A
- WO 9641865 A
- WO 9731898 A
- WO 97131899 A
- US 5364791 A
- WO 9640911 A
- WO 9710337 A
- US 5514578 A
- WO 9738117 A
- WO 9637609 A
- WO 9303162 A
- US 5464758 A
- US 5650298 A
- US 5654168 A
- US 5741679 A
- US 5834186 A
- WO 9534670 A
- US 9507178 W
- US 5672344 A
- US 5399346 A
- US 5631236 A
- US 5672510 A
- US 5635399 A
- US 4970154 A
- US 5679559 A
- US 5676954 A
- WO 9640958 A

### Non-patent literature cited in the description

- **SIMONSEN ; LODISH.** *Trends Pharmacol. Sci.,* 1994, vol. 15, 437-41
- **TARTAGLIA et al.** *Cell,* 1995, vol. 83, 1263-71
- **GRAHAM et al.** *Virology,* 1973, vol. 52, 456

- **SAMBROOK et al.** Molecular Cloning, A Laboratory Manual. Cold Spring Harbor Laboratories, 1989
- **DAVIS et al.** Basic Methods in Molecular Biology. Elsevier, 1986
- **CHU et al.** *Gene,* 1981, vol. 13, 197

- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridisation: A Practical Approach. IRL Press Limited
- **SUGGS et al.** Developmental Biology Using Purified Genes. 1981, vol. 683
- **KYTE et al.** *J. Mol. Biol.,* 1982, vol. 157, 105-31
- **MOULT.** *Curr. Opin. Biotechnol.,* 1996, vol. 7, 422-27
- **CHOU et al.** *Biochemistry,* 1974, vol. 13, 222-45
- **CHOU et al.** *Biochemistry,* 1974, vol. 113, 211-22
- **CHOU et al.** *Adv. Enzymol. Relat. Areas Mol. Biol.,* 1978, vol. 47, 45-48
- **CHOU et al.** *Ann. Rev. Biochem.,* 1978, vol. 47, 251-276
- **CHOU et al.** *Biophys. J.,* 1979, vol. 26, 367-84
- **HOLM et al.** *Nucleic Acids Res.,* 1999, vol. 27, 244-47
- **BRENNER et al.** *Curr. Opin. Struct. Biol.,* 1997, vol. 7, 369-76
- **JONES.** *Curr. Opin. Struct. Biol.,* 1997, vol. 7, 377-87
- **SIPPL et al.** *Structure,* 1996, vol. 4, 15-19
- **BOWIE et al.** *Science,* 1991, vol. 253, 164-70
- **GRIBSKOV et al.** *Methods Enzymol.,* 1990, vol. 183, 146-59
- **GRIBSKOV et al.** *Proc. Nat. Acad. Sci. U.S.A.,* 1987, vol. 84, 4355-58
- **CAPON et al.** *Nature,* 1989, vol. 337, 525-31
- **ZHENG et al.** *J. Immunol.,* 1995, vol. 154, 5590-600
- **FISHER et al.** *N. Engl. J. Med.,* 1996, vol. 334, 1697-1702
- **VAN ZEE et al.** *J. Immunol.,* 1996, vol. 156, 2221-30
- **HARVILL et al.** *Immunotech.,* 1995, vol. 1, 95-105
- **LINSLEY.** *J. Exp. Med.,* 1991, vol. 174, 561-69
- Computational Molecular Biology. Oxford University Press, 1988
- Biocomputing: Informatics and Genome Projects. Academic Press, 1993
- Computer Analysis of Sequence Data. Humana Press, 1994
- Sequence Analysis in Molecular Biology. Academic Press, 1987
- Sequence Analysis Primer. M. Stockton Press, 1991
- **CARILLO et al.** *SIAM J. Applied Math,* 1988, vol. 48, 1073
- **DEVEREUX et al.** *Nucleic Acids Res.,* 1984, vol. 12, 387
- **ALTSCHUL et al.** *J. Mol. Biol.,* 1990, vol. 215, 403-10
- **ALTSCHUL et al.** BLAST Manual
- **HENIKOFF et al.** *Proc. Natl. Acad. Sci USA,* 1992, vol. 89, 10915-19
- **NEEDLEMAN ; WUNSCH.** *J. Mol. Biol.,* 1970, vol. 48, 443-53
- Program Manual, Wisconsin Package. September 1997
- **SAMBROOK et al.** Molecular Cloning: A Laboratory Manual. Cold Spring Harbor Laboratory Press, 1989
- Current Protocols in Molecular Biology. Green Publishers Inc. and Wiley and Sons, 1994
- **ENGELS et al.** *Angew. Chem. Intl. Ed.,* 1989, vol. 28, 716-34
- Meth. Enz. Academic Press, 1990, vol. 185
- **BEMOIST ; CHAMBON.** *Nature,* 1981, vol. 290, 304-10
- **YAMAMOTO et al.** *Cell,* 1980, vol. 22, 787-97
- **WAGNER et al.** *Proc. Natl. Acad. Sci. U.S.A.,* 1981, vol. 78, 1444-45
- **BRINSTER et al.** *Nature,* 1982, vol. 296, 39-42
- **KAMAROFF et al.** *Proc. Natl. Acad. Sci. U.S.A.,* 1978, vol. 75, 3727-31
- **DEBOER et al.** *Proc. Natl. Acad. Sci. U.S.A.,* 1983, vol. 80, 21-25
- **SWIFT et al.** *Cell,* 1984, vol. 38, 639-46
- **ORNITZ et al.** *Cold Spring Harbor Symp. Quant. Biol.,* 1986, vol. 50, 399-409
- **MACDONALD.** *Hepatology,* 1987, vol. 7, 425-515
- **HANAHAN.** *Nature,* 1985, vol. 315, 115-22
- **GROSSCHEDL et al.** *Cell,* 1984, vol. 38, 647-58
- **ADAMES et al.** *Nature,* 1985, vol. 318, 533-38
- **ALEXANDER et al.** *Mol. Cell. Biol,* 1987, vol. 7, 1436-44
- **LEDER et al.** *Cell,* 1986, vol. 45, 485-95
- **PINKERT et al.** *Genes and Devel.,* 1987, vol. 1, 268-76
- **KRUMLAUF et al.** *Mol. Cell. Biol.,* 1985, vol. 5, 1639-48
- **HAMMER et al.** *Science,* 1987, vol. 235, 53-58
- **KELSEY et al.** *Genes and Devel.,* 1987, vol. 1, 161-71
- **MOGRAM et al.** *Nature,* 1985, vol. 315, 338-40
- **KOLLIAS et al.** *Cell,* 1986, vol. 46, 89-94
- **READHEAD et al.** *Cell,* 1987, vol. 48, 703-12
- **SANI.** *Nature,* 1985, vol. 314, 283-86
- **MASON et al.** *Science,* 1986, vol. 234, 1372-78
- **URLAUB et al.** *Proc. Natl. Acad. Sci. US.A.,* 1980, vol. 97, 4216-20

- **KITTS et al.** *Biotechniques,* 1993, vol. 14, 810-17
- **LUCKLOW.** *Curr. Opin. Biotechnol.,* 1993, vol. 4, 564-72
- **LUCKLOW et al.** *J. Virol,* 1993, vol. 67, 4566-79
- **MARSTON et al.** *Meth. Enz,* 1990, vol. 182, 264-75
- **AUSUBEL et al.** Current Protocols in Molecular Biology. Green Publishers Inc. and Wiley and Sons, 1993
- **MERRIFIELD et al.** *J. Am. Chem. Soc.,* 1963, vol. 85, 2149
- **HOUGHTEN et al.** *Proc Natl Acad. Sci. USA,* 1985, vol. 82, 5132
- **STEWART ; YOUNG.** Solid Phase Peptide Synthesis. Pierce Chemical Co, 1984
- **ROBERTS et al.** *Proc. Natl. Acad. Sci. U.S.A.,* 1997, vol. 94, 12297-303
- **ROBERTS.** *Curr. Opin. Chem. Biol.,* 1999, vol. 3, 268-73
- **KOHLER et al.** *Nature,* 1975, vol. 256, 495-97
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MORRISON et al.** *Proc. Natl. Acad. Sci.,* 1985, vol. 81, 6851-S5
- **JONES et al.** *Nature,* 1986, vol. 321, 522-25
- **RIECHMANN et al.** *Nature,* 1998, vol. 332, 323-27
- **VERHOEYEN et al.** *Science,* 1988, vol. 239, 1534-36
- **JAKOBOVITS et al.** *Proc. Natl. Acad. Sci.,* 1993, vol. 90, 2551-55
- **JAKOBOVITS et al.** *Nature,* 1993, vol. 362, 255-58
- **BRUGGERMANN et al.** *Year in Immuno,* 1993, vol. 7, 33
- **HOOGENBOOM et al.** *J. Mol. Biol.,* 1991, vol. 227, 381
- **MARKS et al.** *J. Mol. Biol.,* 1991, vol. 222, 581
- **SOLA.** Monoclonal Antibodies: A Manual of Techniques. CRC Press, Inc, 1987, 147-158
- **BAYER et al.** *Meth. Enz.,* 1990, vol. 184, 138-63
- **FRANCIS et al.** *Focus on Growth Factors,* 1992, vol. 3, 4-10
- Current Protocols in Molecular Biology. Green Publishers Inc. and Wiley and Sons, 1995
- **FALWELL et al.** *Proc. Natl. Acad. Sci. U.S.A.,* 1994, vol. 91, 664-68
- **SCHWARZE et al.** *Science,* 1999, vol. 285, 1569-72
- **NAGAHARA et al.** *Nat. Med.,* 1998, vol. 4, 1449-52
- **STRAUS.** *Science,* 1999, vol. 285, 1466-67
- Remington's Pharmaceutical Sciences. Mack Publishing Company, 1990
- **SIDMAN et al.** *Biopolymers,* 1983, vol. 22, 547-56
- **LANGER et al.** *J. Biomed. Mater. Res.,* 1981, vol. 15, 167-277
- **LANGER.** *Chem. Tech.,* 1982, vol. 12, 98-105
- **EPPSTEIN et al.** *Proc. Natl. Acad. Sci. USA,* 1985, vol. 82, 3688-92
- **KUCHERLAPATI.** *Prog. in Nucl. Acid Res. & Mol. Biol.,* 1989, vol. 36, 301
- **THOMAS et al.** *Cell,* 1986, vol. 44, 419-28
- **THOMAS ; CAPECCHI.** *Cell,* 1987, vol. 51, 503-12
- **DOETSCHMAN et al.** *Proc. Natl. Acad. Sci. U.S.A.,* 1988, vol. 85, 8583-87
- **DOETSCHMAN et al.** *Nature,* 1987, vol. 330, 576-78
- **SAUER.** *Curr. Opin. Biotechnol,* 1994, vol. 5, 521-27
- **SAUER.** *Methods Enzymol.,* 1993, vol. 225, 890-900
- **BAUBONIS ; SAUER.** *Nucleic Acids Res.,* 1993, vol. 21, 2025-29
- **O'GORMAN et al.** *Science,* 1991, vol. 251, 1351-55
- **WINN et al.** *Exper. Neurol.,* 1991, vol. 113, 322-29
- **AEBISCHER et al.** *Exper. Neurol.,* 1991, vol. 111, 269-75
- **TRESCO et al.** *ASAIO,* 1992, vol. 38, 17-23
- **ARIDOR et al.** *Science,* 2000, vol. 287, 816-17
- **RIVERA et al.** *Science,* 2000, vol. 287, 826-30
- **HEFTI.** *Neurobiology,* 1994, vol. 25, 1418-35
- The ADHR Consortium. *Nature Genetics,* 2000, vol. 26, 345-48
- **YAMASHITA et al.** *Biochem. Biophys. Res. Commun.,* 2000, vol. 277, 494-98
- **NISHIMURA et al.** *Biochim. Biophys. Acta,* 2000, vol. 1492, 203-06
- **MCWHIRTER et al.** *Development,* 1997, vol. 124, 3221-32
- **XIE et al.** *Cytokine,* 1999, vol. 11, 729-35
- **MATSUDAIRA et al.** *J. Biol. Chem.,* 1987, vol. 262, 10-35
- **HUDSON ; BAY.** Practical Immunology. Blackwell Scientific Publications
- Pathologic Basis of Disease. 1994